# EUROPEAN PATENT APPLICATION

(11) **EP 1 085 024 A1**
(43) Date of publication of application: **21.03.2001**
(21) Application number: 99923926.2
(22) Date of filing: 04.06.1999
(51) Int. Cl.: C07K 14/47, C12N 5/10, C12N 15/12, C12P 21/02

(54) **PEPTIDES HAVING NUCLEAR TRANSPORT ACTIVITY**

(30) Priority: 05.06.1998 JP 17406598; 14.04.1999 JP 10726299
(71) Applicant: Helix Research Institute, Kisarazu-shi, Chiba 292-0812 (JP)
(72) Inventor: UEKI, Nobuhide, Port Jefferson Station, NY 11776 (US); YANO, Kazuhiro, Osaka 573-0163 (JP)
(74) Representative: VOSSIUS & PARTNER
(86) International application number: JP9903015
(87) International publication number: WO9964455

(57) **Abstract**

A method, in which properties of transcription factor are utilized, was originally developed for simply isolating a DNA encoding peptide having nuclear transport activity. Multiple novel DNAs encoding peptides having nuclear transport activity were successfully isolated by using this method.

## Description

### Technical Field

The present invention relates to a peptide having nuclear transport activity and DNA encoding the peptide. The peptide can be used as a carrier for transferring substances to the nucleus. Therefore, the present invention chiefly belongs to the field of drug delivery.

### Background Art

In order to optimize drug efficacy and decrease adverse effects of a drug, it is quite important that the drug are specifically delivered to its target site(s) and that its optimal concentration is maintained at the target site(s). A variety of systems have been developed to deliver drugs to particular target sites.

There are many previously developed methods for transferring substances to the nucleus, which include microinjection, transfection, antibody method, peptide method, and others.

Microinjection (Experimental Medicine, supplement, New Handbook for Genetic Engineering, Yodosha, 173-176 (1996)) is a method in which samples are directly microinjected without failure into a nucleus by using a microneedle; usually, this method has been frequently used for introducing macromolecules such as nucleic acid and protein. However, only micro-quantities can be injected into the nucleus; and, in particular, if samples do not stably stay in the nucleus, there is a possibility that the samples rapidly diffuse out of the nucleus soon after the injection.

Transfection (Experimental Medicine, supplement, New Handbook for Genetic Engineering, Yodosha, 155-168 (1996)) has been frequently used particularly for introducing foreign genes; this method includes calcium-phosphate method, DEAE-dextran method, liposome method, and lipofection method. In these methods, samples are complexed with carriers and then allowed to transfer to cells, which is mediated through phagocytosis or membrane fusion; the samples are finally imported into a nucleus, although the detailed mechanism remains to be clarified.

In the antibody method (Avrameas A, et al. Proc. Natl. Acad. Sci. USA 1998 May 12, 95(10) 5601-5606), a specific antibody is prepared against a particular nuclear protein or DNA, and the antibody is used as a carrier or part of the carrier, thereby introducing samples into a nucleus. This method is very sophisticated, but establishment of a suitable antibody requires much time and effort.

In the peptide method (Chaloin L, et al. Biochem. Biophys. Res. Commun. 1998 Feb 13, 243(2) 601-608; Sebestyen MG, et al. Nat. Biotechnol. 1998 Jan, 16(1): 80-85), instead of the antibody used in the antibody method, a peptide containing a nuclear localization signal or the like is used; such a peptide is known to function as a much smaller molecule, whose minimal length is 4-5 amino acid residues, than the antibody. Further, unlike the antibody, which transfers passively to a nucleus by diffusion, the nuclear transport peptide is actively incorporated into a nucleus, which is mediated by nuclear localization signal-dependent carriers present in a cell itself; the method, thus, has the great advantage of transporting to a nucleus highly efficiently and rapidly.

### Disclosure of the Invention

The objective of the present invention is to provide a novel peptide with nuclear transport activity and DNA encoding the peptide. Another objective is to provide a system for nuclear transport of substance using them.

To achieve the objectives as mentioned above, the present inventors, first, have originally developed a method for simply isolating a DNA encoding a peptide having nuclear transport activity, utilizing properties of transcription factors. This method was designated as "NTT method". The principle of NTT method is as follows. Eukaryotic transcription factors transfer to a nucleus and interact with promoter regions of particular genes, thereby inducing the expression of the particular genes. Nuclear localization signal present in the transcription factors is believed to be responsible for the nuclear transport activity of the transcription factors. Accordingly, when an unidentified peptide is substituted for the portion with nuclear transport activity in a transcription factor and the resulting fusion protein is expressed in cells, if the unidentified peptide has nuclear transport activity, the fusion protein transfers to a nucleus, interacts with some specific promoter regions, and thus, induces the expression of particular genes downstream of the promoter regions. On the other hand, if the unidentified peptide of the fusion protein has no nuclear transport activity, the fusion protein neither transfers to the nucleus nor induces the expression of the particular genes. Thus, by monitoring whether or not the expression of genes downstream of a specific promoter is induced by a fusion protein between an unidentified peptide and a transcription factor lacking nuclear transport activity, it is possible to judge whether or not the unidentified peptide in the fusion protein has nuclear transport activity.

According to the principle, the present inventors prepared a fusion DNA between DNA encoding a transcription factor lacking nuclear transport activity and DNA to be tested, introduced the resulting fusion DNA into a eukaryotic host containing a promoter region to be activated by binding of the transcription factor and a reporter gene of which expression is to be induced by the activation of the promoter region, and then detected the expression of the reporter gene. The result showed that the expression of the reporter gene was induced when DNA to be tested was a DNA encoding a peptide having nuclear transport activity, but that no expression was induced when DNA to be tested was a DNA encoding a peptide lacking nuclear transport activity. Namely, the inventors found that it is practically attainable, based on the above-mentioned principle, to specifically isolate a DNA encoding a peptide having nuclear transport.

In the next step, the present inventors prepared a library of cDNA encoding a fusion protein of a transcription factor lacking a region responsible for nuclear transport activity and an unrelated peptide, and then introduced the library constructs into cells; the inventors screened the library for cDNA encoding a peptide having nuclear transport activity by monitoring the expression of the reporter gene. The inventors thus successfully isolated multiple novel DNAs encoding peptide having nuclear transport activity from the cDNA library.

The present invention relates to novel DNAs encoding peptides with nuclear transport activity that were isolated by using NTT method developed originally by the inventors, peptides encoded by the DNAs, and the use thereof. More specifically, the present invention relates to:
(1) a peptide comprising any one of the amino acid sequences set forth in SEQ ID NOs: 1-21 and 101-107 and having nuclear transport activity;
(2) a peptide comprising any one of the amino acid sequences set forth in SEQ ID NOs: 1-21 and 101-107, in which one or more amino acids are substituted, deleted, and/or added, and having nuclear transport activity;
(3) the peptide of (1) or (2), wherein the peptide is a fusion peptide comprising a desired peptide to be transported to a nucleus;
(4) a DNA encoding the peptide of any one of (1) to (3);
(5) a vector comprising the DNA of (4);
(6) a transformant carrying the vector of (5); and
(7) a method for producing the peptide of (1) or (2), comprising culturing the transformant of (6).

The term "peptide" used herein means a compound in which amino acids are connected with each other through peptide bonds. The "peptide" of the present invention includes "polypeptide" and "protein", both of which are longer "peptide".

DNAs encoding the peptide having nuclear transport activity, which were isolated by NTT method developed by the present inventors, are shown in SEQ ID NOs: 22-42 and 92-100. Respective peptides encoded by these DNAs are also shown in SEQ ID NOs: 1-21 and 101-107, which are included in the peptides of the present invention.

The peptides of the present invention share a common feature that the peptides have nuclear transport activity. Thus the peptide of the present invention can be used as a carrier for transporting desired substances to a nucleus. A peptide comprising any of the amino acid sequences of SEQ ID NOs: 1-21 and 101-107 is suitable as the peptide of the present invention that is used for transporting substances into a nucleus.

When one or more amino acids are substituted, deleted, and/or added in the amino acid sequences of these peptides, such peptides are also usable as far as they have nuclear transport activity. Such amino-acid modification can be performed by methods known to those skilled in the art, for example, site-specific mutagenesis (Current Protocols in Molecular Biology, edit. Ausubel et al. (1987) Publich. John Wily & Sons, Section 8.1-8.5) or PCR (PCR Primer: A Laboratory Manual, Cold Spring Harbor Laboratory Press, 1995, 581-621). There is no particular limitation on modification site and the number of modifications in the amino acid sequence, as far as the modified peptide has nuclear transport activity. The number of amino acid modifications is generally 20 amino acids or less, preferably 10 amino acids or less, more preferably 5 amino acids or less, still more preferably 3 amino acids or less.

There is no limitation on substances to be transported to a nucleus by using the peptide of the present invention as a carrier. For example, such substances include a peptide, nucleic acid, polysaccharide, metal, synthetic compound, and others. These substances can be drugs used for the treatment, prevention, and/or diagnosis of diseases, but are not limited to these uses. The substances can be those used not only for clinical purposes but also, for example, in biological analyses or others.

In order to transfer substances to a nucleus by using the peptide of the present invention, the substances are needed to bind to the peptide of the present invention. There is no limitation on the types of binding as far as the binding force is sufficient for the purpose. The binding may be a chemical cross-linking formed by using a cross-linking agent ("Protein Chemistry (2)", Sequel to Lectures in Biochemical Experiment, volume 2, Ed., Japanese Biochemical Society, Tokyo Kagaku Doujin, pp. 604-618). Such a cross-linking agent includes, for example, dimethyl suberoimidate dihydrochloride, suberic acid di-*N*-hydroxysuccinimide ester, tartaric acid di-*N*-hydroxysuccinimide ester, *p*-phenylene bismaleimide, methyl 4-mercaptobutylimidate hydrochloride, methyl 4-azidebenzoimidate hydrochloride, glutaraldehyde, *N*-succinimidyl 3-(2-pyridyldithio)-propionate, *N*-(γ-maleimide butyryloxy)succinimide, *N*-(ε-maleimide hexanoyloxy)succinimide, 1-ethyl-3-(3-dimethylaminopropyl)carbodiimide, all of which have been used frequently. When the substance to be transferred to a nucleus is a peptide, a DNA encoding the peptide is connected to a DNA encoding the peptide of the present invention and the peptides are expressed as a fusion peptide in which both peptides are linked together. The two peptides may be linked via a spacer region. Other than covalent bonding as described above, binding based on interaction between the peptide of the present invention and the substance to be transported to a nucleus can be used. Such binding includes, for example, binding based on electrostatic interaction or hydrophobic interaction, binding using a binding motif of protein-protein interaction, protein-nucleic acid interaction, or enzyme-substrate interaction, and the like. When originally the peptide of the present invention and the substance to be transported to a nucleus do not have such binding ability between them, a functional domain(s) having the binding ability may be newly added to either or both of them.

There is no particular limitation on target cells for the transport of a substance using the peptide of the present invention as a carrier; a variety of cells can be used according to the purpose for using the peptide of the present invention. The target cells can be, for example, cells in a living body or cultured cells *in vitro*. In addition, these cells can be, for example, cells from animals including human, plant cells, or cells of microorganisms.

Further, the present invention relates to a DNA encoding the peptide of the present invention. There is no particular limitation on the DNA of the present invention, as far as the DNA is capable of encoding the peptide of the present invention. For example, the DNA includes cDNA, genomic DNA, and synthetic DNA. The DNA of the present invention can be prepared, for example, by screening a cDNA library of human fetal brain or a genomic DNA library by utilizing the entire length of any one of the nucleotide sequences of SEQ ID NO: 22-42 and 92-100 or a part thereof as a probe. Alternatively, the DNA of the present invention can also be prepared by polymerase chain reaction using mRNA or cDNA from human fetal brain, or genomic DNA as a template and also using an oligonucleotide specifically hybridizing to the DNA comprising any one of the nucleotide sequences of SEQ ID NO: 22-42 and 92-100 as a primer.

The DNA of the present invention can be utilized, for example, for producing the peptide of the present invention. The production of the peptide is generally conducted by inserting the DNA of the present invention into an appropriate vector; introducing the resulting vector into an appropriate host cell; cultivating the transformant obtained; and purifying the recombinant protein expressed in the transformant. Commonly used expression systems are usable to express the recombinant peptide. For example, the systems can be not only *in vivo* expression systems using *E. coli, Bacillus subtilis*, yeast, insect cells, plant cells, animal cells, and the like but also *in vitro* expression systems using cell extract from *E. coli*, wheat germ, rabbit reticulocyte, etc. The recombinant peptide thus expressed can be purified in high purity, for example, by using a combination of chromatographic steps such as ion exchange, gel filtration, partition, affinity chromatography and others, all of which have been used, or by utilizing HPLC with an appropriate chromatographic carrier.

If the peptide of the present invention becomes visible by modifying the peptide with a suitable substance, the peptide can be utilized as a "nuclear transport indicator substance". Specifically, substance transport from cytoplasm to a nucleus can be monitored in real time with such a modified peptide introduced into cells. Peptide-modifying methods comprise direct labeling of peptide with a fluorescent dye, and preparation of a fusion peptide in which the peptide is fused together with a fluorescent protein such as EGFP or the like. The "nuclear transport indicator substance" can be applied, for example, to a screening system to develop nuclear transport inhibitors, etc.

### Brief Description of the Drawings

Figure 1 illustrates plasmid "pLexAD".
Figure 2 illustrates plasmid "pLexADrev".
Figure 3 illustrates plasmid "pRS1F".
Figure 4 illustrates plasmid "pRS3F".
Figure 5 illustrates assay results showing nuclear transport activity of a transcription factor to be fused with peptides to be tested.
Figure 6 illustrates assay results showing nuclear transport activity of a transcription factor fused with peptides to be tested.
Figure 7 illustrates plasmid "pNS".
Figure 8 is a micrograph showing results obtained in the measurement of nuclear transport activity of the 21 genes isolated by NTT method. The respective numerals in this Figure correspond to order of the gene clones listed in Table 1 and Table 2 (the first gene in Table 1 is indicated as 1 here; the last gene in Table 2 is indicated as 21 here).

### Best Mode for Carrying out the Invention

The present invention is illustrated more specifically below with reference to Examples, but is not to be construed as being limited thereto. Unless otherwise stated, general techniques of genetic engineering used in Examples below are commonly used ones (Sambrook, J., Molecular Cloning: A Laboratory Manual, 1989, 2nd Ed. Cold Spring Harbor Laboratory Press, Cold Spring Harbor, NY.). Products for genetic engineering such as restriction enzymes, modification enzymes, and others were purchased from Takara Shuzo and they were used under recommended conditions as described in the attached manuals. Plasmids were purified from *E. coli* by using a "QIAprep Kit" (QIAGEN). Determination of nucleotide sequences was carried out with an automatic sequencer "ABI PRISM 377" (PERKIN ELMER). Samples to be analyzed were prepared by using reagents provided by the same supplier, and the procedures for using the products were in accordance with the supplier's manuals. Yeast was manipulated (medium, host, shuttle vector, method of introducing genes, method of reporter-gene assay, gene cloning, etc.) by using "MATCHMAKER LexA Two-Hybrid System" (CLONTECH) according to the attached "Yeast Protocols Handbook". Custom-synthesized oligonucleotides were purchased from Toa Gosei.

### Example 1. Preparation of nuclear transport protein-trap vector

### (1) PCR amplification of the DNA sequence encoding GAL4 transcription-activation domain

A DNA fragment containing the GAL4 transcription-activation domain, whose nucleotide sequence was shown in SEQ ID NO: 45, was amplified by PCR using "primer NU13" (SEQ ID NO: 43), at the 5 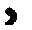 end of which an add-inn EcoRI site was designed, and "MATCHMAKER 3' AD LD-Insert Screening Amplimer" (SEQ ID NO: 44) (CLONTECH) as well as using plasmid "pACT2" (CLONTECH) as a template, with a thermal cycler "GeneAmp PCR System 2400" (PERKIN ELMER). Taq DNA polymerase used was "TaKaRa Ex Taq" (TaKaRa), and the reaction condition was selected according to the product manual. The DNA fragment amplified was purified by ethanol precipitation, and then digested with restriction enzymes, EcoRI and NcoI. Subsequently, the digested DNA was electrophoresed in a 6% polyacrylamide gel, and then the band containing the DNA fragment of interest was excised from the gel; the DNA fragment was recovered by electroelution.

### (2) Preparation of vector "pLexAD" for expressing a fusion protein between LexA protein and GAL4 transcription-activation domain

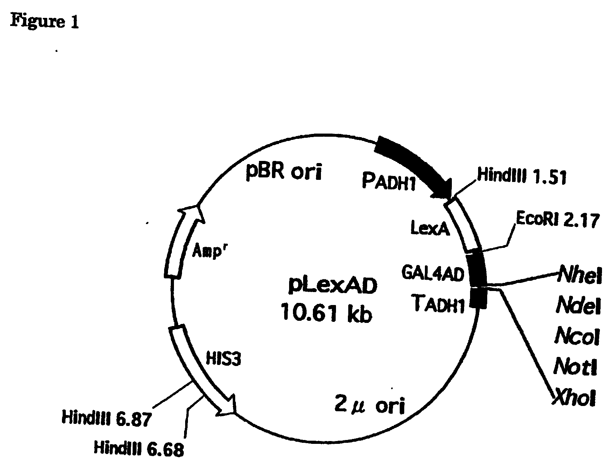

Vector plasmid "pLexAD" was constructed by inserting a DNA fragment encoding the GAL4 transcription-activation domain, as described above in (1), between the EcoRI site and NcoI site within the multi-cloning site of "pLexA" (CLONTECH) (Figure 1). Proper insertion of the target fragment was verified by nucleotide sequencing. The nucleotide sequence of the LexA gene is shown in SEQ ID NO: 46.

### (3) Preparation of vector "pLexADrev", in which a nuclear export signal (NES) is placed at the N-terminus of LexA

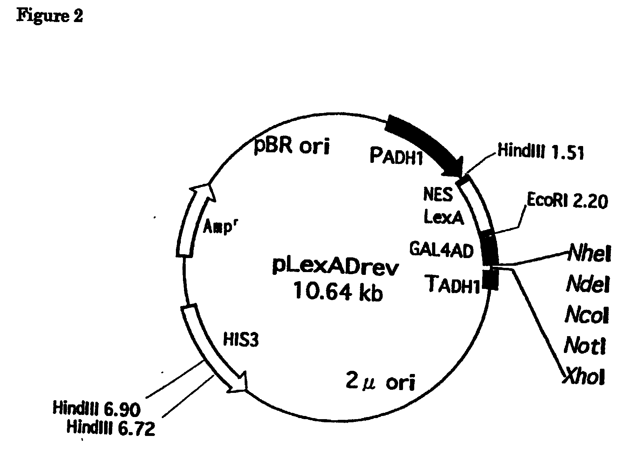

A nuclear export signal (NES) (SEQ ID NO: 47) from Rev protein of HIV was synthesized as described below and was inserted into "pLexAD" at a HpaI site in the vicinity of a portion corresponding to the N-terminus of the encoded LexA protein. Oligonucleotides, "NU9" (SEQ ID NO: 48) as a sense strand and "NU10" (SEQ ID NO: 49) as an antisense strand, were synthesized and then were separately phosphorylated at their 5' ends by using T4 polynucleotide kinase. The oligonucleotides were annealed to each other. The resulting DNA fragment was inserted into "pLexAD" that had been, prior to the insertion, digested with HpaI and then dephosphorylated with alkaline phosphatase. The "pLexADrev" was thus constructed (Figure 2). Proper insertion of the target fragment was verified by nucleotide sequencing.

### (4) Construction of plasmid "pRS1F" containing a CEN/ARS region as a replication origin for expressing a fusion protein between LexA protein and GAL4 transcription-activation domain, and of plasmid "pRS3F" containing a CEN/ARS region as a replication origin for expressing a fusion protein between LexA having an inserted "NES" protein and GAL4 transcription-activation domain

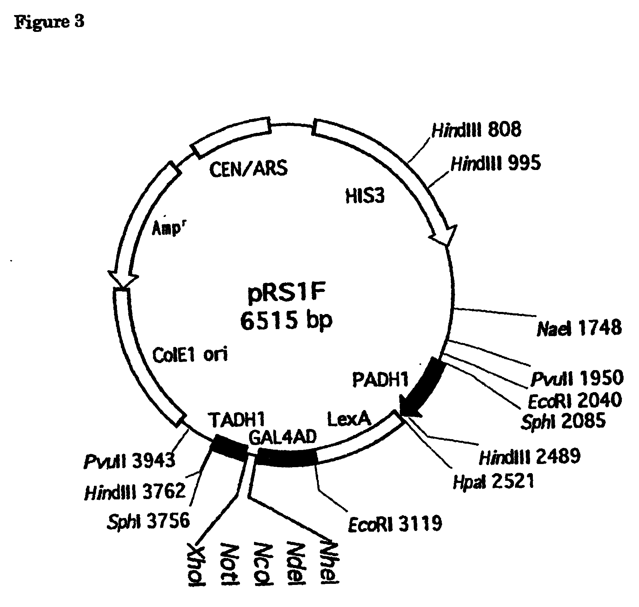

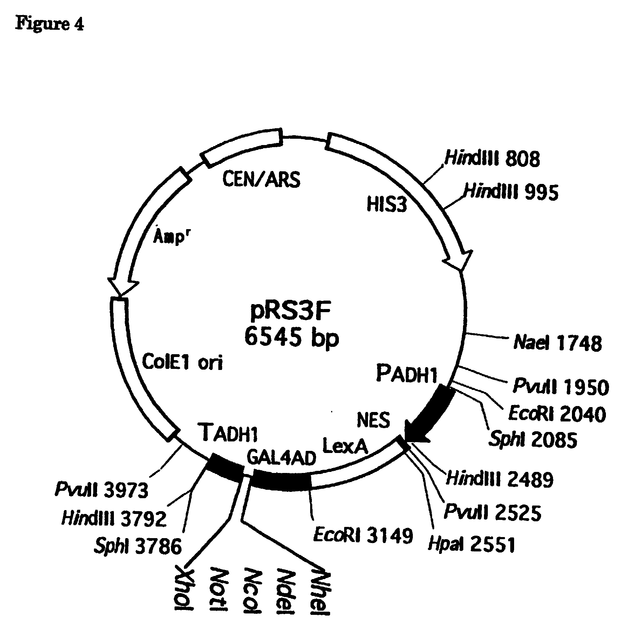

An about 1.7-kb DNA fragment obtained by digesting "pLexAD" with SphI is the minimal unit required for expressing, in yeast, the fusion protein between normal LexA protein having no inserted nuclear export signal (NES) and GAL4 transcription-activation domain. An about 1.7-kb DNA fragment obtained by digesting "pLexADrev" with SphI is the minimal unit required for expressing, in yeast, the fusion protein between LexA protein having a inserted NES at the N-terminus and GAL4 transcription-activation domain, where the nucleotide sequence of the fusion protein is shown in SEQ ID NO: 50 together with the corresponding amino acid sequence. The expression unit contains the ADH1 promoter region, a coding region of protein to be expressed, a multi-cloning site, and the ADH1 terminator region. Each DNA fragment for the expression unit was purified and then inserted into an SphI site of vector "pRSF" to construct "pRS1F" (Figure 3) or "pRS3F" (Figures 4). "pRSF" was constructed by replacing a PvuII-restriction fragment containing the multi-cloning site of plasmid "pRS413" (STRATAGENE) (yeast shuttle vector with a CEN/ARS origin) with a PvuII-restriction fragment containing the multi-cloning site of a widely used plasmid, pUC19. Proper insertion of the target fragment was verified by nucleotide sequencing. Because the constructed plasmids, "pRS1F" (as a positive control) and "pRS3F", have a multi-cloning site derived from pLexA immediately after the coding region of the fusion protein functioning as a transcription factor, a DNA fragment such as a cDNA of interest can readily be fused with the multi-cloning site and expressed according to a usual method.

### Example 2. Demonstration of effectiveness of nuclear transport protein-trap vector "pRS3F" by fusion with cDNA of an artificial nuclear transport protein

### (1) Fusion with a known cDNA fragment

A cDNA fragment encoding an artificial nuclear transport protein in which *Pseudomonas* branched-chain amino acid binding protein ('BraC) from which its secretory signal, known to be localized in the cytoplasm, was removed, where the nucleotide sequence of the 'BraC protein is shown in SEQ ID NO: 51 together with the corresponding amino acid sequence of the protein, (M. Tanaka, Biomembrane and Membrane Transport (2), New Lectures in Biochemical Experiment, volume 6, Ed., Japanese Biochemical Society, 1992, Tokyo Kagaku Doujin, pp. 9-15) was fused at its N-terminus with the nuclear localization signal derived from SV40 large T antigen was used as a known cDNA. fragment. The cDNA fragment was fused in-frame to the nucleotide portion corresponding to the C-terminal end of GAL4 transcription activation domain encoded in the vector "pRS3F". Specifically, prior to the insertion, "pRS3F" was digested with XhoI, blunted with the Klenow enzyme; the resulting DNA was further digested with NcoI and then purified; the DNA fragment (NcoI-DraI) encoding "'BraC" was inserted into the above-treated "pRS3F" to construct "pRS3F'BraC". Further, a synthetic DNA fragment encoding SV40 large T antigen-derived nuclear localization signal (SEQ ID NO: 52), namely, consisting of "NU17" (SEQ ID NO: 53) as a sense strand and "NU18" (SEQ ID NO: 54) as an antisense strand, both of which were phosphorylated at the 5' end with T4 polynucleotide kinase and annealed to each other, was inserted into the NheI/NcoI-predigested and purified vector "pRS3F'BraC", to construct "pRS3FN'BraC". For control experiment, "pRS3FN" having the fragment encoding nuclear localization signal alone but not having "'BraC" fragment was constructed as well. Proper insertion of the target fragment was verified by nucleotide sequencing.

### (2) Assay for nuclear transport activity by utilizing the expression of the reporter gene

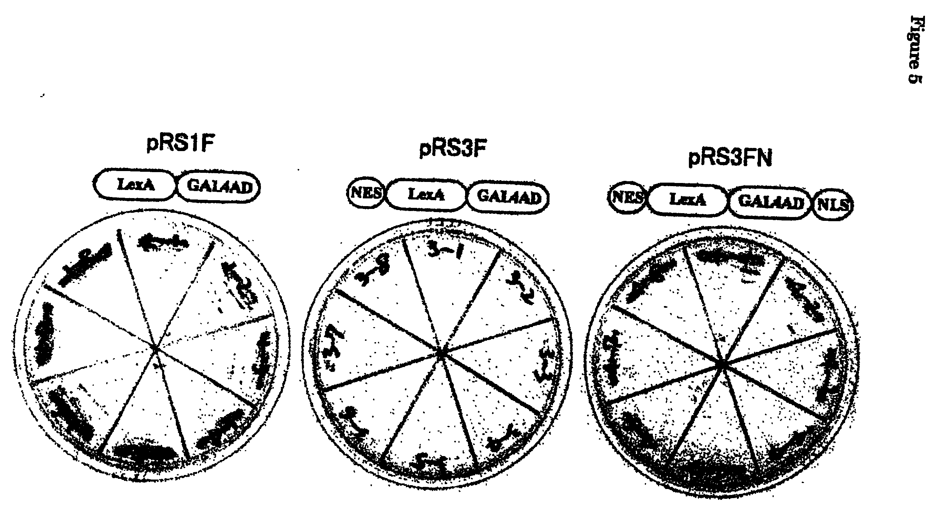

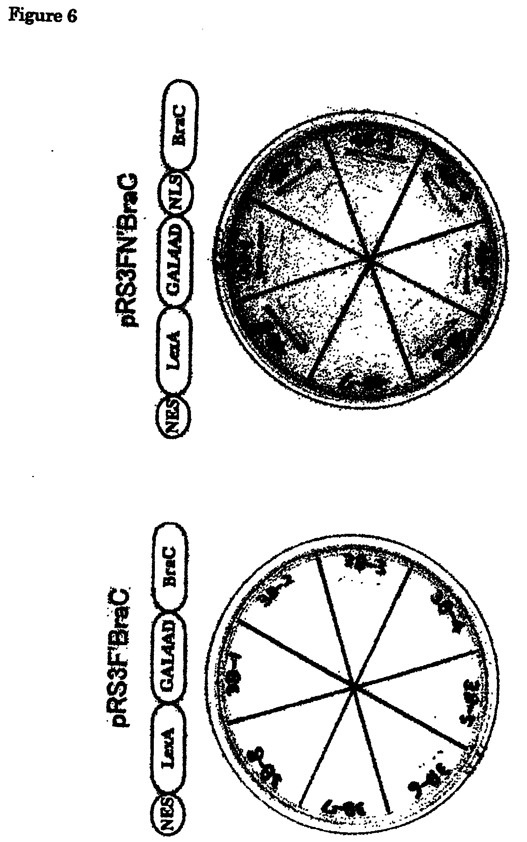

An yeast host EGY48[p8OP-lacZ] (purchased from CLONTECH) contains a promoter region (SEQ ID NO: 55) having the LexA operator sequence (Estojak, J., Mole. Cell. Biol., 1995, 15: 5820-5829) on the chromosome and also contains the downstream reporter genes, LEU2 ανδ β-galactosidase, on a plasmid; the host was transformed with each of the three types of plasmids "pRS3F'BraC", "pRS3FN'BraC", and "pRS3FN", as described in (1) above, as well as "pRS1F" and "pRS3F" constructed in Example 1. Introduction of the plasmid of interest into the host was confirmed by complementation test using an auxotrophic maker, HIS. In the next step, each transformant was inoculated by replica plating on a plate containing medium (SD/-LEU, -HIS, -URA, X-gal) for assaying the expression of the reporter gene, and then incubated at 30°C for 2 to 3 days. The results showed that both β-galactosidase and LEU2 as reporter genes were expressed, exhibiting blue color and normal growth (Figures 5 and 6), when the transformant contained any of introduced plasmids, "pRS3FN'BraC", encoding the fusion protein containing the artificial nuclear transport protein, "pRS3FN", encoding the fusion protein containing nuclear localization signal alone, and "pRS1F", a positive control; on the other hand, the reporter gene was hardly expressed exhibiting no blue color nor growth (Figures 5 and 6), when the transformant contained "pRS3F'BraC", encoding the fusion of protein lacking nuclear localization signal, or "pRS3F" encoding no fusion.

As clearly seen in the above result, a DNA fragment encoding a particular peptide was fused in-frame to an end of "pRS3F", corresponding to the C-terminus of the encoded transcription factor, and the construct was expressed in yeast, thereby conveniently detecting the presence of nuclear transport activity by modifying the expression of the reporter gene.

### Example 3. Construction of vector pNS for preparing cDNA library

The plasmid "pRS3F" was modified for improvement. There were three improvements as follows: (i) an EcoRI site was removed from the junction between LexA and GAL4AD; (ii) an EcoRI site was newly introduced in the multi-cloning site; (iii) unnecessary regions derived from "pRS413" were removed to downsize the plasmid as much as possible.

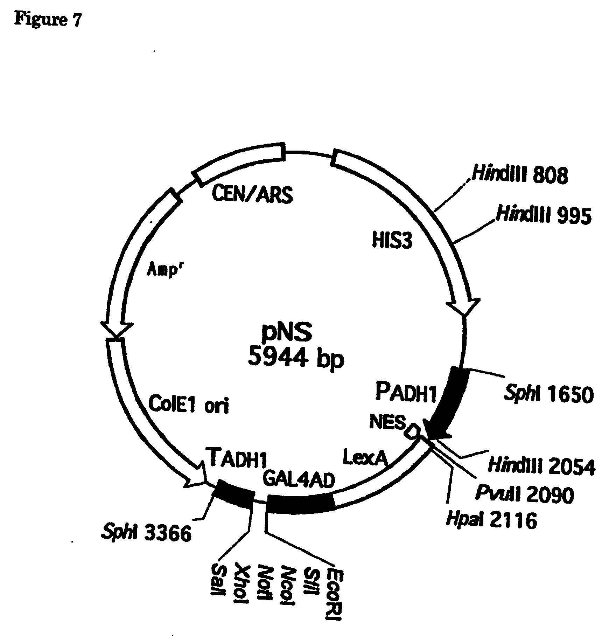

First, a synthetic linker, consisting of "NU31" (SEQ ID NO: 56) as a sense strand and "NU30" (SEQ ID NO: 57) as an antisense strand, was inserted into the EcoRI site of "pLexADrev" to construct plasmid "pLexADrev-dE". An about 1.7-kb DNA fragment containing ADH1 expression unit, which was obtained by digesting the plasmid "pLexADrev-dE" with restriction enzyme SphI, was subcloned into the SphI site of the widely used plasmid pUC19, to prepare plasmid "pULexADrev-dE". In the next step, a synthetic linker with an EcoRI site, which consists of "NU28" (SEQ ID NO: 58) as a sense strand and "NU29" (SEQ ID NO: 59) as an antisense strand, was inserted between the NheI site and NcoI site of "pULexADrev-dE" to construct plasmid "pULexADrev-E". Also, a 757-bp DNA fragment containing a multi-cloning site was removed from "pRS413" by digestion with DraIII and PvuII, and an SphI-site containing synthetic linker, which consists of "NU25" (SEQ ID NO: 60) as a sense strand and "NU26" (SEQ ID NO: 61) as an antisense strand, was inserted thereinto, to construct plasmid "pRS-S". After the above-mentioned "pULexADrev-E" was digested with SphI, the DNA fragment of about 1.7kb, which contains ADH1 expression unit, was inserted into "pRS-S" at the SphI site, thereby achieving the construction of vector pNS (Figure 7) for preparing cDNA library (transcription direction of ADH1 is the same as that of HIS3).

### Example 4. Preparation of fusion protein expression library (derived from human culture cell of NT2 precursor cell) and nuclear transport assay

### (1) Preparation of fusion protein expression library

Human culture cells of NT2 precursor cell (Stratagene) were cultured according to the attached protocol (Catalog #204101, Revision #036002(a)). Messenger RNA was prepared from the cells by using commercially available Total RNA Extraction kit and mRNA Extraction kit (Pharmacia). Complementary DNA library was prepared by using a sample (3 µg) of the mRNA and a commercial cDNA synthesis kit (Pharmacia Co.). Specifically, cDNA synthesized with oligo(dT)12-18 primer was inserted into pNS vector between the EcoRI site and NotI site. Directional Cloning Toolbox (Pharmacia) was utilized for unidirectional insertion of the cDNA. Subsequently, a commercial strain of *E. coli* (ElectroMAX DH10B Cells; Gibco-BRL) was transformed with a sample of the prepared cDNA library by electroporation (Gene Pulser; BIO RAD) according to the usual method (New Experimental protocols for Cell Technology, Shujunsya, pp. 114-115). The resulting transformants were cultured on a LB agar plate containing ampicillin (100 µg/ml) at 30°C for 16 hours; the cells were collected and the plasmid was prepared from the cells by using a QIAGEN Maxi kit (QIAGEN).

### (2) Nuclear transport assay using yeast

Yeast strain EGY48 was transformed with the prepared plasmid (60 µg) of fusion protein expression library according to a usual method (CLONTECH; Yeast Protocols Handbook, PT3024-1: 17-20). By the selection using the expression of the reporter gene LEU, about 1,000 positive clones were yielded on an SD agar plate (-His/-Leu) incubated at 30°C for 3 to 7 days.

### (3) Determination of nucleotide sequence

Sequencing was carried out to determine the nucleotide sequence of an insert DNA in the vector with respect to a part (12 clones) of the resultant positive clones. Prior to the nucleotide sequence determination, a template DNA was prepared from each clone by colony PCR. A small amount of bacterial cells of each clone from the plate was added to 20 µl of PCR reaction mixture (thermostable DNA polymerase (0.5 unit, Ex Taq; TaKaRa), dNTP mixture (4 nmol each), "primer NU15" (SEQ ID NO: 62) and "primer NU36" (SEQ ID NO: 63) (0.4 pmol each), attached buffer (2 µl), and sterilized water). The cDNA insert was amplified with a thermal cycler "GeneAmp PCR System 2400" (PERKIN ELMER) for 40 cycles of denaturation at 94°C, annealing at 60°C, and extension at 72°C. Each of the PCR products was treated with Microcon-100 (Millipore) for desalting and removing unreacted primer molecules; and thus the template DNA was obtained. Nucleotide sequence determination was performed by using a sample (100-200 ng) of the template DNA according to the supplier's manual from ABI.

### (4) Database search for clones isolated

Nucleotide sequence of each clone was searched in a public database of National Center for Biotechnology Information (NCBI) by using Basic BLAST program (http://www.ncbi.nlm.nih.gov/cgi-bin/BLAST/nph-blast?Jform=0). The search revealed that all the 12 clones coincide with known genes. Among them, there are 10 clones that have been reported or suggested to function in the nucleus. Of the ten, 5 clones are NP220 (Inagaki, H., J. Biol. Chem., 1996, 271: 12525-12531), PC4 (Ge, H., Cell, 1994, 78: 513-523), ERC-55 (Imai, T., Biochem. Biophys. Res. Commun., 1997, 233: 765-769), histone-binding protein (O' Rand, M. G., Dev. Biol., 1992, 154: 37-44), and pro-thymosin-α1 (Manrow, R. E., J. Biol. Chem., 1991, 266: 3916-3924), all of which have a sequence similar to SV40 large T antigen-type nuclear localization signal being rich in basic amino acid; and a clone of the ten is hnRNPA1 (Michael, W. M., Cell, 1995, 83: 415-422), which has an M9 sequence responsible for shuttling between the cytoplasm and nucleus. Four clones were identified as ferritin H chain (Cai, C. X., J. Biol. Chem., 1997, 272: 12831-12839), chaperonin 10 (Bonardi, M. A., Biochem. Biophys. Res. Commun., 1995, 206: 260-265), protein kinase C inhibitor-I (Brzoska, P. M., Proc. Natl. Acad. Sci., 1995, 92: 7824-7828), and steroid receptor coactivator 1 (Onate, S. A, Science, 1995, 270: 1354-1357), all of which have no known nuclear localization signal. The remaining 2 clones are tropomyosin (Lin, C. -S., Mol. Cell. Biol., 1988, 8: 160-168) and G-rich sequence factor-1 (Qian, Z., Nucleic Acids Res., 1994, 22: 2334-2343), both of which have no known nuclear localization signal and are so far believed not to function in the nucleus.

### Example 5. Preparation of fusion protein expression library (derived from human fetal brain) and nuclear transport assay

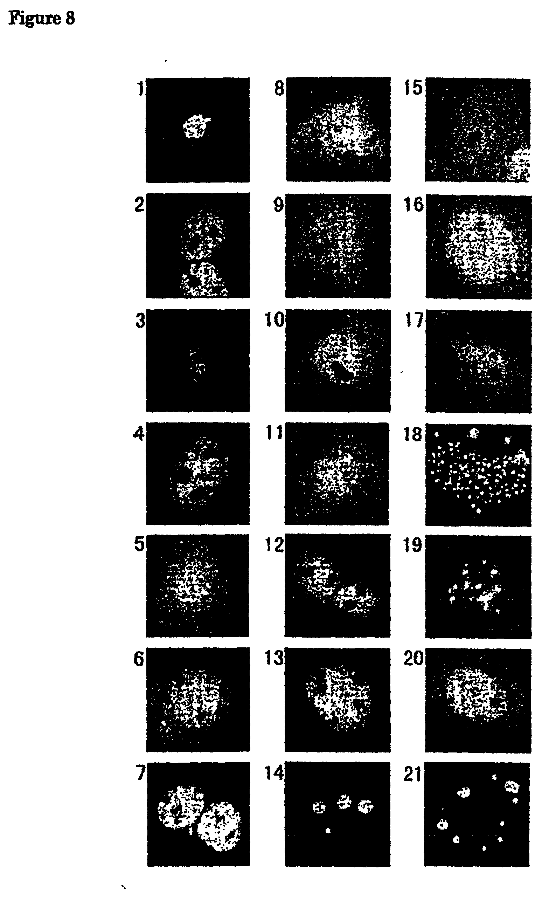

(1) To prepare a fusion protein expression library, first, a commercial human fetal brain cDNA library (SUPERSCRIPT library; Gibco-BRL) was amplified according to the attached protocol, and then plasmid with inserted cDNA fragment was purified by using a plasmid purification kit (QIAGEN). In the next step, a sample (30 µg) of the plasmid was digested with restriction enzymes EcoRI and NotI. The resulting DNA fragments were fractionated on a 0.8% agarose by electrophoresis; cDNA fragments of 0.7kb-4kb were selected and purified. The cDNA fragments obtained were inserted into the above-mentioned pNS vector between the sites of EcoRI and NotI. A commercial strain of *E. coli* (ElectroMAX DH10B Cells; Gibco-BRL) was transformed with a sample of the prepared cDNA library by electroporation (Gene Pulser; BIO RAD) according to a usual method (New Experimental protocols for Cell Technology, Shujunsya, pp. 114-115). The resulting transformants were cultured on a LB agar plate containing ampicillin (100 µg/ml) at 30°C for 16 hours; the cells were collected and the plasmid was prepared from the cells by using a QIAGEN Maxi kit (QIAGEN).
(2) Nuclear transport assay using yeast
   Yeast strain EGY48 was transformed with the prepared plasmid (60 µg) of fusion protein expression library according to a usual method (CLONTECH; Yeast Protocols Handbook, PT3024-1: 17-20). By the selection using the expression of the reporter gene LEU, about 1000 positive clones were yielded on an SD agar plate (-His/-Leu) incubated at 30°C for 3 to 7 days.
(3) Determination of nucleotide sequence
   Sequencing was carried out to determine the nucleotide sequence of an insert DNA the in vector with respect to a part (489 clones) of the resultant positive clones. Prior to the nucleotide sequence determination, a template DNA was prepared from each clone by colony PCR. A small amount of bacterial cells of each clone from the plate was added to 20 µl of PCR reaction mixture (thermostable DNA polymerase (0.5 unit, Ex Taq; TaKaRa), dNTP mixture (4 nmol each), "primer NU15" (SEQ ID NO: 64) and "primer NU36" (SEQ ID NO: 65) (0.4 pmol each), attached buffer (2 µl), and sterilized water). The cDNA insert was amplified with a thermal cycler "GeneAmp PCR System 9600" (PERKIN ELMER) for 40 cycles of denaturation at 94°C, annealing at 60°C and extension at 72°C. Each of the PCR products was treated with Microcon-100 (Millipore) for desalting and removing unreacted primer molecules; and thus the template DNA was obtained. Nucleotide sequence determination was performed by using an sample (100-200 ng) of the template DNA according to the supplier's manual from ABI.
(4) Database search for clones isolated
   Nucleotide sequences of the 489 clone were searched in a public database of National Center for Biotechnology Information (NCBI) by using Basic BLAST program (http://www.ncbi.nlm.nih.gov/cgi-bin/BLAST/nph-blast?Jform=0). By the search, 220 clones were classified into 172 genes with novel sequences or highly homologous sequences to known EST (Expressed Sequence Tag) sequences. These were judged to be candidate genes for novel nuclear transport proteins.
(5) Sequence determination of candidate genes for novel nuclear transport protein
   Out of the 172 genes, which are candidates for gene encoding novel nuclear transport protein, 21 clones were analyzed by nucleotide sequencing (partial sequencing). Determination of nucleotide sequence was carried out by primer walking method using appropriate primers designed according to the supplier's manual provided by ABI.
   The structural features were also analyzed based on the determined sequences. Specifically, (i) when there are 4 or more basic amino acids (lysine or arginine) in a cluster consisting of consecutive 6 amino acids, the cluster is defined as "putative nuclear localization signal (NLS)". Then it was tested whether or not such NLS was present in the amino acid sequence deduced from the nucleotide sequence; (ii) known and typical structural motifs (for example, zinc finger, leucine zipper, and the like), repetitive region, region that is rich in particular amino acids, and the like, were searched in the amino acid sequence; (iii) the probability of nuclear localization was predicted by using "PSORT" program, which is used for predicting intracellular localization of a protein; (iv) prediction of the coil structure in the protein of interest was carried out by using "Coils" program (Lupas, A. Methods Enzymol., 1996, 266:513-525), which is used for predicting coiled-coil structures. These results are shown in Table 1 and Table 2.
   Plating efficiency shown as item a) in these Tables is used as an index of expression level of the reporter gene. When the yeast EGY48 strain is transformed with 100 ng of DNA and directly plated on the medium, the efficiency indicates the number of colonies present on a plate containing SD(-LEU) medium, which is used for detecting the expression of the reporter gene (LEU2). The plating efficiency is considered to reflect not only the level of nuclear transport activity but also partly the ability of transcription activation of the fusion protein in yeast. However, the fused protein could also be toxic in yeast and therefore the plating efficiency is just a rough index. Predicted nuclear localization shown as item b) in the Tables indicates a value (%) for nuclear localization probability predicted by PSORT. 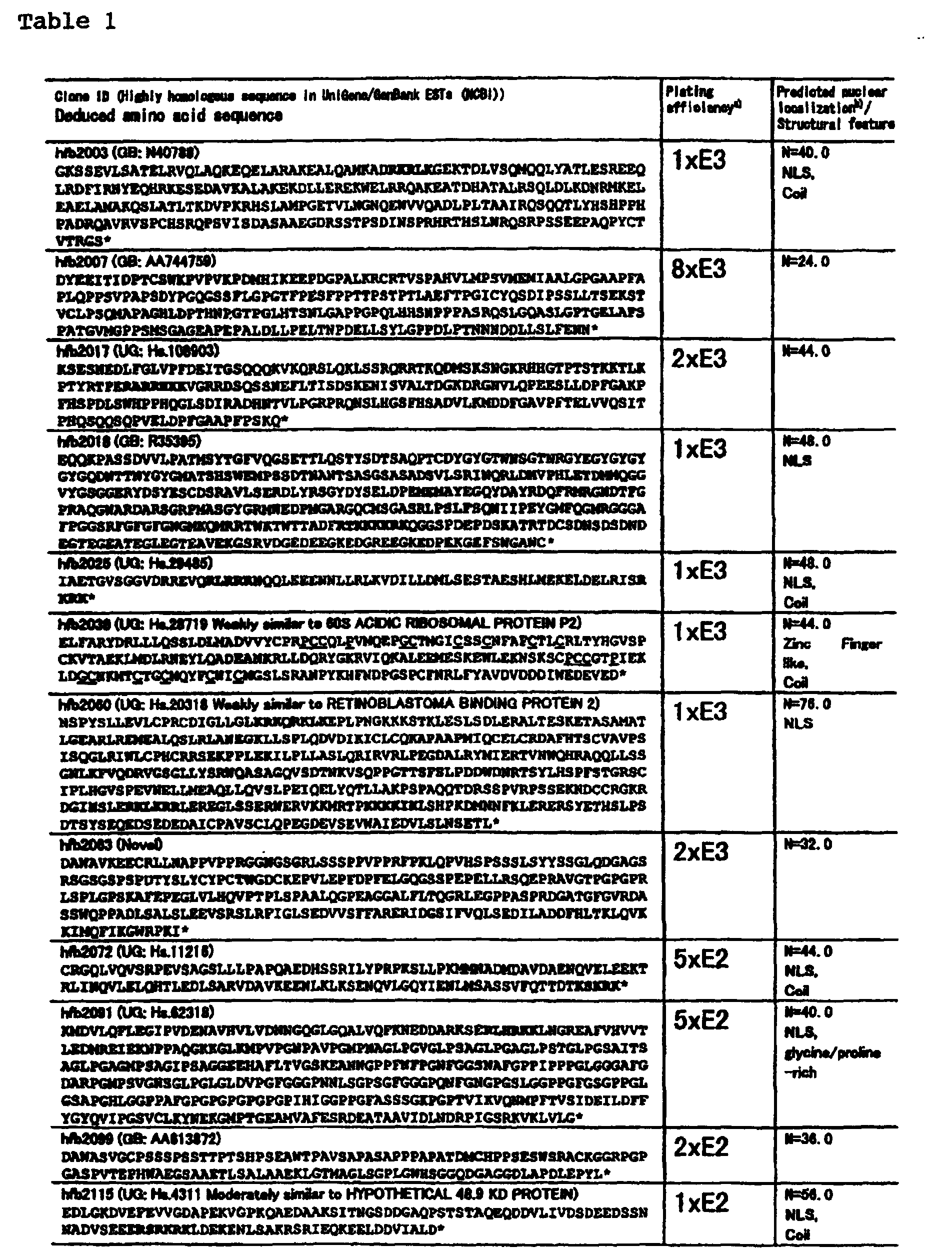 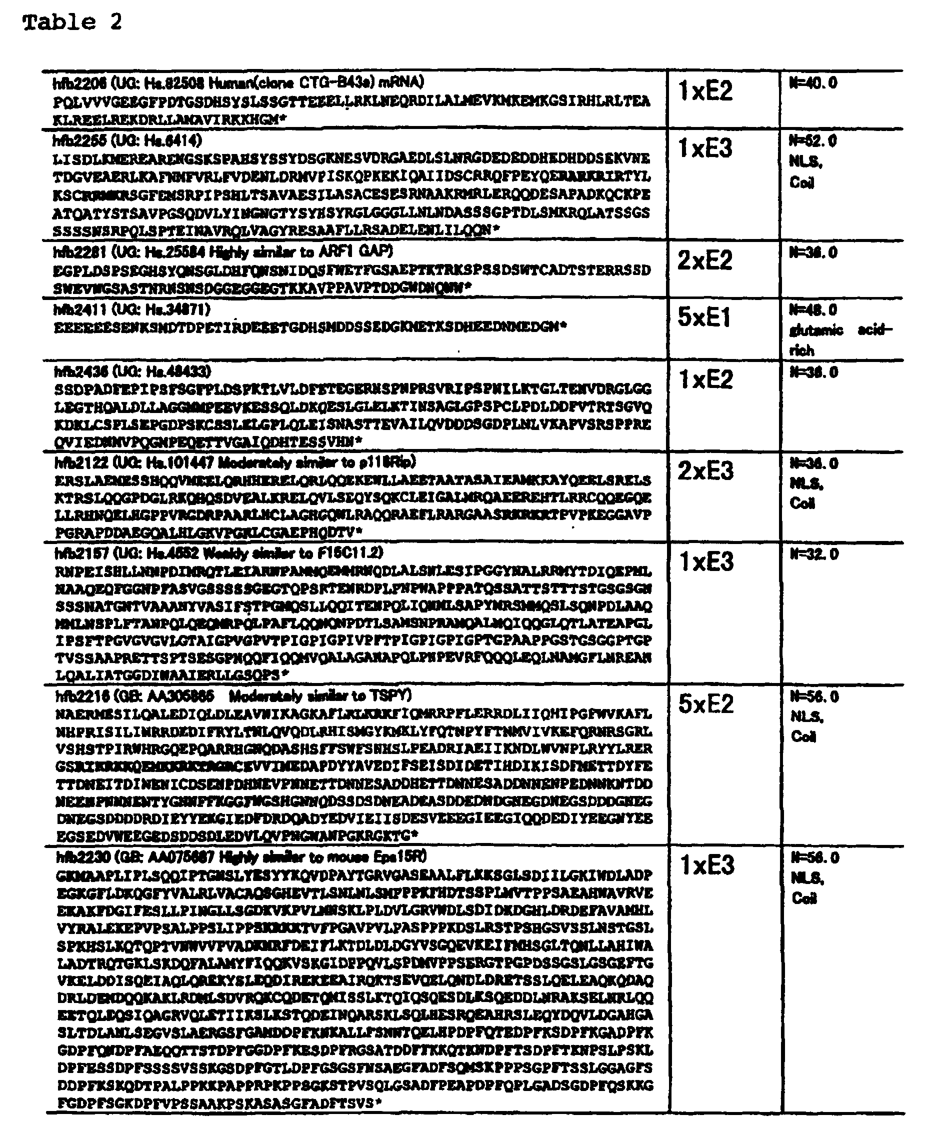
(6) Verification of nuclear transport in a culture cell (COS-7)
   The above-described 21 genes were transiently expressed as fusion proteins with EGFP (Enhanced Green Fluorescent Protein) in culture cells (COS-7), and the localization of the products in the living cells were observed, thereby judging whether or not the each gene obtained encodes a peptide evidently having nuclear transport activity. The result showed that all the fusion proteins are unambiguously localized in the nucleus while the degree of their nuclear accumulations are different to each other (Figure 8).

### Example 6. Isolation of full-length NTT clone

To obtain full-length NTT clones, clones hfb030, hfb044, hfb060, hfb066, hfb101, and hfb341 were isolated in the same manner as described in Example 5. The full-length cDNAs of NTT clones were obtained by using a SuperScript™ Human Fetal Brain cDNA Library (Gibco-BRL) as a cDNA source and by using GENE TRPPER™ cDNA Positive Selection System (Gibco-BRL).

The principle of GENE TRPPER is that a single-stranded cDNA library is hybridized to a biotinylated oligonucleotide specific to a target cDNA and the target cDNA is separated and obtained by utilizing biotin-avidin binding.

### (1) Kits and reagents used are listed below:

- SuperScript™ Human Fetal Brain cDNA Library (Gibco-BRL)
- GENE TRPPER™ cDNA Positive Selection System (Gibco-BRL)
- Attachments to the kit:
   Gene II (used for nicking the sense strand of the f1 intergenic region)
   Exonuclease III
   Biotin-14-dCTP
   Terminal deoxynucleotidyl transferase
   Streptavidin-paramagnetic beads
   Washing buffer
   Elution buffer
   dNTPs
   Repair enzyme
- ElectroMAX DH10B™ Cells (Gibco-BRL)
- Oligonucleotides A,B,C, and D ( The sequences are indicated below.)
- Hybond™-N+ (Amersham Pharmacia Biotech)
- DIG Oligonucleotide Tailing Kit(BOEHRINGER MANNHEIM)
- DIG Easy Hyb (BOEHRINGER MANNHEIM)
- DIG Wash and Block Buffer Set (BOEHRINGER MANNHEIM)
- DIG Nucleic Acid Detection Kit (BOEHRINGER MANNHEIM)
- KOD Dash (TOYOBO)
- QIAprep Spin Miniprep Kit (QIAGEN)
- BigDye Terminator Cycle Sequencing FS Ready Reaction Kit (Perkin-Elmer Applied Biosystems)
- dRhodamine Terminator Cycle Sequencing FS Ready Reaction Kit(Perkin-Elmer Applied Biosystems)
- ABI PRISM 377XL (Perkin-Elmer Applied Biosystems)

### (2) Preparation of single strand of cDNA library

SuperScript™ Human Fetal Brain cDNA Library (Gibco-BRL Co.) was a source of full-length cDNAs to be isolated. By using Gene II, a nick was introduced in the sense strand of the f1 intergenic region in the library vector pCMV SPORT2. The nicked sense strand was digested with Exonuclease III. cDNAs of SuperScript™ Human Fetal Brain cDNA Library are inserted unidirectionally in pCMV SPORT2. Because sense strands of both the f1 intergenic region and the insert cDNA are present in the same strand of the vector, the sense strand is digested by the treatment with Gene II and Exonuclease III; the antisense strand remains as single-stranded, unaffected by the treatment. Thus, the treatment gave a library consisting of single-stranded antisense cDNA. The untreated cDNA library and Gene II-treated cDNA library were compared with each other by agarose gel electrophoresis to verify whether the library became single-stranded.

### (3) Preparation of oligonucleotide

Oligonucleotides A, B, and C, which were specific to the nucleotide sequences of a cDNA fragment cloned by NTT method, were designed. Nucleotide sequences of the cDNA fragments, hfb030, hfb044, htb060, hfb066, hfb101, and hfb341, cloned by NTT method, were determined by 5' one pass sequencing and are shown in SEQ ID NOs: 66-71, respectively (Tables 3 and 4). The oligonucleotides A, B, and C, designed for each clone are shown in Tables 3 and 4 (SEQ ID NOs: 72-89). Oligonucleotide A is marked with a solid underline; oligonuoleotide B is marked with double underlines; oligonucleotide C is marked with a dotted underline. It should be noted that the oligonucleotide C is an antisense oligonucleotide and therefore is an annealing sequence in the cDNA sequence.

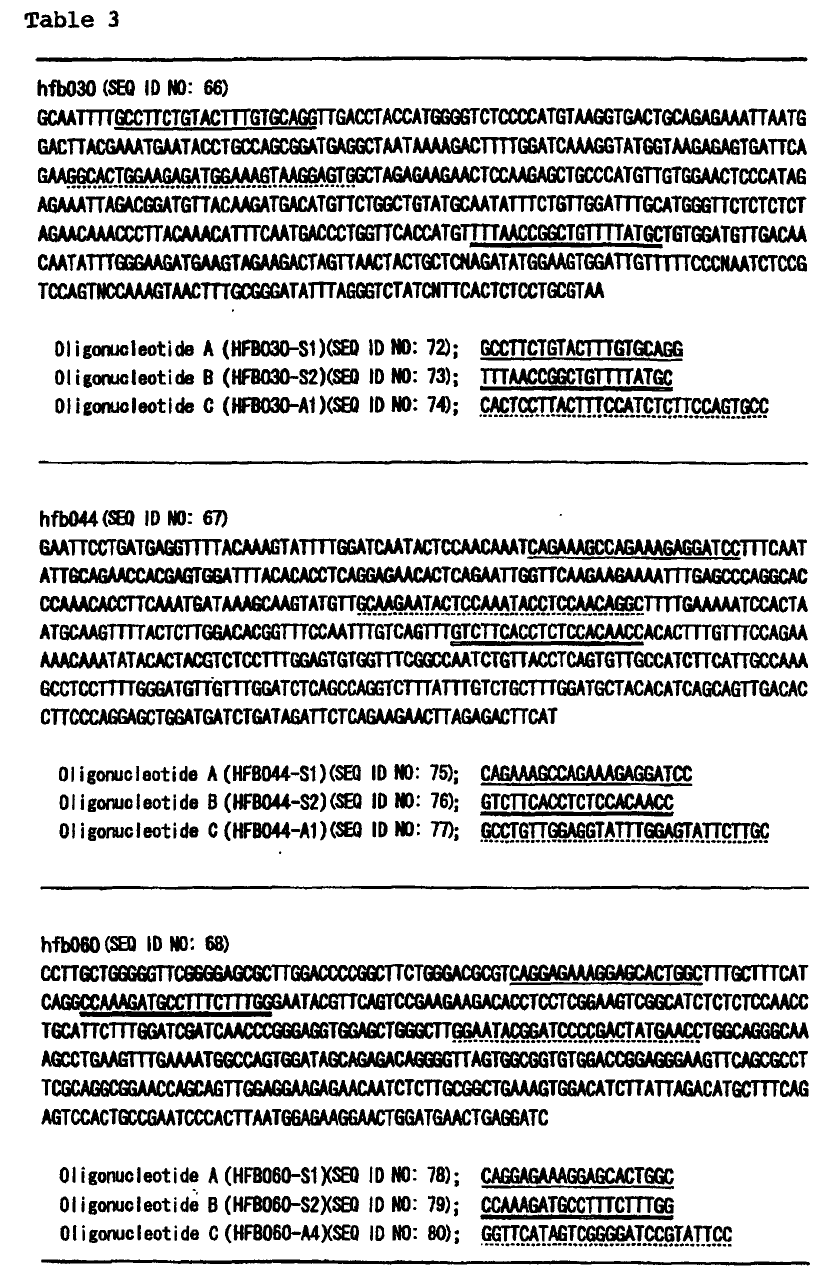

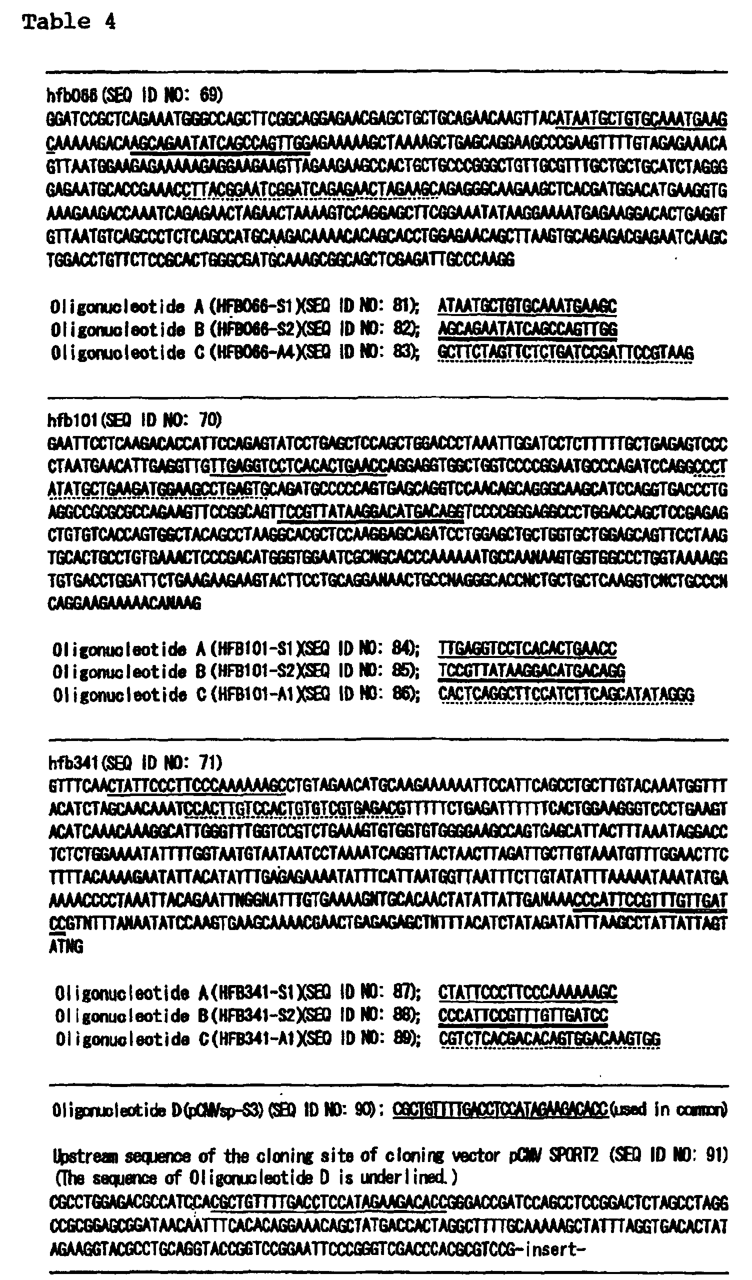

The oligonucleotides A and B are sense strands, and the oligonucleotide C is an antisense strand. To ensure higher stringency, the oligonucleotide A was designed for hybridization and the oligonucleotide B was designed for repairing treatment. Another oligonucleotide C was used for colony hybridization and colony PCR. In addition, sense-strand oligonucleotide D, which was specific to an upstream portion of the cDNA insertion site in pCMV SPORT2, was designed. Nucleotide sequence of the oligonucleotide D is shown in SEQ ID NO: 90; the upstream portion of the cDNA insertion site in pCMV SPORT2, which corresponds to the oligonucleotide D, is shown in SEQ ID NO: 91. The custom-synthesized oligonucleotides were purchased form Toa Gosei.

### (4) Biotinylation of oligonucleotide used for hybridization

Biotin-14-dCTP was added to the 3 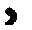 end of the oligonucleotide A by using terminal deoxynucleotidyl transferase, thereby achieving biotin-labeling. The presence of biotin-labels in the oligonucleotide was verified by polyacrylamide gel electrophoresis.

### (5) Hybridization and separation of target cDNA library

The single-stranded cDNA library prepared in step (2) was hybridized to the biotinylated oligonucleotide A prepared in step (4). Streptavidin-paramagnetic beads were added in the hybridization solution, and then the supernatant was discarded while the beads were magnetically fixed by using a stand with a magnet; unhybridized cDNAs were, thus, removed. The beads were washed several times with washing buffer contained in the kit, and then target cDNA library hybridized on the beads was eluted with elution buffer.

### (6) Repair as double strand and cloning

The eluted cDNA was repaired as a double-stranded cDNA library by using the oligonucleotide B as a primer and also using dNTPs and repair enzyme. The double-stranded cDNA library repaired was introduced into ElectroMAX DH10B™ Cells (Gibco-BRL) by electroporation, and the transformed cells were plated on an agar plate to form colonies. The presence of the target cDNA was judged by colony hybridization as described in (7) or colony PCR in (8).

### (7) Colony hybridization

The colonies on the agar plate were transferred onto a nylon membrane of Hybond™-N+ (Amersham Pharmacia Biotech), and colony hybridization was carried out with DIG-labeled oligonucleotide C. DIG-labeling of the oligonucleotide C was performed by using a DIG Oligonucleotide Tailing Kit (BOEHRINGER MANNHEIM). Hybridization was conducted by using DIG Easy Hyb (BOEHRINGER MANNHEIM). Detection was achieved by using DIG Wash and Block Buffer Set (BOEHRINGER MANNHEIM) and a DIG Nucleic Acid Detection Kit (BOEHRINGER MANNHEIM).

### (8) Colony PCR

PCR amplification was performed by using as a template each colony formed on the agar plate and using KOD Dash (TOYOBO). Primers employed were the target cDNA-specific oligonucleotide C and cloning vector-specific oligonucleotide D. The resulting PCR reaction solution was analyzed by agarose electrophoresis to confirm the presence of the band of interest.

### (9) Determination of nucleotide sequence

The bacteria from colony containing cDNA of interest, of which presence was confirmed by the method as described in (7) or (8), were cultured, and the plasmid was extracted from the bacterial cells to determine its nucleotide sequence. Plasmid extraction was carried out by using a QIAprep Spin Miniprep Kit (QIAGEN). Nucleotide sequence was determined with an automatic sequencer, ABI PRISM 377XL (Perkin-Elmer Applied Biosystems) by using a BigDye Terminator Cycle Sequencing FS Ready Reaction Kit (Perkin-Elmer Applied Biosystems) or dRhodamine Terminator Cycle Sequencing FS Ready Reaction Kit (Perkin-Elmer Applied Biosystems).

Respective nucleotide sequences corresponding to the following clones isolated are shown in SEQ ID NOs: 92-100: the clones are hfb030 (3092bp; deduced coding region, 237-1661), hfb044-1 (full-length; 3041bp; deduced coding region, 48-2735), hfb044-2 (full-length; 3060bp; deduced coding region, 69-2756), hfb060-1, hfb060-2, and hfb066 (full-length or a part of a coding region), hfb101-1 (full-length; 2565bp; deduced coding region, 474-2120), hfb101-2 (full-length; 2694bp; deduced coding region; 402-2267), hfb341 (full-length; 6193bp; deduced coding region, 466-3873). Deduced amino acid sequences (HFB030, 474 amino acids; HFB044-1, 895 amino acids; HFB044-2, 895 amino acids; HFB066 and HFB101-1, 548 amino acids; HFB101-2, 621 amino acids; and HFB341, 1135 amino acids) of proteins encoded by these clones, except for hfb060-1 and hfb060-2, are shown in SEQ ID NO: 101-107.

### Industrial Applicability

The present invention provides a peptide having nuclear transport activity. The peptide of the present invention is useful as a carrier for transporting desired substances into a nucleus. Further, when visualized by modifying with an appropriate substance, the peptide of the present invention is usable as a nuclear transport indicator substance.

## Claims

1. A peptide comprising any one of the amino acid sequences set forth in SEQ ID NOs: 1-21 and 101-107 and having nuclear transport activity.

2. A peptide comprising any one of the amino acid sequences set forth in SEQ ID NOs: 1-21 and 101-107, in which one or more amino acids are substituted, deleted, and/or added, and having nuclear transport activity.

3. The peptide of claim 1 or 2, wherein the peptide is a fusion peptide comprising a desired peptide to be transported to a nucleus.

4. A DNA encoding the peptide of any one of claims 1 to 3.

5. A vector comprising the DNA of claim 4.

6. A transformant carrying the vector of claim 5.

7. A method for producing the peptide of claim 1 or 2, comprising culturing the transformant of claim 6.


SEQUENCE LISTING

<110> Helix Research Institute

<120> Peptides having nuclear transport activity

<130> H1-805DP2PCT

<140>
<141>

<150> JP 1998-174065
      JP 1999-107262
<151> 1998-06-05
      1999-04-14

<160> 107

<170> PatentIn Ver. 2.0

<210> 1
<211> 261
<212> PRT
<213> Homo sapiens

<220>
<223> clone name: hfb2003

<400> 1
Gly Lys Ser Ser Glu Val Leu Ser Ala Thr Glu Leu Arg Val Gln Leu
  1               5                  10                  15

Ala Gln Lys Glu Gln Glu Leu Ala Arg Ala Lys Glu Ala Leu Gln Ala
             20                  25                  30

Met Lys Ala Asp Arg Lys Arg Leu Lys Gly Glu Lys Thr Asp Leu Val
         35                  40                  45

Ser Gln Met Gln Gln Leu Tyr Ala Thr Leu Glu Ser Arg Glu Glu Gln
     50                  55                  60

Leu Arg Asp Phe Ile Arg Asn Tyr Glu Gln His Arg Lys Glu Ser Glu
65                  70                  75                  80

Asp Ala Val Lys Ala Leu Ala Lys Glu Lys Asp Leu Leu Glu Arg Glu
                85                  90                  95

Lys Trp Glu Leu Arg Arg Gln Ala Lys Glu Ala Thr Asp His Ala Thr
            100                 105                 110

Ala Leu Arg Ser Gln Leu Asp Leu Lys Asp Asn Arg Met Lys Glu Leu
        115                 120                 125

Glu Ala Glu Leu Ala Met Ala Lys Gln Ser Leu Ala Thr Leu Thr Lys
    130                 135                 140

Asp Val Pro Lys Arg His Ser Leu Ala Met Pro Gly Glu Thr Val Leu
145                 150                 155                 160

Asn Gly Asn Gln Glu Trp Val Val Gln Ala Asp Leu Pro Leu Thr Ala
                165                 170                 175

Ala Ile Arg Gln Ser Gln Gln Thr Leu Tyr His Ser His Pro Pro His
            180                 185                 190

Pro Ala Asp Arg Gln Ala Val Arg Val Ser Pro Cys His Ser Arg Gln
        195                 200                 205

Pro Ser Val Ile Ser Asp Ala Ser Ala Ala Glu Gly Asp Arg Ser Ser
    210                 215                 220

Thr Pro Ser Asp Ile Asn Ser Pro Arg His Arg Thr His Ser Leu Trp
225                 230                 235                 240

Arg Gln Ser Arg Pro Ser Ser Glu Glu Pro Ala Gln Pro Tyr Cys Thr
                245                 250                 255

Val Thr Arg Gly Ser
            260

<210> 2
<211> 253
<212> PRT
<213> Homo sapiens

<220>
<223> clone name: hfb2007

<400> 2
Asp Tyr Glu Glu Ile Thr Ile Asp Pro Thr Cys Ser Trp Lys Pro Val
1               5                   10                  15

Pro Val Lys Pro Asp Met His Ile Lys Glu Glu Pro Asp Gly Pro Ala
            20                  25                  30

Leu Lys Arg Cys Arg Thr Val Ser Pro Ala His Val Leu Met Pro Ser
        35                  40                  45

Val Met Glu Met Ile Ala Ala Leu Gly Pro Gly Ala Ala Pro Phe Ala
    50                  55                  60

Pro Leu Gln Pro Pro Ser Val Pro Ala Pro Ser Asp Tyr Pro Gly Gln
65                  70                  75                  80

Gly Ser Ser Phe Leu Gly Pro Gly Thr Phe Pro Glu Ser Phe Pro Pro
                85                  90                  95

Thr Thr Pro Ser Thr Pro Thr Leu Ala Glu Phe Thr Pro Gly Pro Pro
            100                 105                 110

Pro Ile Cys Tyr Gln Ser Asp Ile Pro Ser Ser Leu Leu Thr Ser Glu
        115                 120                 125

Lys Ser Thr Val Cys Leu Pro Ser Gln Met Ala Pro Ala Gly His Leu
    130                 135                 140

Asp Pro Thr His Asn Pro Gly Thr Pro Gly Leu His Thr Ser Asn Leu
145                 150                 155                 160

Gly Ala Pro Pro Gly Pro Gln Leu His His Ser Asn Pro Pro Pro Ala
                165                 170                 175

Ser Arg Gln Ser Leu Gly Gln Ala Ser Leu Gly Pro Thr Gly Glu Leu
            180                 185                 190

Ala Phe Ser Pro Ala Thr Gly Val Met Gly Pro Pro Ser Met Ser Gly
        195                 200                 205

Ala Gly Glu Ala Pro Glu Pro Ala Leu Asp Leu Leu Pro Glu Leu Thr
    210                 215                 220

Asn Pro Asp Glu Leu Leu Ser Tyr Leu Gly Pro Pro Asp Leu Pro Thr
225                 230                 235                 240

Asn Asn Asn Asp Asp Leu Leu Ser Leu Phe Glu Asn Asn
                245                 250

<210> 3
<211> 216
<212> PRT
<213> Homo sapiens

<220>
<223> clone name: hfb2017

<400> 3
Lys Ser Glu Ser Asn Glu Asp Leu Phe Gly Leu Val Pro Phe Asp Glu
1               5                   10                  15

Ile Thr Gly Ser Gln Gln Gln Lys Val Lys Gln Arg Ser Leu Gln Lys
            20                  25                  30

Leu Ser Ser Arg Gln Arg Arg Thr Lys Gln Asp Met Ser Lys Ser Asn
        35                  40                  45

Gly Lys Arg His His Gly Thr Pro Thr Ser Thr Lys Lys Thr Leu Lys
    50                  55                  60

Pro Thr Tyr Arg Thr Pro Glu Arg Ala Arg Arg His Lys Lys Val Gly
65                  70                  75                  80

Arg Arg Asp Ser Gln Ser Ser Asn Glu Phe Leu Thr Ile Ser Asp Ser
                85                  90                  95

Lys Glu Asn Ile Ser Val Ala Leu Thr Asp Gly Lys Asp Arg Gly Asn
            100                 105                 110

Val Leu Gln Pro Glu Glu Ser Leu Leu Asp Pro Phe Gly Ala Lys Pro
        115                 120                 125

Phe His Ser Pro Asp Leu Ser Trp His Pro Pro His Gln Gly Leu Ser
    130                 135                 140

Asp Ile Arg Ala Asp His Asn Thr Val Leu Pro Gly Arg Pro Arg Gln
145                 150                 155                 160

Asn Ser Leu His Gly Ser Phe His Ser Ala Asp Val Leu Lys Met Asp
                165                 170                 175

Asp Phe Gly Ala Val Pro Phe Thr Glu Leu Val Val Gln Ser Ile Thr
            180                 185                 190

Pro His Gln Ser Gln Gln Ser Gln Pro Val Glu Leu Asp Pro Phe Gly
        195                 200                 205

Ala Ala Pro Phe Pro Ser Lys Gln
    210                 215

<210> 4
<211> 373
<212> PRT
<213> Homo sapiens

<220>
<223> clone name: hfb2018

<400> 4

Glu Gln Gln Lys Pro Ala Ser Ser Asp Val Val Leu Pro Ala Thr Met
1               5                   10                  15

Ser Tyr Thr Gly Phe Val Gln Gly Ser Glu Thr Thr Leu Gln Ser Thr
            20                  25                  30

Tyr Ser Asp Thr Ser Ala Gln Pro Thr Cys Asp Tyr Gly Tyr Gly Thr
        35                  40                  45

Trp Asn Ser Gly Thr Asn Arg Gly Tyr Glu Gly Tyr Gly Tyr Gly Tyr
    50                  55                  60

Gly Tyr Gly Gln Asp Asn Thr Thr Asn Tyr Gly Tyr Gly Met Ala Thr
65                  70                  75                  80

Ser His Ser Trp Glu Met Pro Ser Ser Asp Thr Asn Ala Asn Thr Ser
                85                  90                  95

Ala Ser Gly Ser Ala Ser Ala Asp Ser Val Leu Ser Arg Ile Asn Gln
            100                 105                 110

Arg Leu Asp Met Val Pro His Leu Glu Thr Asp Met Met Gln Gly Gly
        115                 120                 125

Val Tyr Gly Ser Gly Gly Glu Arg Tyr Asp Ser Tyr Glu Ser Cys Asp
    130                 135                 140

Ser Arg Ala Val Leu Ser Glu Arg Asp Leu Tyr Arg Ser Gly Tyr Asp
145                 150                 155                 160

Tyr Ser Glu Leu Asp Pro Glu Met Glu Met Ala Tyr Glu Gly Gln Tyr
                165                 170                 175

Asp Ala Tyr Arg Asp Gln Phe Arg Met Arg Gly Asn Asp Thr Phe Gly
            180                 185                 190

Pro Arg Ala Gln Gly Trp Ala Arg Asp Ala Arg Ser Gly Arg Pro Met
        195                 200                 205

Ala Ser Gly Tyr Gly Arg Met Trp Glu Asp Pro Met Gly Ala Arg Gly

210                 215                 220

Gln Cys Met Ser Gly Ala Ser Arg Leu Pro Ser Leu Phe Ser Gln Asn
225                 230                 235                 240

Ile Ile Pro Glu Tyr Gly Met Phe Gln Gly Met Arg Gly Gly Gly Ala
                245                 250                 255

Phe Pro Gly Gly Ser Arg Phe Gly Phe Gly Phe Gly Asn Gly Met Lys
            260                 265                 270

Gln Met Arg Arg Thr Trp Lys Thr Trp Thr Thr Ala Asp Phe Arg Thr
        275                 280                 285

Lys Lys Lys Lys Arg Lys Gln Gly Gly Ser Pro Asp Glu Pro Asp Ser
    290                 295                 300

Lys Ala Thr Arg Thr Asp Cys Ser Asp Asn Ser Asp Ser Asp Asn Asp
305                 310                 315                 320

Glu Gly Thr Glu Gly Glu Ala Thr Glu Gly Leu Glu Gly Thr Glu Ala
                325                 330                 335

Val Glu Lys Gly Ser Arg Val Asp Gly Glu Asp Glu Glu Gly Lys Glu
            340                 345                 350

Asp Gly Arg Glu Glu Gly Lys Glu Asp Pro Glu Lys Gly Glu Phe Ser
        355                 360                 365

Trp Gly Ala Trp Cys
    370

<210> 5
<211> 189
<212> PRT
<213> Homo sapiens

<220>
<223> clone name: hfb2038

<400> 5
Glu Leu Phe Ala Arg Tyr Asp Arg Leu Leu Leu Gln Ser Ser Leu Asp
1               5                   10                  15

Leu Met Ala Asp Val Val Tyr Cys Pro Arg Pro Cys Cys Gln Leu Pro
            20                  25                  30

Val Met Gln Glu Pro Gly Cys Thr Met Gly Ile Cys Ser Ser Cys Asn
        35                  40                  45

Phe Ala Phe Cys Thr Leu Cys Arg Leu Thr Tyr His Gly Val Ser Pro
    50                  55                  60

Cys Lys Val Thr Ala Glu Lys Leu Met Asp Leu Arg Asn Glu Tyr Leu
65                  70                  75                  80

Gln Ala Asp Glu Ala Asn Lys Arg Leu Leu Asp Gln Arg Tyr Gly Lys
                85                  90                  95

Arg Val Ile Gln Lys Ala Leu Glu Glu Met Glu Ser Lys Glu Trp Leu
            100                 105                 110

Glu Lys Asn Ser Lys Ser Cys Pro Cys Cys Gly Thr Pro Ile Glu Lys
        115                 120                 125

Leu Asp Gly Cys Asn Lys Met Thr Cys Thr Gly Cys Met Gln Tyr Phe
    130                 135                 140

Cys Trp Ile Cys Met Gly Ser Leu Ser Arg Ala Asn Pro Tyr Lys His
145                 150                 155                 160

Phe Asn Asp Pro Gly Ser Pro Cys Phe Asn Arg Leu Phe Tyr Ala Val
                165                 170                 175

Asp Val Asp Asp Asp Ile Trp Glu Asp Glu Val Glu Asp
            180                 185

<210> 6

<211> 123
<212> PRT
<213> Homo sapiens

<220>
<223> clone name: hfb2099

<400> 6


<210> 7
<211> 125
<212> PRT
<213> Homo sapiens

<220>
<223> clone name: hfb2072

<400> 7
Cys Arg Gly Gln Leu Val Gln Val Ser Arg Pro Glu Val Ser Ala Gly
1               5                   10                  15

Ser Leu Leu Leu Pro Ala Pro Gln Ala Glu Asp His Ser Ser Arg Ile
            20                  25                  30

Leu Tyr Pro Arg Pro Lys Ser Leu Leu Pro Lys Met Met Asn Ala Asp
        35                  40                  45

Met Asp Ala Val Asp Ala Glu Asn Gln Val Glu Leu Glu Glu Lys Thr
    50                  55                  60

Arg Leu Ile Asn Gln Val Leu Glu Leu Gln His Thr Leu Glu Asp Leu
65                  70                  75                  80

Ser Ala Arg Val Asp Ala Val Lys Glu Glu Asn Leu Lys Leu Lys Ser
                85                  90                  95

Glu Asn Gln Val Leu Gly Gln Tyr Ile Glu Asn Leu Met Ser Ala Ser
            100                 105                 110

Ser Val Phe Gln Thr Thr Asp Thr Lys Ser Lys Arg Lys
        115                 120                 125


<210> 8
<211> 376
<212> PRT
<213> Homo sapiens

<220>
<223> clone name: hfb2091

<400> 8
Lys Met Asp Val Leu Gln Phe Leu Glu Gly Ile Pro Val Asp Glu Asn
1               5                   10                  15

Ala Val His Val Leu Val Asp Asn Asn Gly Gln Gly Leu Gly Gln Ala

            20                  25                  30

Leu Val Gln Phe Lys Asn Glu Asp Asp Ala Arg Lys Ser Glu Arg Leu
        35                  40                  45

His Arg Lys Lys Leu Asn Gly Arg Glu Ala Phe Val His Val Val Thr
    50                  55                  60

Leu Glu Asp Met Arg Glu Ile Glu Lys Asn Pro Pro Ala Gln Gly Lys
65                  70                  75                  80

Lys Gly Leu Lys Met Pro Val Pro Gly Asn Pro Ala Val Pro Gly Met
                85                  90                  95

Pro Asn Ala Gly Leu Pro Gly Val Gly Leu Pro Ser Ala Gly Leu Pro
            100                 105                 110

Gly Ala Gly Leu Pro Ser Thr Gly Leu Pro Gly Ser Ala Ile Thr Ser
        115                 120                 125

Ala Gly Leu Pro Gly Ala Gly Met Pro Ser Ala Gly Ile Pro Ser Ala
    130                 135                 140

Gly Gly Glu Glu His Ala Phe Leu Thr Val Gly Ser Lys Glu Ala Asn
145                 150                 155                 160

Asn Gly Pro Pro Phe Asn Phe Pro Gly Asn Phe Gly Gly Ser Asn Ala
                165                 170                 175

Phe Gly Pro Pro Ile Pro Pro Pro Gly Leu Gly Gly Gly Ala Phe Gly
            180                 185                 190

Asp Ala Arg Pro Gly Met Pro Ser Val Gly Asn Ser Gly Leu Pro Gly
        195                 200                 205

Leu Gly Leu Asp Val Pro Gly Phe Gly Gly Gly Pro Asn Asn Leu Ser
    210                 215                 220

Gly Pro Ser Gly Phe Gly Gly Gly Pro Gln Asn Phe Gly Asn Gly Pro
225                 230                 235                 240

Gly Ser Leu Gly Gly Pro Pro Gly Phe Gly Ser Gly Pro Pro Gly Leu
                245                 250                 255

Gly Ser Ala Pro Gly His Leu Gly Gly Pro Pro Ala Phe Gly Pro Gly
            260                 265                 270

Pro Gly Pro Gly Pro Gly Pro Gly Pro Ile His Ile Gly Gly Pro Pro
        275                 280                 285

Gly Phe Ala Ser Ser Ser Gly Lys Pro Gly Pro Thr Val Ile Lys Val
    290                 295                 300

Gln Asn Met Pro Phe Thr Val Ser Ile Asp Glu Ile Leu Asp Phe Phe
305                 310                 315                 320

Tyr Gly Tyr Gln Val Ile Pro Gly Ser Val Cys Leu Lys Tyr Asn Glu
                325                 330                 335

Lys Gly Met Pro Thr Gly Glu Ala Met Val Ala Phe Glu Ser Arg Asp
            340                 345                 350

Glu Ala Thr Ala Ala Val Ile Asp Leu Asn Asp Arg Pro Ile Gly Ser
        355                 360                 365

Arg Lys Val Lys Leu Val Leu Gly
    370                 375

<210> 9
<211> 105
<212> PRT
<213> Homo sapiens

<220>
<223> clone name: hfb2115

<400> 9
Glu Asp Leu Gly Lys Asp Val Glu Phe Glu Val Val Gly Asp Ala Pro
  1               5                  10                  15

Glu Lys Val Gly Pro Lys Gln Ala Glu Asp Ala Ala Lys Ser Ile Thr
            20                  25                  30

Asn Gly Ser Asp Asp Gly Ala Gln Pro Ser Thr Ser Thr Ala Gln Glu
        35                  40                  45

Gln Asp Asp Val Leu Ile Val Asp Ser Asp Glu Glu Asp Ser Ser Asn
    50                  55                  60

Asn Ala Asp Val Ser Glu Glu Glu Arg Ser Arg Lys Arg Lys Leu Asp
65                  70                  75                  80

Glu Lys Glu Asn Leu Ser Ala Lys Arg Ser Arg Ile Glu Gln Lys Glu
                85                  90                  95

Glu Leu Asp Asp Val Ile Ala Leu Asp
            100                 105

<210> 10
<211> 88
<212> PRT
<213> Homo sapiens

<220>
<223> clone name: hfb2206

<400> 10
Pro Gln Leu Val Val Val Gly Glu Glu Gly Phe Pro Asp Thr Gly Ser
1               5                   10                  15

Asp His Ser Tyr Ser Leu Ser Ser Gly Thr Thr Glu Glu Glu Leu Leu
            20                  25                  30

Arg Lys Leu Asn Glu Gln Arg Asp Ile Leu Ala Leu Met Glu Val Lys
        35                  40                  45

Met Lys Glu Met Lys Gly Ser Ile Arg His Leu Arg Leu Thr Glu Ala
    50                  55                  60

Lys Leu Arg Glu Glu Leu Arg Glu Lys Asp Arg Leu Leu Ala Met Ala
65                  70                  75                  80

Val Ile Arg Lys Lys His Gly Met
                85

<210> 11
<211> 303
<212> PRT
<213> Homo sapiens

<220>
<223> clone name: hfb2255

<400> 11
Leu Ile Ser Asp Leu Lys Met Glu Arg Glu Ala Arg Glu Asn Gly Ser
1               5                   10                  15

Lys Ser Pro Ala His Ser Tyr Ser Ser Tyr Asp Ser Gly Lys Asn Glu
            20                  25                  30

Ser Val Asp Arg Gly Ala Glu Asp Leu Ser Leu Asn Arg Gly Asp Glu
        35                  40                  45

Asp Glu Asp Asp His Glu Asp His Asp Asp Ser Glu Lys Val Asn Glu
    50                  55                  60

Thr Asp Gly Val Glu Ala Glu Arg Leu Lys Ala Phe Asn Met Phe Val
65                  70                  75                  80

Arg Leu Phe Val Asp Glu Asn Leu Asp Arg Met Val Pro Ile Ser Lys
                85                  90                  95

Gln Pro Lys Glu Lys Ile Gln Ala Ile Ile Asp Ser Cys Arg Arg Gln
            100                 105                 110

Phe Pro Glu Tyr Gln Glu Arg Ala Arg Lys Arg Ile Arg Thr Tyr Leu
        115                 120                 125

Lys Ser Cys Arg Arg Met Lys Arg Ser Gly Phe Glu Met Ser Arg Pro
    130                 135                 140

Ile Pro Ser His Leu Thr Ser Ala Val Ala Glu Ser Ile Leu Ala Ser
145                 150                 155                 160

Ala Cys Glu Ser Glu Ser Arg Asn Ala Ala Lys Arg Met Arg Leu Glu
                165                 170                 175

Arg Gln Gln Asp Glu Ser Ala Pro Ala Asp Lys Gln Cys Lys Pro Glu
            180                 185                 190

Ala Thr Gln Ala Thr Tyr Ser Thr Ser Ala Val Pro Gly Ser Gln Asp
        195                 200                 205

Val Leu Tyr Ile Asn Gly Asn Gly Thr Tyr Ser Tyr His Ser Tyr Arg
    210                 215                 220

Gly Leu Gly Gly Gly Leu Leu Asn Leu Asn Asp Ala Ser Ser Ser Gly
225                 230                 235                 240

Pro Thr Asp Leu Ser Met Lys Arg Gln Leu Ala Thr Ser Ser Gly Ser
                245                 250                 255

Ser Ser Ser Ser Asn Ser Arg Pro Gln Leu Ser Pro Thr Glu Ile Asn
            260                 265                 270

Ala Val Arg Gln Leu Val Ala Gly Tyr Arg Glu Ser Ala Ala Phe Leu
        275                 280                 285

Leu Arg Ser Ala Asp Glu Leu Glu Asn Leu Ile Leu Gln Gln Asn
    290                 295                 300

<210> 12
<211> 866
<212> PRT
<213> Homo sapiens

<220>
<223> clone name: hfb2230

<400> 12
Gly Lys Met Ala Ala Pro Leu Ile Pro Leu Ser Gln Gln Ile Pro Thr
1               5                   10                  15

Gly Asn Ser Leu Tyr Glu Ser Tyr Tyr Lys Gln Val Asp Pro Ala Tyr
            20                  25                  30

Thr Gly Arg Val Gly Ala Ser Glu Ala Ala Leu Phe Leu Lys Lys Ser
        35                  40                  45

Gly Leu Ser Asp Ile Ile Leu Gly Lys Ile Trp Asp Leu Ala Asp Pro
    50                  55                  60

Glu Gly Lys Gly Phe Leu Asp Lys Gln Gly Phe Tyr Val Ala Leu Arg
65                  70                  75                  80

Leu Val Ala Cys Ala Gln Ser Gly His Glu Val Thr Leu Ser Asn Leu
                85                  90                  95

Asn Leu Ser Met Pro Pro Pro Lys Phe His Asp Thr Ser Ser Pro Leu
            100                 105                 110

Met Val Thr Pro Pro Ser Ala Glu Ala His Trp Ala Val Arg Val Glu
        115                 120                 125

Glu Lys Ala Lys Phe Asp Gly Ile Phe Glu Ser Leu Leu Pro Ile Asn
    130                 135                 140

Gly Leu Leu Ser Gly Asp Lys Val Lys Pro Val Leu Met Asn Ser Lys
145                 150                 155                 160

Leu Pro Leu Asp Val Leu Gly Arg Val Trp Asp Leu Ser Asp Ile Asp
                165                 170                 175

Lys Asp Gly His Leu Asp Arg Asp Glu Phe Ala Val Ala Met His Leu
            180                 185                 190

Val Tyr Arg Ala Leu Glu Lys Glu Pro Val Pro Ser Ala Leu Pro Pro
        195                 200                 205

Ser Leu Ile Pro Pro Ser Lys Arg Lys Lys Thr Val Phe Pro Gly Ala
    210                 215                 220

Val Pro Val Leu Pro Ala Ser Pro Pro Pro Lys Asp Ser Leu Arg Ser
225                 230                 235                 240

Thr Pro Ser His Gly Ser Val Ser Ser Leu Asn Ser Thr Gly Ser Leu
                245                 250                 255

Ser Pro Lys His Ser Leu Lys Gln Thr Gln Pro Thr Val Asn Trp Val
            260                 265                 270

Val Pro Val Ala Asp Lys Met Arg Phe Asp Glu Ile Phe Leu Lys Thr
        275                 280                 285

Asp Leu Asp Leu Asp Gly Tyr Val Ser Gly Gln Glu Val Lys Glu Ile
    290                 295                 300

Phe Met His Ser Gly Leu Thr Gln Asn Leu Leu Ala His Ile Trp Ala
305                 310                 315                 320

Leu Ala Asp Thr Arg Gln Thr Gly Lys Leu Ser Lys Asp Gln Phe Ala
                325                 330                 335

Leu Ala Met Tyr Phe Ile Gln Gln Lys Val Ser Lys Gly Ile Asp Pro
            340                 345                 350

Pro Gln Val Leu Ser Pro Asp Met Val Pro Pro Ser Glu Arg Gly Thr
        355                 360                 365

Pro Gly Pro Asp Ser Ser Gly Ser Leu Gly Ser Gly Glu Phe Thr Gly
    370                 375                 380

Val Lys Glu Leu Asp Asp Ile Ser Gln Glu Ile Ala Gln Leu Gln Arg
385                 390                 395                 400

Glu Lys Tyr Ser Leu Glu Gln Asp Ile Arg Glu Lys Glu Glu Ala Ile

                405                 410                 415

Arg Gln Lys Thr Ser Glu Val Gln Glu Leu Gln Asn Asp Leu Asp Arg
            420                 425                 430

Glu Thr Ser Ser Leu Gln Glu Leu Glu Ala Gln Lys Gln Asp Ala Gln
        435                 440                 445

Asp Arg Leu Asp Glu Met Asp Gln Gln Lys Ala Lys Leu Arg Asp Met
    450                 455                 460

Leu Ser Asp Val Arg Gln Lys Cys Gln Asp Glu Thr Gln Met Ile Ser
465                 470                 475                 480

Ser Leu Lys Thr Gln Ile Gln Ser Gln Glu Ser Asp Leu Lys Ser Gln
                485                 490                 495

Glu Asp Asp Leu Asn Arg Ala Lys Ser Glu Leu Asn Arg Leu Gln Gln
            500                 505                 510

Glu Glu Thr Gln Leu Glu Gln Ser Ile Gln Ala Gly Arg Val Gln Leu
        515                 520                 525

Glu Thr Ile Ile Lys Ser Leu Lys Ser Thr Gln Asp Glu Ile Asn Gln
    530                 535                 540

Ala Arg Ser Lys Leu Ser Gln Leu His Glu Ser Arg Gln Glu Ala His
545                 550                 555                 560

Arg Ser Leu Glu Gln Tyr Asp Gln Val Leu Asp Gly Ala His Gly Ala
                565                 570                 575

Ser Leu Thr Asp Leu Ala Asn Leu Ser Glu Gly Val Ser Leu Ala Glu
            580                 585                 590

Arg Gly Ser Phe Gly Ala Met Asp Asp Pro Phe Lys Asn Lys Ala Leu
        595                 600                 605

Leu Phe Ser Asn Asn Thr Gln Glu Leu His Pro Asp Pro Phe Gln Thr
    610                 615                 620

Glu Asp Pro Phe Lys Ser Asp Pro Phe Lys Gly Ala Asp Pro Phe Lys
625                 630                 635                 640

Gly Asp Pro Phe Gln Asn Asp Pro Phe Ala Glu Gln Gln Thr Thr Ser
                645                 650                 655

Thr Asp Pro Phe Gly Gly Asp Pro Phe Lys Glu Ser Asp Pro Phe Arg
            660                 665                 670

Gly Ser Ala Thr Asp Asp Phe Phe Lys Lys Gln Thr Lys Asn Asp Pro
        675                 680                 685

Phe Thr Ser Asp Pro Phe Thr Lys Asn Pro Ser Leu Pro Ser Lys Leu
    690                 695                 700

Asp Pro Phe Glu Ser Ser Asp Pro Phe Ser Ser Ser Ser Val Ser Ser
705                 710                 715                 720

Lys Gly Ser Asp Pro Phe Gly Thr Leu Asp Pro Phe Gly Ser Gly Ser
                725                 730                 735

Phe Asn Ser Ala Glu Gly Phe Ala Asp Phe Ser Gln Met Ser Lys Pro
            740                 745                 750

Pro Pro Ser Gly Pro Phe Thr Ser Ser Leu Gly Gly Ala Gly Phe Ser
        755                 760                 765

Asp Asp Pro Phe Lys Ser Lys Gln Asp Thr Pro Ala Leu Pro Pro Lys
    770                 775                 780

Lys Pro Ala Pro Pro Arg Pro Lys Pro Pro Ser Gly Lys Ser Thr Pro
785                 790                 795                 800

Val Ser Gln Leu Gly Ser Ala Asp Phe Pro Glu Ala Pro Asp Pro Phe
                805                 810                 815

Gln Pro Leu Gly Ala Asp Ser Gly Asp Pro Phe Gln Ser Lys Lys Gly
            820                 825                 830

Phe Gly Asp Pro Phe Ser Gly Lys Asp Pro Phe Val Pro Ser Ser Ala
        835                 840                 845

Ala Lys Pro Ser Lys Ala Ser Ala Ser Gly Phe Ala Asp Phe Thr Ser
    850                 855                 860

Val Ser
865

<210> 13
<211> 269
<212> PRT
<213> Homo sapiens

<220>
<223> clone name: hfb2063

<400> 13
Asp Ala Trp Ala Val Lys Glu Glu Cys Arg Leu Leu Asn Ala Pro Pro
  1               5                  10                  15

Val Pro Pro Arg Gly Gly Asn Gly Ser Gly Arg Leu Ser Ser Ser Pro
             20                  25                  30

Pro Val Pro Pro Arg Phe Pro Lys Leu Gln Pro Val His Ser Pro Ser
         35                  40                  45

Ser Ser Leu Ser Tyr Tyr Ser Ser Gly Leu Gln Asp Gly Ala Gly Ser
     50                  55                  60

Arg Ser Gly Ser Gly Ser Pro Ser Pro Asp Thr Tyr Ser Leu Tyr Cys
 65                  70                  75                  80

Tyr Pro Cys Thr Trp Gly Asp Cys Lys Glu Pro Val Leu Glu Pro Phe
                 85                  90                  95

Asp Pro Phe Glu Leu Gly Gln Gly Ser Ser Pro Glu Pro Glu Leu Leu
            100                 105                 110

Arg Ser Gln Glu Pro Arg Ala Val Gly Thr Pro Gly Pro Gly Pro Arg
        115                 120                 125

Leu Ser Pro Leu Gly Pro Ser Lys Ala Phe Glu Pro Glu Gly Leu Val
    130                 135                 140

Leu His Gln Val Pro Thr Pro Leu Ser Pro Ala Ala Leu Gln Gly Pro
145                 150                 155                 160

Glu Ala Gly Gly Ala Leu Phe Leu Thr Gln Gly Arg Leu Glu Gly Pro
                165                 170                 175

Pro Ala Ser Pro Arg Asp Gly Ala Thr Gly Phe Gly Val Arg Asp Ala
            180                 185                 190

Ser Ser Trp Gln Pro Pro Ala Asp Leu Ser Ala Leu Ser Leu Glu Glu
        195                 200                 205

Val Ser Arg Ser Leu Arg Phe Ile Gly Leu Ser Glu Asp Val Val Ser
    210                 215                 220

Phe Phe Ala Arg Glu Arg Ile Asp Gly Ser Ile Phe Val Gln Leu Ser
225                 230                 235                 240

Glu Asp Ile Leu Ala Asp Asp Phe His Leu Thr Lys Leu Gln Val Lys
                245                 250                 255

Lys Ile Met Gln Phe Ile Lys Gly Trp Arg Pro Lys Ile
            260                 265

<210> 14
<211> 54
<212> PRT
<213> Homo sapiens

<220>
<223> clone name: hfb2411

<400> 14

Glu Glu Glu Glu Glu Glu Ser Glu Asn Lys Ser Met Asp Thr Asp Pro
1               5                   10                  15

Glu Thr Ile Arg Asp Glu Glu Glu Thr Gly Asp His Ser Met Asp Asp
            20                  25                  30

Ser Ser Glu Asp Gly Lys Met Glu Thr Lys Ser Asp His Glu Glu Asp
        35                  40                  45

Asn Met Glu Asp Gly Met
    50

<210> 15
<211> 224
<212> PRT
<213> Homo sapiens

<220>
<223> clone name: hfb2436

<400> 15
Ser Ser Asp Pro Ala Asp Phe Glu Pro Ile Pro Ser Phe Ser Gly Phe
1               5                   10                  15

Pro Leu Asp Ser Pro Lys Thr Leu Val Leu Asp Phe Glu Thr Glu Gly
            20                  25                  30

Glu Arg Asn Ser Pro Asn Pro Arg Ser Val Arg Ile Pro Ser Pro Asn
        35                  40                  45

Ile Leu Lys Thr Gly Leu Thr Glu Asn Val Asp Arg Gly Leu Gly Gly
    50                  55                  60

Leu Glu Gly Thr His Gln Ala Leu Asp Leu Leu Ala Gly Gly Met Met
65                  70                  75                  80

Pro Glu Glu Val Lys Glu Ser Ser Gln Leu Asp Lys Gln Glu Ser Leu
                85                  90                  95

Gly Leu Glu Leu Lys Thr Ile Asn Ser Ala Gly Leu Gly Pro Ser Pro
            100                 105                 110

Cys Leu Pro Asp Leu Asp Asp Phe Val Thr Arg Thr Ser Gly Val Gln
        115                 120                 125

Lys Asp Lys Leu Cys Ser Pro Leu Ser Glu Pro Gly Asp Pro Ser Lys
    130                 135                 140

Cys Ser Ser Leu Glu Leu Gly Pro Leu Gln Leu Glu Ile Ser Asn Ala
145                 150                 155                 160

Ser Thr Thr Glu Val Ala Ile Leu Gln Val Asp Asp Asp Ser Gly Asp
                165                 170                 175

Pro Leu Asn Leu Val Lys Ala Pro Val Ser Arg Ser Pro Pro Arg Glu
            180                 185                 190

Gln Val Ile Glu Asp Asn Met Val Pro Gln Gly Met Pro Glu Gln Glu
        195                 200                 205

Thr Thr Val Gly Ala Ile Gln Asp His Thr Glu Ser Ser Val His Asn
    210                 215                 220

<210> 16
<211> 224
<212> PRT
<213> Homo sapiens

<220>
<223> clone name: hfb2122

<400> 16
Glu Arg Ser Leu Ala Glu Met Glu Ser Ser His Gln Gln Val Met Glu
  1               5                  10                  15

Glu Leu Gln Arg His His Glu Arg Glu Leu Gln Arg Leu Gln Gln Glu
             20                  25                  30

Lys Glu Trp Leu Leu Ala Glu Glu Thr Ala Ala Thr Ala Ser Ala Ile
        35                  40                  45

Glu Ala Met Lys Lys Ala Tyr Gln Glu Glu Leu Ser Arg Glu Leu Ser
    50                  55                  60

Lys Thr Arg Ser Leu Gln Gln Gly Pro Asp Gly Leu Arg Lys Gln His
65                  70                  75                  80

Gln Ser Asp Val Glu Ala Leu Lys Arg Glu Leu Gln Val Leu Ser Glu
                85                  90                  95

Gln Tyr Ser Gln Lys Cys Leu Glu Ile Gly Ala Leu Met Arg Gln Ala
            100                 105                 110

Glu Glu Arg Glu His Thr Leu Arg Arg Cys Gln Gln Glu Gly Gln Glu
        115                 120                 125

Leu Leu Arg His Asn Gln Glu Leu His Gly Pro Pro Val Arg Gly Asp
    130                 135                 140

Arg Pro Ala Ala Arg Leu His Cys Leu Ala Gly His Gly Gln Trp Leu
145                 150                 155                 160

Arg Ala Gln Gln Arg Ala Glu Phe Leu Arg Ala Arg Gly Ala Ala Ser
                165                 170                 175

Arg Lys Arg Lys Arg Thr Pro Val Pro Lys Glu Gly Gly Ala Val Pro
            180                 185                 190

Pro Gly Arg Ala Pro Asp Asp Ala Glu Gly Gln Ala Leu His Leu Gly
        195                 200                 205

Lys Val Pro Gly Arg Leu Cys Gly Ala Glu Pro His Gln Asp Thr Val
    210                 215                 220

<210> 17
<211> 408
<212> PRT

<213> Homo sapiens

<220>
<223> clone name: hfb2157

<400> 17
Arg Asn Pro Glu Ile Ser His Leu Leu Asn Asn Pro Asp Ile Met Arg
1               5                   10                  15

Gln Thr Leu Glu Ile Ala Arg Asn Pro Ala Met Met Gln Glu Met Met
            20                  25                  30

Arg Asn Gln Asp Leu Ala Leu Ser Asn Leu Glu Ser Ile Pro Gly Gly
        35                  40                  45

Tyr Asn Ala Leu Arg Arg Met Tyr Thr Asp Ile Gln Glu Pro Met Leu
    50                  55                  60

Asn Ala Ala Gln Glu Gln Phe Gly Gly Asn Pro Phe Ala Ser Val Gly
65                  70                  75                  80

Ser Ser Ser Ser Ser Gly Glu Gly Thr Gln Pro Ser Arg Thr Glu Asn
                85                  90                  95

Arg Asp Pro Leu Pro Asn Pro Trp Ala Pro Pro Pro Ala Thr Gln Ser
            100                 105                 110

Ser Ala Thr Thr Ser Thr Thr Thr Ser Thr Gly Ser Gly Ser Gly Asn
        115                 120                 125

Ser Ser Ser Asn Ala Thr Gly Asn Thr Val Ala Ala Ala Asn Tyr Val
    130                 135                 140

Ala Ser Ile Phe Ser Thr Pro Gly Met Gln Ser Leu Leu Gln Gln Ile
145                 150                 155                 160

Thr Glu Asn Pro Gln Leu Ile Gln Asn Met Leu Ser Ala Pro Tyr Met
                165                 170                 175

Arg Ser Met Met Gln Ser Leu Ser Gln Asn Pro Asp Leu Ala Ala Gln

            180                 185                 190

Met Met Leu Asn Ser Pro Leu Phe Thr Ala Asn Pro Gln Leu Gln Glu
        195                 200                 205

Gln Met Arg Pro Gln Leu Pro Ala Phe Leu Gln Gln Met Gln Asn Pro
    210                 215                 220

Asp Thr Leu Ser Ala Met Ser Asn Pro Arg Ala Met Gln Ala Leu Met
225                 230                 235                 240

Gln Ile Gln Gln Gly Leu Gln Thr Leu Ala Thr Glu Ala Pro Gly Leu
                245                 250                 255

Ile Pro Ser Phe Thr Pro Gly Val Gly Val Gly Val Leu Gly Thr Ala
            260                 265                 270

Ile Gly Pro Val Gly Pro Val Thr Pro Ile Gly Pro Ile Gly Pro Ile
        275                 280                 285

Val Pro Phe Thr Pro Ile Gly Pro Ile Gly Pro Ile Gly Pro Thr Gly
    290                 295                 300

Pro Ala Ala Pro Pro Gly Ser Thr Gly Ser Gly Gly Pro Thr Gly Pro
305                 310                 315                 320

Thr Val Ser Ser Ala Ala Pro Arg Glu Thr Thr Ser Pro Thr Ser Glu
                325                 330                 335

Ser Gly Pro Asn Gln Gln Phe Ile Gln Gln Met Val Gln Ala Leu Ala
            340                 345                 350

Gly Ala Asn Ala Pro Gln Leu Pro Asn Pro Glu Val Arg Phe Gln Gln
        355                 360                 365

Gln Leu Glu Gln Leu Asn Ala Met Gly Phe Leu Asn Arg Glu Ala Asn
    370                 375                 380

Leu Gln Ala Leu Ile Ala Thr Gly Gly Asp Ile Asn Ala Ala Ile Glu
385                 390                 395                 400

Arg Leu Leu Gly Ser Gln Pro Ser
                405

<210> 18
<211> 486
<212> PRT
<213> Homo sapiens

<220>
<223> clone name: hfb2216

<400> 18
Asn Ala Glu Arg Met Glu Ser Ile Leu Gln Ala Leu Glu Asp Ile Gln
 1               5                   10                  15

Leu Asp Leu Glu Ala Val Asn Ile Lys Ala Gly Lys Ala Phe Leu Arg
            20                  25                  30

Leu Lys Arg Lys Phe Ile Gln Met Arg Arg Pro Phe Leu Glu Arg Arg
        35                  40                  45

Asp Leu Ile Ile Gln His Ile Pro Gly Phe Trp Val Lys Ala Phe Leu
    50                  55                  60

Asn His Pro Arg Ile Ser Ile Leu Ile Asn Arg Arg Asp Glu Asp Ile
65                  70                  75                  80

Phe Arg Tyr Leu Thr Asn Leu Gln Val Gln Asp Leu Arg His Ile Ser
                85                  90                  95

Met Gly Tyr Lys Met Lys Leu Tyr Phe Gln Thr Asn Pro Tyr Phe Thr
            100                 105                 110

Asn Met Val Ile Val Lys Glu Phe Gln Arg Asn Arg Ser Gly Arg Leu
        115                 120                 125

Val Ser His Ser Thr Pro Ile Arg Trp His Arg Gly Gln Glu Pro Gln
    130                 135                 140

Ala Arg Arg His Gly Asn Gln Asp Ala Ser His Ser Phe Phe Ser Trp
145                 150                 155                 160

Phe Ser Asn His Ser Leu Pro Glu Ala Asp Arg Ile Ala Glu Ile Ile
                165                 170                 175

Lys Asn Asp Leu Trp Val Asn Pro Leu Arg Tyr Tyr Leu Arg Glu Arg
            180                 185                 190

Gly Ser Arg Ile Lys Arg Lys Lys Gln Glu Met Lys Lys Arg Lys Thr
        195                 200                 205

Arg Gly Arg Cys Glu Val Val Ile Met Glu Asp Ala Pro Asp Tyr Tyr
    210                 215                 220

Ala Val Glu Asp Ile Phe Ser Glu Ile Ser Asp Ile Asp Glu Thr Ile
225                 230                 235                 240

His Asp Ile Lys Ile Ser Asp Phe Met Glu Thr Thr Asp Tyr Phe Glu
                245                 250                 255

Thr Thr Asp Asn Glu Ile Thr Asp Ile Asn Glu Asn Ile Cys Asp Ser
            260                 265                 270

Glu Asn Pro Asp His Asn Glu Val Pro Asn Asn Glu Thr Thr Asp Asn
        275                 280                 285

Asn Glu Ser Ala Asp Asp His Glu Thr Thr Asp Asn Asn Glu Ser Ala
    290                 295                 300

Asp Asp Asn Asn Glu Asn Pro Glu Asp Asn Asn Lys Asn Thr Asp Asp
305                 310                 315                 320

Asn Glu Glu Asn Pro Asn Asn Asn Glu Asn Thr Tyr Gly Asn Asn Phe
                325                 330                 335

Phe Lys Gly Gly Phe Trp Gly Ser His Gly Asn Asn Gln Asp Ser Ser
            340                 345                 350

Asp Ser Asp Asn Glu Ala Asp Glu Ala Ser Asp Asp Glu Asp Asn Asp
        355                 360                 365

Gly Asn Glu Gly Asp Asn Glu Gly Ser Asp Asp Asp Gly Asn Glu Gly
    370                 375                 380

Asp Asn Glu Gly Ser Asp Asp Asp Asp Arg Asp Ile Glu Tyr Tyr Glu
385                 390                 395                 400

Lys Gly Ile Glu Asp Phe Asp Arg Asp Gln Ala Asp Tyr Glu Asp Val
                405                 410                 415

Ile Glu Ile Ile Ser Asp Glu Ser Val Glu Glu Glu Gly Ile Glu Glu
            420                 425                 430

Gly Ile Gln Gln Asp Glu Asp Ile Tyr Glu Glu Gly Asn Tyr Glu Glu
        435                 440                 445

Glu Gly Ser Glu Asp Val Trp Glu Glu Gly Glu Asp Ser Asp Asp Ser
    450                 455                 460

Asp Leu Glu Asp Val Leu Gln Val Pro Asn Gly Trp Ala Asn Pro Gly
465                 470                 475                 480

Lys Arg Gly Lys Thr Gly
                485

<210> 19
<211> 67
<212> PRT
<213> Homo sapiens

<220>
<223> clone name: hfb2025

<400> 19
Ile Ala Glu Thr Gly Val Ser Gly Gly Val Asp Arg Arg Glu Val Gln
  1               5                  10                  15

Arg Leu Arg Arg Arg Asn Gln Gln Leu Glu Glu Glu Asn Asn Leu Leu
            20                  25                  30

Arg Leu Lys Val Asp Ile Leu Leu Asp Met Leu Ser Glu Ser Thr Ala
        35                  40                  45

Glu Ser His Leu Met Glu Lys Glu Leu Asp Glu Leu Arg Ile Ser Arg
    50                  55                  60

Lys Arg Lys
65

<210> 20
<211> 108
<212> PRT
<213> Homo sapiens

<220>
<223> clone name: hfb2281

<400> 20
Glu Gly Pro Leu Asp Ser Pro Ser Glu Gly His Ser Tyr Gln Asn Ser
 1               5                  10                  15

Gly Leu Asp His Phe Gln Asn Ser Asn Ile Asp Gln Ser Phe Trp Glu
            20                  25                  30

Thr Phe Gly Ser Ala Glu Pro Thr Lys Thr Arg Lys Ser Pro Ser Ser
        35                  40                  45

Asp Ser Trp Thr Cys Ala Asp Thr Ser Thr Glu Arg Arg Ser Ser Asp
    50                  55                  60

Ser Trp Glu Val Trp Gly Ser Ala Ser Thr Asn Arg Asn Ser Asn Ser
65                  70                  75                  80

Asp Gly Gly Glu Gly Gly Glu Gly Thr Lys Lys Ala Val Pro Pro Ala
                85                  90                  95

Val Pro Thr Asp Asp Gly Trp Asp Asn Gln Asn Trp
            100                 105

<210> 21
<211> 431
<212> PRT
<213> Homo sapiens

<220>
<223> clone name: hfb2060

<400> 21
Asn Ser Pro Tyr Ser Leu Leu Glu Val Leu Cys Pro Arg Cys Asp Ile
1               5                   10                  15

Gly Leu Leu Gly Leu Lys Arg Lys Gln Arg Lys Leu Lys Glu Pro Leu
            20                  25                  30

Pro Asn Gly Lys Lys Lys Ser Thr Lys Leu Glu Ser Leu Ser Asp Leu
        35                  40                  45

Glu Arg Ala Leu Thr Glu Ser Lys Glu Thr Ala Ser Ala Met Ala Thr
    50                  55                  60

Leu Gly Glu Ala Arg Leu Arg Glu Met Glu Ala Leu Gln Ser Leu Arg
65                  70                  75                  80

Leu Ala Asn Glu Gly Lys Leu Leu Ser Pro Leu Gln Asp Val Asp Ile
                85                  90                  95

Lys Ile Cys Leu Cys Gln Lys Ala Pro Ala Ala Pro Met Ile Gln Cys
            100                 105                 110

Glu Leu Cys Arg Asp Ala Phe His Thr Ser Cys Val Ala Val Pro Ser
        115                 120                 125

Ile Ser Gln Gly Leu Arg Ile Trp Leu Cys Pro His Cys Arg Arg Ser
    130                 135                 140

Glu Lys Pro Pro Leu Glu Lys Ile Leu Pro Leu Leu Ala Ser Leu Gln
145                 150                 155                 160

Arg Ile Arg Val Arg Leu Pro Glu Gly Asp Ala Leu Arg Tyr Met Ile
                165                 170                 175

Glu Arg Thr Val Asn Trp Gln His Arg Ala Gln Gln Leu Leu Ser Ser
            180                 185                 190

Gly Asn Leu Lys Phe Val Gln Asp Arg Val Gly Ser Gly Leu Leu Tyr
        195                 200                 205

Ser Arg Trp Gln Ala Ser Ala Gly Gln Val Ser Asp Thr Asn Lys Val
    210                 215                 220

Ser Gln Pro Pro Gly Thr Thr Ser Phe Ser Leu Pro Asp Asp Trp Asp
225                 230                 235                 240

Asn Arg Thr Ser Tyr Leu His Ser Pro Phe Ser Thr Gly Arg Ser Cys
                245                 250                 255

Ile Pro Leu His Gly Val Ser Pro Glu Val Asn Glu Leu Leu Met Glu
            260                 265                 270

Ala Gln Leu Leu Gln Val Ser Leu Pro Glu Ile Gln Glu Leu Tyr Gln
        275                 280                 285

Thr Leu Leu Ala Lys Pro Ser Pro Ala Gln Gln Thr Asp Arg Ser Ser
    290                 295                 300

Pro Val Arg Pro Ser Ser Glu Lys Asn Asp Cys Cys Arg Gly Lys Arg
305                 310                 315                 320

Asp Gly Ile Asn Ser Leu Glu Arg Lys Leu Lys Arg Arg Leu Glu Arg
                325                 330                 335

Glu Gly Leu Ser Ser Glu Arg Trp Glu Arg Val Lys Lys Met Arg Thr
            340                 345                 350

Pro Lys Lys Lys Lys Ile Lys Leu Ser His Pro Lys Asp Met Asn Asn

355                 360                 365

Phe Lys Leu Glu Arg Glu Arg Ser Tyr Glu Thr His Ser Leu Pro Ser
    370                 375                 380

Asp Thr Ser Tyr Ser Glu Gln Glu Asp Ser Glu Asp Glu Asp Ala Ile
385                 390                 395                 400

Cys Pro Ala Val Ser Cys Leu Gln Pro Glu Gly Asp Glu Val Ser Glu
                405                 410                 415

Val Trp Ala Ile Glu Asp Val Leu Ser Leu Asn Ser Glu Thr Leu
            420                 425                 430

<210> 22
<211> 1027
<212> DNA
<213> Homo sapiens

<220>
<223> clone name: hfb2003

<220>
<221> CDS
<222> (1)..(783)

<400> 22
ggc aag tcc tct gag gtc ctc tcg gcc acc gag ctc agg gtc cag ctg      48
Gly Lys Ser Ser Glu Val Leu Ser Ala Thr Glu Leu Arg Val Gln Leu
1               5                   10                  15

gcc cag aag gag cag gag cta gcc aga gcc aaa gaa gcc ttg cag gcc      96
Ala Gln Lys Glu Gln Glu Leu Ala Arg Ala Lys Glu Ala Leu Gln Ala
            20                  25                  30

atg aaa gct gat cgg aag cgc tta aag ggc gag aag aca gac ctg gtg      144
Met Lys Ala Asp Arg Lys Arg Leu Lys Gly Glu Lys Thr Asp Leu Val
        35                  40                  45

agc cag atg cag cag ctg tat gcc aca ctg gag agc cgc gag gag cag 192
Ser Gln Met Gln Gln Leu Tyr Ala Thr Leu Glu Ser Arg Glu Glu Gln
    50                  55                  60

ctc cga gac ttc atc cgc aac tat gag cag cac cgc aag gag agc gag 240
Leu Arg Asp Phe Ile Arg Asn Tyr Glu Gln His Arg Lys Glu Ser Glu
65                  70                  75                  80

gat gcg gtc aaa gcg ctg gcc aag gag aag gac ctg ctg gag cgt gag 288
Asp Ala Val Lys Ala Leu Ala Lys Glu Lys Asp Leu Leu Glu Arg Glu
                85                  90                  95

aag tgg gag ctg cgg cgc caa gcc aag gag gcc aca gac cac gcc acg 336
Lys Trp Glu Leu Arg Arg Gln Ala Lys Glu Ala Thr Asp His Ala Thr
            100                 105                 110

gca ctg cgc tcc cag ctg gac ctc aag gac aac cgg atg aag gag ctg 384
Ala Leu Arg Ser Gln Leu Asp Leu Lys Asp Asn Arg Met Lys Glu Leu
        115                 120                 125

gag gcc gag ctg gcc atg gcc aaa cag tcc tta gct acg ctg acc aag 432
Glu Ala Glu Leu Ala Met Ala Lys Gln Ser Leu Ala Thr Leu Thr Lys
    130                 135                 140

gac gtc ccc aag cgg cat tcc ctc gcc atg ccg ggc gag acg gtg ctc 480
Asp Val Pro Lys Arg His Ser Leu Ala Met Pro Gly Glu Thr Val Leu
145                 150                 155                 160

aat ggc aac cag gag tgg gtg gtg cag gcg gac ctc ccg ctg acc gca 528
Asn Gly Asn Gln Glu Trp Val Val Gln Ala Asp Leu Pro Leu Thr Ala
                165                 170                 175

gcc atc cgg cag agt caa cag act ctc tac cac tca cac ccc cct cac 576
Ala Ile Arg Gln Ser Gln Gln Thr Leu Tyr His Ser His Pro Pro His
            180                 185                 190

cct gcg gac cgg caa gcg gtc agg gtg agc ccc tgc cac tcc cgg cag 624
Pro Ala Asp Arg Gln Ala Val Arg Val Ser Pro Cys His Ser Arg Gln
        195                 200                 205

ccc tct gtc atc tcc gac gca tct gcc gcc gaa ggc gac cgg tcg tcc 672
Pro Ser Val Ile Ser Asp Ala Ser Ala Ala Glu Gly Asp Arg Ser Ser
    210                 215                 220

aca ccg agc gac atc aac tcc cct cga cac cgg aca cac tcc ctc tgg 720
Thr Pro Ser Asp Ile Asn Ser Pro Arg His Arg Thr His Ser Leu Trp
225                 230                 235                 240

cga cag tcc cgg ccc agt tca gaa gaa cct gca caa ccc tat tgt aca 768
Arg Gln Ser Arg Pro Ser Ser Glu Glu Pro Ala Gln Pro Tyr Cys Thr
                245                 250                 255

gtc act aga gga tct tgaagaccaa aaacggaaaa agaagaaaga gaagatggga 823
Val Thr Arg Gly Ser
            260

ttcggctcca tctcccgcgt cttcgccaga gggaagcagc ggaagtccct cgaccccggc 883

ctctttgatg gtaccgcccc tgattattac atagaggagg acgcggactg gtgatacgcg 943

ctcccctgcg cctgctgccc gcaggcgtgt ctgtgcgtgt gggcgtgtgt gcaagcgagc 1003

gtgggtgcgc gtgtggcccg tgcg 1027

<210> 23
<211> 865
<212> DNA
<213> Homo sapiens

<220>
<223> clone name: hfb2007

<220>
<221> CDS
<222> (1)..(759)

<400> 23
gac tat gag gag atc acc atc gac ccc acg tgc agc tgg aag cca gtg 48
Asp Tyr Glu Glu Ile Thr Ile Asp Pro Thr Cys Ser Trp Lys Pro Val

1                   5                   10                  15

ccc gtg aag cct gac atg cac atc aag gag gag ccg gat ggg cca gca    96
Pro Val Lys Pro Asp Met His Ile Lys Glu Glu Pro Asp Gly Pro Ala
                20                  25                  30

ctg aag cgc tgc cgc acc gtg agc ccc gcc cac gtg ctc atg ccc agc    144
Leu Lys Arg Cys Arg Thr Val Ser Pro Ala His Val Leu Met Pro Ser
            35                  40                  45

gtg atg gag atg atc gcc gcc ctg ggc ccc ggc gct gcc ccc ttt gcc    192
Val Met Glu Met Ile Ala Ala Leu Gly Pro Gly Ala Ala Pro Phe Ala
    50                  55                  60

ccc ctg cag ccc ccc tca gtc cct gcc ccc agc gac tac cct ggc cag    240
Pro Leu Gln Pro Pro Ser Val Pro Ala Pro Ser Asp Tyr Pro Gly Gln
65                  70                  75                  80

ggt tcc agc ttc ctg ggg cct gga act ttc cct gag tcc ttc cca ccc    288
Gly Ser Ser Phe Leu Gly Pro Gly Thr Phe Pro Glu Ser Phe Pro Pro
                85                  90                  95

acc acg ccc agc acc cca acc ctt gct gag ttc acc ccg gga cca ccc    336
Thr Thr Pro Ser Thr Pro Thr Leu Ala Glu Phe Thr Pro Gly Pro Pro
            100                 105                 110

ccc atc tgc tac cag tct gac att ccc agc agc ctc ctg act tca gag    384
Pro Ile Cys Tyr Gln Ser Asp Ile Pro Ser Ser Leu Leu Thr Ser Glu
        115                 120                 125

aag tct acc gtc tgc ctc cca agc cag atg gca cca gca ggt cac ctg    432
Lys Ser Thr Val Cys Leu Pro Ser Gln Met Ala Pro Ala Gly His Leu
    130                 135                 140

gac ccc act cac aat cct ggg aca cca gga cta cac acc tcc aac ctt    480
Asp Pro Thr His Asn Pro Gly Thr Pro Gly Leu His Thr Ser Asn Leu
145                 150                 155                 160

ggg gcc cct cca ggt ccc cag ctg cac cat tca aac cct ccc cca gcg    528
Gly Ala Pro Pro Gly Pro Gln Leu His His Ser Asn Pro Pro Pro Ala

165                 170                 175

tcc cgg cag tcc ttg ggc caa gcg agc tta gga cct acg ggt gaa ctg     576
Ser Arg Gln Ser Leu Gly Gln Ala Ser Leu Gly Pro Thr Gly Glu Leu
            180                 185                 190

gcc ttc agt cct gcc aca ggc gtg atg ggg ccc ccc agc atg tct gga     624
Ala Phe Ser Pro Ala Thr Gly Val Met Gly Pro Pro Ser Met Ser Gly
        195                 200                 205

gcc ggg gag gcc cca gaa cca gct ctg gac ctg ctc ccg gaa ctg acc     672
Ala Gly Glu Ala Pro Glu Pro Ala Leu Asp Leu Leu Pro Glu Leu Thr
    210                 215                 220

aac cct gat gag cta ctg tcc tac ttg ggc cca ccc gac ctc cct acg     720
Asn Pro Asp Glu Leu Leu Ser Tyr Leu Gly Pro Pro Asp Leu Pro Thr
225                 230                 235                 240

aac aac aat gac gac ctg ctt tct ctg ttt gag aac aac tgatcctgtg     769
Asn Asn Asn Asp Asp Leu Leu Ser Leu Phe Glu Asn Asn
                245                 250

tttaccccaa gcccggcggg gacacgctca cagatggcac cacaggcctg cccttcatgc 829

ccagccccat gggacacccg gtggtctttc ccaaac                            865

<210> 24
<211> 1064
<212> DNA
<213> Homo sapiens

<220>
<223> clone name: hfb2017

<220>
<221> CDS
<222> (1)..(648)

<400> 24

aaa agt gaa agc aat gag gac ctt ttt ggg ctt gtg ccc ttt gat gaa 48
Lys Ser Glu Ser Asn Glu Asp Leu Phe Gly Leu Val Pro Phe Asp Glu
1               5                   10                  15

ata acg ggg agc cag cag caa aaa gtc aaa cag cgc agc tta cag aaa 96
Ile Thr Gly Ser Gln Gln Gln Lys Val Lys Gln Arg Ser Leu Gln Lys
            20                  25                  30

ctg tcc tct cgc caa agg cgc aca aag cag gat atg tcc aaa agt aat 144
Leu Ser Ser Arg Gln Arg Arg Thr Lys Gln Asp Met Ser Lys Ser Asn
        35                  40                  45

ggg aag cgg cat cat ggc acg cca act agc aca aag aag act ttg aag 192
Gly Lys Arg His His Gly Thr Pro Thr Ser Thr Lys Lys Thr Leu Lys
    50                  55                  60

cct acc tat cgc act cca gag agg gct cgc agg cac aaa aaa gtg ggc 240
Pro Thr Tyr Arg Thr Pro Glu Arg Ala Arg Arg His Lys Lys Val Gly
65                  70                  75                  80

cgc cga gac tct caa agt agc aat gaa ttt tta acc atc tca gac tcc 288
Arg Arg Asp Ser Gln Ser Ser Asn Glu Phe Leu Thr Ile Ser Asp Ser
                85                  90                  95

aag gag aac att agt gtt gca ctg act gat ggg aaa gat agg ggg aat 336
Lys Glu Asn Ile Ser Val Ala Leu Thr Asp Gly Lys Asp Arg Gly Asn
            100                 105                 110

gtc tta caa cct gag gag agc ctg ttg gac ccc ttc ggt gcc aag ccc 384
Val Leu Gln Pro Glu Glu Ser Leu Leu Asp Pro Phe Gly Ala Lys Pro
        115                 120                 125

ttc cat tct cca gac ctg tca tgg cac cct cca cat cag ggc ctg agc 432
Phe His Ser Pro Asp Leu Ser Trp His Pro Pro His Gln Gly Leu Ser
    130                 135                 140

gac atc cgt gct gat cac aat act gtc ctg cca ggg cgg cca aga caa 480
Asp Ile Arg Ala Asp His Asn Thr Val Leu Pro Gly Arg Pro Arg Gln
145                 150                 155                 160

aat tca cta cat ggg tca ttc cat agt gca gat gta ttg aaa atg gat 528
Asn Ser Leu His Gly Ser Phe His Ser Ala Asp Val Leu Lys Met Asp
                165                 170                 175

gat ttt ggt gcc gtg ccc ttt aca gaa ctt gtg gtg caa agc atc act 576
Asp Phe Gly Ala Val Pro Phe Thr Glu Leu Val Val Gln Ser Ile Thr
            180                 185                 190

cca cat cag tcc caa cag tcc caa cca gtc gaa tta gac cca ttt ggt 624
Pro His Gln Ser Gln Gln Ser Gln Pro Val Glu Leu Asp Pro Phe Gly
        195                 200                 205

gct gct cca ttt cct tct aaa cag tagatacttc tgatggattc tcggcattaa 678
Ala Ala Pro Phe Pro Ser Lys Gln
    210                 215

ctcctgtttc aaaaaagtgt gaacagtttt atgaatttga aagaaaattt ggtagctctt 738

tatagcattc attcttaaag atcagtcaga ataggtgatt tctaaataaa ccaaatagaa 798

gaatgaagta tctctacagg gtagtaactt gattcctctt caggagaaaa gggagctaaa 858

ttgcaagctc taactaaggg tttctgctac tgacatcaca acacagaaat gcaagtgtgg 918

tacttccagt gaaagcacat ggcacctttc taggtgtgta gccactgaga agggacagtg 978

aaactgttat ttttgatatc agaatgtcat ttttatgtgc atatccctaa aattagggtt 1038

atttctacat acactagttc acttgt 1064

<210> 25
<211> 1453
<212> DNA
<213> Homo sapiens

<220>
<223> clone name: hfb2018

<220>

<221> CDS
<222> (1)..(1119)

<400> 25
gag cag cag aag ccg gcg tcg tcg gat gtt gtg ttg ccc gcc acc atg     48
Glu Gln Gln Lys Pro Ala Ser Ser Asp Val Val Leu Pro Ala Thr Met
1               5                   10                  15

agc tac aca ggc ttt gtc cag gga tct gaa acc act ttg cag tcg aca     96
Ser Tyr Thr Gly Phe Val Gln Gly Ser Glu Thr Thr Leu Gln Ser Thr
            20                  25                  30

tac tcg gat acc agc gct cag ccc acc tgt gat tat gga tat gga act     144
Tyr Ser Asp Thr Ser Ala Gln Pro Thr Cys Asp Tyr Gly Tyr Gly Thr
        35                  40                  45

tgg aac tct ggg aca aat aga ggc tac gag ggc tat ggc tat ggc tat     192
Trp Asn Ser Gly Thr Asn Arg Gly Tyr Glu Gly Tyr Gly Tyr Gly Tyr
    50                  55                  60

ggc tat ggc cag gat aac acc acc aac tat ggg tat ggt atg gcc act     240
Gly Tyr Gly Gln Asp Asn Thr Thr Asn Tyr Gly Tyr Gly Met Ala Thr
65                  70                  75                  80

tca cac tct tgg gaa atg cct agc tct gac aca aat gca aac act agt     288
Ser His Ser Trp Glu Met Pro Ser Ser Asp Thr Asn Ala Asn Thr Ser
                85                  90                  95

gcc tcg ggt agc gcc agt gcc gat tcc gtt tta tcc aga att aac cag     336
Ala Ser Gly Ser Ala Ser Ala Asp Ser Val Leu Ser Arg Ile Asn Gln
            100                 105                 110

cgc tta gat atg gtg ccg cat ttg gag aca gac atg atg caa gga ggc     384
Arg Leu Asp Met Val Pro His Leu Glu Thr Asp Met Met Gln Gly Gly
        115                 120                 125

gtg tac ggc tca ggt gga gaa agg tat gac tct tat gag tcc tgc gac     432
Val Tyr Gly Ser Gly Gly Glu Arg Tyr Asp Ser Tyr Glu Ser Cys Asp
    130                 135                 140

tcg agg gcc gtc ctg agt gag cgc gac ctg tac cgg tca ggc tat gac     480
Ser Arg Ala Val Leu Ser Glu Arg Asp Leu Tyr Arg Ser Gly Tyr Asp
145                 150                 155                 160

tac agc gag ctt gac cct gag atg gaa atg gcc tat gag ggc caa tac     528
Tyr Ser Glu Leu Asp Pro Glu Met Glu Met Ala Tyr Glu Gly Gln Tyr
                165                 170                 175

gat gcc tac cgc gac cag ttc cgc atg cgt ggc aac gac acc ttc ggt     576
Asp Ala Tyr Arg Asp Gln Phe Arg Met Arg Gly Asn Asp Thr Phe Gly
            180                 185                 190

ccc agg gca cag ggc tgg gcc cgg gat gcc cgg agc ggc cgg cca atg     624
Pro Arg Ala Gln Gly Trp Ala Arg Asp Ala Arg Ser Gly Arg Pro Met
        195                 200                 205

gcc tca ggc tat ggg cgc atg tgg gaa gac ccc atg ggg gcc cgg ggc     672
Ala Ser Gly Tyr Gly Arg Met Trp Glu Asp Pro Met Gly Ala Arg Gly
    210                 215                 220

cag tgc atg tct ggt gcc tct cgg ctg ccc tcc ctc ttc tcc cag aac     720
Gln Cys Met Ser Gly Ala Ser Arg Leu Pro Ser Leu Phe Ser Gln Asn
225                 230                 235                 240

atc atc ccc gag tac ggc atg ttc cag ggc atg cga ggt ggg ggc gcc     768
Ile Ile Pro Glu Tyr Gly Met Phe Gln Gly Met Arg Gly Gly Gly Ala
                245                 250                 255

ttc ccg ggc ggc tcc cgc ttt ggt ttc ggg ttt ggc aat ggc atg aag     816
Phe Pro Gly Gly Ser Arg Phe Gly Phe Gly Phe Gly Asn Gly Met Lys
            260                 265                 270

cag atg agg cgg acc tgg aag acc tgg acc aca gcc gac ttc cga acc     864
Gln Met Arg Arg Thr Trp Lys Thr Trp Thr Thr Ala Asp Phe Arg Thr
        275                 280                 285

aag aag aag aag aga aag cag ggc ggc agt cct gat gag cca gat agc     912
Lys Lys Lys Lys Arg Lys Gln Gly Gly Ser Pro Asp Glu Pro Asp Ser
    290                 295                 300

aaa gcc acc cgc acg gac tgc tcg gac aac agc gac tca gac aat gat    960
Lys Ala Thr Arg Thr Asp Cys Ser Asp Asn Ser Asp Ser Asp Asn Asp
305                 310                 315                 320

gag ggc acc gag ggg gaa gcc aca gag ggc ctt gaa ggc acc gag gct    1008
Glu Gly Thr Glu Gly Glu Ala Thr Glu Gly Leu Glu Gly Thr Glu Ala
                325                 330                 335

gtg gag aag ggc tcc aga gtg gac gga gag gat gag gag gga aaa gag    1056
Val Glu Lys Gly Ser Arg Val Asp Gly Glu Asp Glu Glu Gly Lys Glu
            340                 345                 350

gat ggg aga gaa gaa ggc aaa gag gat cca gag aag ggt gag ttt tcc    1104
Asp Gly Arg Glu Glu Gly Lys Glu Asp Pro Glu Lys Gly Glu Phe Ser
        355                 360                 365

tgg ggt gcc tgg tgc tagggcggct cttccagggg ctcacagtgg gagggaccca    1159
Trp Gly Ala Trp Cys
    370

gctgagggca gggttgcggc agacatgcgg ccagttgggg acctcccttg tgggcccgag 1219

tgtgtgctgg caggggcctt tcttttcgcc gcctccaccc ccttcccact cttcctcctc 1279

cttcccaggc ggaggctgtg gatggtggtg acccctgcct agcacactgg ttttgccttc 1339

agatgggggc agctagtaaa agtgtgggga accccaggca cgccccctgc cctcggtccc 1399

tcagtaacgc gcagttccaa gtcctcagtg gctggtgtcc gatctgggac cctc       1453

<210> 26
<211> 1156
<212> DNA
<213> Homo sapiens

<220>
<223> clone name: hfb2038

<220>

<221> CDS
<222> (1)..(567)

<400> 26
gag tta ttt gcc cgt tat gac cgc ctt ctc ctc cag tcc tcc ttg gac     48
Glu Leu Phe Ala Arg Tyr Asp Arg Leu Leu Leu Gln Ser Ser Leu Asp
1               5                   10                  15

ctg atg gca gat gtg gtg tac tgc ccc cgg ccg tgc tgc cag ctg cct     96
Leu Met Ala Asp Val Val Tyr Cys Pro Arg Pro Cys Cys Gln Leu Pro
            20                  25                  30

gtg atg cag gaa cct ggc tgc acc atg ggt atc tgc tcc agc tgc aat     144
Val Met Gln Glu Pro Gly Cys Thr Met Gly Ile Cys Ser Ser Cys Asn
        35                  40                  45

ttt gcc ttc tgt act ttg tgc agg ttg acc tac cat ggg gtc tcc cca     192
Phe Ala Phe Cys Thr Leu Cys Arg Leu Thr Tyr His Gly Val Ser Pro
    50                  55                  60

tgt aag gtg act gca gag aaa tta atg gac tta cga aat gaa tac ctg     240
Cys Lys Val Thr Ala Glu Lys Leu Met Asp Leu Arg Asn Glu Tyr Leu
65                  70                  75                  80

caa gcg gat gag gct aat aaa aga ctt ttg gat caa agg tat ggt aag     288
Gln Ala Asp Glu Ala Asn Lys Arg Leu Leu Asp Gln Arg Tyr Gly Lys
                85                  90                  95

aga gtg att cag aag gca ctg gaa gag atg gaa agt aag gag tgg cta     336
Arg Val Ile Gln Lys Ala Leu Glu Glu Met Glu Ser Lys Glu Trp Leu
            100                 105                 110

gag aag aac tca aag agc tgc cca tgt tgt gga act ccc ata gag aaa     384
Glu Lys Asn Ser Lys Ser Cys Pro Cys Cys Gly Thr Pro Ile Glu Lys
        115                 120                 125

tta gac gga tgt aac aag atg aca tgt act ggc tgt atg caa tat ttc     432
Leu Asp Gly Cys Asn Lys Met Thr Cys Thr Gly Cys Met Gln Tyr Phe
    130                 135                 140

tgt tgg att tgc atg ggt tct ctc tct aga gca aac cct tac aaa cat    480
Cys Trp Ile Cys Met Gly Ser Leu Ser Arg Ala Asn Pro Tyr Lys His
145                 150                 155                 160

ttc aat gac cct ggt tca cca tgt ttt aac cgg ctg ttt tat gct gtg    528
Phe Asn Asp Pro Gly Ser Pro Cys Phe Asn Arg Leu Phe Tyr Ala Val
                165                 170                 175

gat gtt gac gac gat att tgg gaa gat gag gta gaa gac tagttaacta    577
Asp Val Asp Asp Asp Ile Trp Glu Asp Glu Val Glu Asp
            180                 185

ctgctcaaga tatggaagtg gattgttttt ccctaatctt ccgtcaagta cacaaagtaa 637

ctttgcggga tatttagggt actattcatt cactcttcct gcgtagaaga tatggaagaa 697

cgaggtttat attttcatgt ggtactactg aagaaggtgc attgatacat ttttaaatgt 757

aagttgagaa aaatttataa gccaaaggtt cagaaaatta aactacagaa tattaaatat 817

tataatgtgc ccaaagctct gaatagttaa aaattaaata tttattttct tccccaagct 877

ttaggtaagg agaagagggg tcaagagtta aacttagaga ccctttgtct ctgagaagca 937

tccttctaag acattctgtt ggagttccct cagtactatt ccttacaact ggagtgggta 997

gaagccttat gaaaattata ctgacaacct gatctcgttt actccatgtt aatcacattc 1057

ctaccaacct aaatttctgc caagtcagtt ctctttggag gaagcccatt actccataac 1117

tgactggatg gtccagtgtc atttttgatc tgctttttc                        1156

<210> 27
<211> 465
<212> DNA
<213> Homo sapiens

<220>
<223> clone name: hfb2099

<220>
<221> CDS
<222> (1)..(369)

<400> 27
gac gcg tgg gcc agc gtc ggc tgt ccc tcc agc tcc cca tcc tcc acc 48
acg cct acc tcc cat cca tcg gag gcg tgg acg cca gct gtg tca gcc 96
cct gcg tca gcc cca ccg cca gcc ccc gcc aca gac atg tgc cac cct 144
cct tcc gag tca tgg tct cgg gcc tgt aag ggt ggg agg cct ggg ccc 192
ggg gcc tcc ccc gtg aca gaa cca cac tgg gca gag ggg tct gct gca 240
gaa aca ctg tcg gct ctg gct gcg gag aag ctg ggc acc atg gct ggc 288
ctc tca gga cca ctc gga tgg cac tca ggt gga cag gac ggg gca ggg 336
gga gac ttg gca cct gac ctc gag cct tat ttg tgaagtcctt atttcttcac 389
aaagaagagg aacggaaatg ggacgtcttc cttaacatct gcaaacaagg aggcgctggg 449
atatcaaact tgcaat                                                465

<210> 28
<211> 464
<212> DNA
<213> Homo sapiens

<220>
<223> clone name: hfb2072

<220>
<221> CDS
<222> (1)..(375)

<400> 28
tgc cgg ggt cag ttg gtc caa gtg tcc cgg cct gag gtg tcg gcc gga    48
Cys Arg Gly Gln Leu Val Gln Val Ser Arg Pro Glu Val Ser Ala Gly
 1               5                  10                  15

tcc ctc ctt ctc ccg gcg cct caa gcg gaa gac cat tcc tca aga att    96
Ser Leu Leu Leu Pro Ala Pro Gln Ala Glu Asp His Ser Ser Arg Ile
            20                  25                  30

ttg tat cca agg ccc aaa agt ttg tta ccc aag atg atg aat gct gac    144
Leu Tyr Pro Arg Pro Lys Ser Leu Leu Pro Lys Met Met Asn Ala Asp
        35                  40                  45

atg gat gca gtt gat gct gaa aat caa gtg gaa ctg gag gaa aaa aca    192
Met Asp Ala Val Asp Ala Glu Asn Gln Val Glu Leu Glu Glu Lys Thr
    50                  55                  60

aga ctt att aat caa gtg ttg gaa ctc caa cac aca ctt gaa gat ctc    240
Arg Leu Ile Asn Gln Val Leu Glu Leu Gln His Thr Leu Glu Asp Leu
65                  70                  75                  80

tct gca aga gta gat gca gtt aag gaa gaa aat ctg aag cta aaa tca    288
Ser Ala Arg Val Asp Ala Val Lys Glu Glu Asn Leu Lys Leu Lys Ser
                85                  90                  95

gaa aac caa gtt ctt gga caa tat ata gaa aat ctc atg tca gct tct    336
Glu Asn Gln Val Leu Gly Gln Tyr Ile Glu Asn Leu Met Ser Ala Ser
            100                 105                 110

agt gtt ttt caa aca act gac aca aaa agc aaa aga aag taagggattg    385
Ser Val Phe Gln Thr Thr Asp Thr Lys Ser Lys Arg Lys
        115                 120                 125

acacccttct gttttatgga attgctgctg atcatttttt ctttaaaact tggatagatt 445

ccaaaagtta cagtacctt                                               464

<210> 29
<211> 1436
<212> DNA
<213> Homo sapiens

<220>
<223> clone name: hfb2091

<220>
<221> CDS
<222> (1)..(1128)

<400> 29
aag atg gat gtt ctt cag ttc cta gaa gga atc cca gtg gat gaa aat    48
Lys Met Asp Val Leu Gln Phe Leu Glu Gly Ile Pro Val Asp Glu Asn
  1               5                  10                  15

gct gta cat gtt ctt gtt gat aac aat ggg caa ggt cta gga cag gca    96
Ala Val His Val Leu Val Asp Asn Asn Gly Gln Gly Leu Gly Gln Ala
             20                  25                  30

ttg gtt cag ttt aaa aat gaa gat gat gca cgt aag tct gaa cgc tta    144
Leu Val Gln Phe Lys Asn Glu Asp Asp Ala Arg Lys Ser Glu Arg Leu
         35                  40                  45

cac cgt aaa aaa ctt aat ggg aga gaa gct ttt gtt cat gta gtt acc    192
His Arg Lys Lys Leu Asn Gly Arg Glu Ala Phe Val His Val Val Thr

    50                  55                  60

cta gaa gat atg aga gag att gag aaa aat ccc cct gcc caa gga aaa 240
Leu Glu Asp Met Arg Glu Ile Glu Lys Asn Pro Pro Ala Gln Gly Lys
65                  70                  75                  80

aag gga tta aag atg cct gtg cca ggt aat cct gca gtt cca gga atg 288
Lys Gly Leu Lys Met Pro Val Pro Gly Asn Pro Ala Val Pro Gly Met
                85                  90                  95

ccc aat gcg gga ctg ccc ggt gtg gga ctg ccc agt gca gga ctt ccc 336
Pro Asn Ala Gly Leu Pro Gly Val Gly Leu Pro Ser Ala Gly Leu Pro
            100                 105                 110

ggt gca ggc ctg ccc agc aca gga ctg cct ggt tca gca ata acc agt 384
Gly Ala Gly Leu Pro Ser Thr Gly Leu Pro Gly Ser Ala Ile Thr Ser
        115                 120                 125

gca gga ctg cct ggt gcg gga atg ccc agt gca gga ata cct agt gca 432
Ala Gly Leu Pro Gly Ala Gly Met Pro Ser Ala Gly Ile Pro Ser Ala
    130                 135                 140

gga ggt gaa gag cat gcc ttc ctg act gta gga tca aag gaa gcc aat 480
Gly Gly Glu Glu His Ala Phe Leu Thr Val Gly Ser Lys Glu Ala Asn
145                 150                 155                 160

aat ggg cct cca ttt aac ttt cct ggt aat ttt ggt gga tca aat gcc 528
Asn Gly Pro Pro Phe Asn Phe Pro Gly Asn Phe Gly Gly Ser Asn Ala
                165                 170                 175

ttt ggg cca cca atc cct cct cca gga tta gga ggc ggg gcc ttt ggt 576
Phe Gly Pro Pro Ile Pro Pro Pro Gly Leu Gly Gly Gly Ala Phe Gly
            180                 185                 190

gat gct agg cct ggt atg cct tca gtt gga aac agt ggt ttg cct ggt 624
Asp Ala Arg Pro Gly Met Pro Ser Val Gly Asn Ser Gly Leu Pro Gly
        195                 200                 205

cta gga ctg gat gtt ccg ggt ttt gga ggt gga cca aac aat tta agt 672
Leu Gly Leu Asp Val Pro Gly Phe Gly Gly Gly Pro Asn Asn Leu Ser

210                 215                 220

ggg cca tcg gga ttt gga ggg ggc cct cag aat ttt gga aat ggc cct    720
Gly Pro Ser Gly Phe Gly Gly Gly Pro Gln Asn Phe Gly Asn Gly Pro
225                 230                 235                 240

ggt agc tta ggc ggt ccc ccg ggg ttt gga agt ggc cct cct ggt ctt    768
Gly Ser Leu Gly Gly Pro Pro Gly Phe Gly Ser Gly Pro Pro Gly Leu
                245                 250                 255

gga agt gcc cct ggg cat ttg ggt ggg cca cca gct ttt ggg cct ggc    816
Gly Ser Ala Pro Gly His Leu Gly Gly Pro Pro Ala Phe Gly Pro Gly
            260                 265                 270

ccc ggc ccc ggc ccc ggc cct ggc cca atc cat att ggt ggt ccc cct    864
Pro Gly Pro Gly Pro Gly Pro Gly Pro Ile His Ile Gly Gly Pro Pro
        275                 280                 285

ggc ttt gca tct agt tct gga aaa cca gga ccg aca gta att aaa gtg    912
Gly Phe Ala Ser Ser Ser Gly Lys Pro Gly Pro Thr Val Ile Lys Val
    290                 295                 300

caa aac atg ccc ttt act gtg tct att gat gag att tta gat ttc ttt    960
Gln Asn Met Pro Phe Thr Val Ser Ile Asp Glu Ile Leu Asp Phe Phe
305                 310                 315                 320

tat ggc tat caa gta atc cca ggc tca gtg tgt tta aaa tac aat gaa    1008
Tyr Gly Tyr Gln Val Ile Pro Gly Ser Val Cys Leu Lys Tyr Asn Glu
                325                 330                 335

aaa ggt atg ccc aca ggt gaa gcc atg gtg gcc ttt gag tct cgg gat    1056
Lys Gly Met Pro Thr Gly Glu Ala Met Val Ala Phe Glu Ser Arg Asp
            340                 345                 350

gaa gcc aca gct gct gtc att gac tta aat gac agg cct ata ggt tca    1104
Glu Ala Thr Ala Ala Val Ile Asp Leu Asn Asp Arg Pro Ile Gly Ser
        355                 360                 365

aga aaa gta aaa ctt gta tta ggg tagccattca catcattttt tatagggtag    1158
Arg Lys Val Lys Leu Val Leu Gly

370                 375

atcttcatat tgctgtgatt aatgcatcca gattgttttc ctagtatttc caggttagaa 1218

cctgtggatt gtttcaattg catatagctt ggtttccata acatagagca ttggttgact 1278

gtttacagaa gactcactca ccaggataaa cattgctgta tgttacagta aagctatctg 1338

gagagaacac ataaatgatt ttggcatacc attagagaaa ccatttgtaa aactcaaatg 1398

accacataaa gcttatcaag gagtctagat tggttttg                         1436

<210> 30
<211> 537
<212> DNA
<213> Homo sapiens

<220>
<223> clone name: hfb2115

<220>
<221> CDS
<222> (1)..(315)

<400> 30
gaa gac cta gga aag gac gtt gaa ttt gaa gtt gtt ggt gat gcc ccg    48
Glu Asp Leu Gly Lys Asp Val Glu Phe Glu Val Val Gly Asp Ala Pro
 1               5                  10                  15

gaa aaa gtg ggg ccc aaa caa gct gaa gat gct gcc aaa agc ata acc    96
Glu Lys Val Gly Pro Lys Gln Ala Glu Asp Ala Ala Lys Ser Ile Thr
            20                  25                  30

aat ggc agt gat gat gga gct cag ccc tcc acc tcc aca gct caa gag    144
Asn Gly Ser Asp Asp Gly Ala Gln Pro Ser Thr Ser Thr Ala Gln Glu
        35                  40                  45

caa gat gac gtt ctc ata gtt gat tcg gat gaa gaa gat tct tca aat    192
Gln Asp Asp Val Leu Ile Val Asp Ser Asp Glu Glu Asp Ser Ser Asn

50                  55                  60

aat gcc gac gtc agt gaa gaa gag aga agc cgc aag agg aaa tta gat     240
Asn Ala Asp Val Ser Glu Glu Glu Arg Ser Arg Lys Arg Lys Leu Asp
65                  70                  75                  80

gag aaa gag aat ctc agt gca aag agg tca cgt ata gaa cag aag gaa     288
Glu Lys Glu Asn Leu Ser Ala Lys Arg Ser Arg Ile Glu Gln Lys Glu
                85                  90                  95

gag ctt gat gat gtc ata gca tta gat tgaacagaaa tgcctctaaa           335
Glu Leu Asp Asp Val Ile Ala Leu Asp
            100                 105

cagaaccctc ttactattta gtttatctgg gcagaaccag attgttatgt cctttgttcc 395

aaagggaaaa aattgacagc agtgacttga aaatgattct gctccctttg aaagcattca 455

ttttgctaga actggtagac acattgcagt atgctgtatt gaaagtagga atatagtttt 515

aaaaaccctt tgacaaagtg tg                                            537

<210> 31
<211> 536
<212> DNA
<213> Homo sapiens

<220>
<223> clone name: hfb2206

<220>
<221> CDS
<222> (1)..(264)

<400> 31
ccc cag ttg gtg gtg gta ggg gaa gag ggc ttc cct gat act ggc tcc     48
Pro Gln Leu Val Val Val Gly Glu Glu Gly Phe Pro Asp Thr Gly Ser
1               5                   10                  15

gac cat tcg tac tcc ttg tcg tca ggc acc acg gag gag gag ctc ctg    96
Asp His Ser Tyr Ser Leu Ser Ser Gly Thr Thr Glu Glu Glu Leu Leu
            20                  25                  30

cgc aag ctg aat gag cag cgg gac atc ctg gct ctg atg gaa gtg aag    144
Arg Lys Leu Asn Glu Gln Arg Asp Ile Leu Ala Leu Met Glu Val Lys
        35                  40                  45

atg aaa gag atg aaa ggc agc att cgc cac ctg cgt ctc act gag gcc    192
Met Lys Glu Met Lys Gly Ser Ile Arg His Leu Arg Leu Thr Glu Ala
    50                  55                  60

aag ctg cgc gaa gaa ctg cgt gag aag gat cgg ctg ctt gcc atg gct    240
Lys Leu Arg Glu Glu Leu Arg Glu Lys Asp Arg Leu Leu Ala Met Ala
65                  70                  75                  80

gtc atc cgc aag aag cac gga atg tgaactggtg ccccggcagc ctgctggact    294
Val Ile Arg Lys Lys His Gly Met
                85

cccagacccc atccagccag gggaccgcag gccattgttg aactcctcta tactcctggg 354

cactggttga cagtactgag gcttaaggca gctggactct cttgctggtg acctggcatc 414

ctcaattgtt tctcctgaag tggaagctgg ggccttagac tctgcctggt gacaccagca 474

attatgactt tgctaccctt cctcccagtt attggtgcag attctggtta agcagaagct 534

tc                                                                536

<210> 32
<211> 952
<212> DNA
<213> Homo sapiens

<220>
<223> clone name: hfb2255

<220>

<221> CDS
<222> (1)..(909)

<400> 32
cta att tct gac ctc aag atg gaa cga gag gcg aga gaa aat gga agc 48
Leu Ile Ser Asp Leu Lys Met Glu Arg Glu Ala Arg Glu Asn Gly Ser
1               5                   10                  15

aag tct cct gca cat agt tac tcc agc tat gac tct ggc aaa aat gag 96
Lys Ser Pro Ala His Ser Tyr Ser Ser Tyr Asp Ser Gly Lys Asn Glu
            20                  25                  30

agt gta gac cga gga gct gag gac ctc tca cta aac agg gga gat gag 144
Ser Val Asp Arg Gly Ala Glu Asp Leu Ser Leu Asn Arg Gly Asp Glu
        35                  40                  45

gac gaa gat gac cac gag gac cat gac gat tcg gag aaa gtt aat gag 192
Asp Glu Asp Asp His Glu Asp His Asp Asp Ser Glu Lys Val Asn Glu
    50                  55                  60

aca gac ggc gtt gaa gcc gag cgg ctg aaa gct ttt aat atg ttt gtc 240
Thr Asp Gly Val Glu Ala Glu Arg Leu Lys Ala Phe Asn Met Phe Val
65                  70                  75                  80

agg ctg ttt gta gat gaa aac ttg gac cga atg gtc cca atc tct aag 288
Arg Leu Phe Val Asp Glu Asn Leu Asp Arg Met Val Pro Ile Ser Lys
                85                  90                  95

cag ccc aaa gaa aag atc cag gct atc att gac tca tgc agg cga caa 336
Gln Pro Lys Glu Lys Ile Gln Ala Ile Ile Asp Ser Cys Arg Arg Gln
            100                 105                 110

ttc cct gag tat caa gag cgt gcc aga aaa cgt ata cgt act tac ctc 384
Phe Pro Glu Tyr Gln Glu Arg Ala Arg Lys Arg Ile Arg Thr Tyr Leu
        115                 120                 125

aag tcc tgc agg cgg atg aaa aga agt ggt ttt gag atg tct cga cct 432
Lys Ser Cys Arg Arg Met Lys Arg Ser Gly Phe Glu Met Ser Arg Pro
    130                 135                 140

att cct tcc cac ctt act tca gca gtt gca gag agt atc ttg gct tca 480
Ile Pro Ser His Leu Thr Ser Ala Val Ala Glu Ser Ile Leu Ala Ser
145                 150                 155                 160

gct tgt gag agt gag agt aga aat gcc gcc aag agg atg cgt ctg gag 528
Ala Cys Glu Ser Glu Ser Arg Asn Ala Ala Lys Arg Met Arg Leu Glu
                165                 170                 175

aga cag cag gat gag tct gct cca gct gac aaa cag tgt aaa cca gag 576
Arg Gln Gln Asp Glu Ser Ala Pro Ala Asp Lys Gln Cys Lys Pro Glu
            180                 185                 190

gcg acc cag gcc act tac tca aca tca gct gtt cca ggc tca cag gac 624
Ala Thr Gln Ala Thr Tyr Ser Thr Ser Ala Val Pro Gly Ser Gln Asp
        195                 200                 205

gtg ctg tac atc aat gga aat ggg acc tat agt tac cat agt tac aga 672
Val Leu Tyr Ile Asn Gly Asn Gly Thr Tyr Ser Tyr His Ser Tyr Arg
    210                 215                 220

ggg cta gga ggg ggt ctg cta aat ctg aat gat gct tcc agc agt gga 720
Gly Leu Gly Gly Gly Leu Leu Asn Leu Asn Asp Ala Ser Ser Ser Gly
225                 230                 235                 240

ccc act gat ctc agc atg aag aga caa ttg gcg act agc tca gga tcc 768
Pro Thr Asp Leu Ser Met Lys Arg Gln Leu Ala Thr Ser Ser Gly Ser
                245                 250                 255

tcc agc agc tca aac tcc aga ccc cag ctg agt cca act gaa atc aat 816
Ser Ser Ser Ser Asn Ser Arg Pro Gln Leu Ser Pro Thr Glu Ile Asn
            260                 265                 270

gcc gtg aga cag ctt gtt gca gga tat cga gaa tca gct gca ttt tta 864
Ala Val Arg Gln Leu Val Ala Gly Tyr Arg Glu Ser Ala Ala Phe Leu
        275                 280                 285

ttg cga tct gca gat gaa ctg gaa aat ctc att tta caa cag aac 909
Leu Arg Ser Ala Asp Glu Leu Glu Asn Leu Ile Leu Gln Gln Asn
    290                 295                 300

tgagacagac gaccaccata ttcactgagg tctaaattgc agt                    952

<210> 33
<211> 2774
<212> DNA
<213> Homo sapiens

<220>
<223> clone name: hfb2230

<220>
<221> CDS
<222> (1)..(2598)

<400> 33
ggg aag atg gcg gcg ccg ctc atc ccc ctc tcc cag cag att ccc act     48
Gly Lys Met Ala Ala Pro Leu Ile Pro Leu Ser Gln Gln Ile Pro Thr
1               5                   10                  15

gga aat tcg ttg tat gaa tct tat tac aag cag gtc gat ccg gca tac     96
Gly Asn Ser Leu Tyr Glu Ser Tyr Tyr Lys Gln Val Asp Pro Ala Tyr
            20                  25                  30

aca ggg agg gtg ggg gcg agt gaa gct gcg ctt ttt cta aag aag tct     144
Thr Gly Arg Val Gly Ala Ser Glu Ala Ala Leu Phe Leu Lys Lys Ser
        35                  40                  45

ggc ctc tcg gac att atc ctt ggg aag ata tgg gac ttg gcc gat cca     192
Gly Leu Ser Asp Ile Ile Leu Gly Lys Ile Trp Asp Leu Ala Asp Pro
    50                  55                  60

gaa ggt aaa ggg ttc ttg gac aaa cag ggt ttc tat gtt gca ctg aga     240
Glu Gly Lys Gly Phe Leu Asp Lys Gln Gly Phe Tyr Val Ala Leu Arg
65                  70                  75                  80

ctg gtg gcc tgt gca cag agt ggc cat gaa gtt acc ttg agc aat ctg     288
Leu Val Ala Cys Ala Gln Ser Gly His Glu Val Thr Leu Ser Asn Leu
                85                  90                  95

aat ttg agc atg cca ccg cct aaa ttt cac gac acc agc agc cct ctg 336
Asn Leu Ser Met Pro Pro Pro Lys Phe His Asp Thr Ser Ser Pro Leu
            100                 105                 110

atg gtc aca ccg ccc tct gca gag gcc cac tgg gct gtg agg gtg gaa 384
Met Val Thr Pro Pro Ser Ala Glu Ala His Trp Ala Val Arg Val Glu
        115                 120                 125

gaa aag gcc aaa ttt gat ggg att ttt gaa agc ctc ttg ccc atc aat 432
Glu Lys Ala Lys Phe Asp Gly Ile Phe Glu Ser Leu Leu Pro Ile Asn
    130                 135                 140

ggt ttg ctc tct gga gac aaa gtc aag cca gtc ctc atg aac tca aag 480
Gly Leu Leu Ser Gly Asp Lys Val Lys Pro Val Leu Met Asn Ser Lys
145                 150                 155                 160

ctg cct ctt gat gtc ctg ggc agg gtc tgg gac ctc agt gac att gac 528
Leu Pro Leu Asp Val Leu Gly Arg Val Trp Asp Leu Ser Asp Ile Asp
                165                 170                 175

aag gat ggg cac ttg gat cga gat gag ttc gct gtg gcc atg cac ttg 576
Lys Asp Gly His Leu Asp Arg Asp Glu Phe Ala Val Ala Met His Leu
            180                 185                 190

gtg tac cga gcc ctg gag aag gag ccc gtg ccc tcc gcc ctg ccc ccg 624
Val Tyr Arg Ala Leu Glu Lys Glu Pro Val Pro Ser Ala Leu Pro Pro
        195                 200                 205

tcc ctc atc cca ccc tcc aag aga aag aag act gtg ttc cct ggc gcc 672
Ser Leu Ile Pro Pro Ser Lys Arg Lys Lys Thr Val Phe Pro Gly Ala
    210                 215                 220

gtc ccc gtc ctg cct gcc agc ccc cca cca aaa gac agc ctc cgc tcc 720
Val Pro Val Leu Pro Ala Ser Pro Pro Pro Lys Asp Ser Leu Arg Ser
225                 230                 235                 240

acg ccg tcc cac ggc agc gtc agc agc ctc aac agc aca ggg agc ctg 768
Thr Pro Ser His Gly Ser Val Ser Ser Leu Asn Ser Thr Gly Ser Leu
                245                 250                 255

tcc ccc aag cac agc ctc aag caa aca cag cca aca gtg aac tgg gtg 816
Ser Pro Lys His Ser Leu Lys Gln Thr Gln Pro Thr Val Asn Trp Val
            260                 265                 270

gtg ccc gtg gca gac aag atg cga ttt gat gag ata ttc ctg aag acc 864
Val Pro Val Ala Asp Lys Met Arg Phe Asp Glu Ile Phe Leu Lys Thr
        275                 280                 285

gac ctg gac ctg gat ggc tac gtg agt ggc cag gag gtg aag gag atc 912
Asp Leu Asp Leu Asp Gly Tyr Val Ser Gly Gln Glu Val Lys Glu Ile
    290                 295                 300

ttc atg cac tcg ggc ctc acc cag aac ctt cta gca cac ata tgg gcc 960
Phe Met His Ser Gly Leu Thr Gln Asn Leu Leu Ala His Ile Trp Ala
305                 310                 315                 320

ctg gcc gat acg agg caa acg ggg aag tta agc aaa gac caa ttc gcg 1008
Leu Ala Asp Thr Arg Gln Thr Gly Lys Leu Ser Lys Asp Gln Phe Ala
                325                 330                 335

tta gct atg tat ttc att cag cag aag gtc agt aaa ggc atc gac cct 1056
Leu Ala Met Tyr Phe Ile Gln Gln Lys Val Ser Lys Gly Ile Asp Pro
            340                 345                 350

cct caa gtc ctc tcg ccg gac atg gtc ccg cct tcg gag aga ggc acg 1104
Pro Gln Val Leu Ser Pro Asp Met Val Pro Pro Ser Glu Arg Gly Thr
        355                 360                 365

ccc ggc ccg gac agt tca ggc tct ctc ggc tcc ggg gag ttt act ggc 1152
Pro Gly Pro Asp Ser Ser Gly Ser Leu Gly Ser Gly Glu Phe Thr Gly
    370                 375                 380

gtg aag gag ctt gat gac atc agt caa gag att gcc cag tta caa aga 1200
Val Lys Glu Leu Asp Asp Ile Ser Gln Glu Ile Ala Gln Leu Gln Arg
385                 390                 395                 400

gag aaa tat tca ctg gaa caa gac att cga gaa aag gaa gag gca atc 1248
Glu Lys Tyr Ser Leu Glu Gln Asp Ile Arg Glu Lys Glu Glu Ala Ile
                405                 410                 415

aga cag aaa acc agc gag gtg cag gaa tta caa aat gac cta gac cgg 1296
Arg Gln Lys Thr Ser Glu Val Gln Glu Leu Gln Asn Asp Leu Asp Arg
            420                 425                 430

gaa aca agc agt ttg cag gag ctc gag gct cag aaa cag gat gct caa 1344
Glu Thr Ser Ser Leu Gln Glu Leu Glu Ala Gln Lys Gln Asp Ala Gln
        435                 440                 445

gac cgc ctg gac gag atg gac cag cag aag gcc aag ctc cga gac atg 1392
Asp Arg Leu Asp Glu Met Asp Gln Gln Lys Ala Lys Leu Arg Asp Met
    450                 455                 460

ctg agc gac gtc cgg cag aag tgc cag gat gag act cag atg atc tca 1440
Leu Ser Asp Val Arg Gln Lys Cys Gln Asp Glu Thr Gln Met Ile Ser
465                 470                 475                 480

tca ctg aaa acg caa atc caa tct cag gaa tct gac tta aag tcc cag 1488
Ser Leu Lys Thr Gln Ile Gln Ser Gln Glu Ser Asp Leu Lys Ser Gln
                485                 490                 495

gaa gac gat ctg aac cga gcc aag tcg gag ctg aac cga ttg cag cag 1536
Glu Asp Asp Leu Asn Arg Ala Lys Ser Glu Leu Asn Arg Leu Gln Gln
            500                 505                 510

gag gaa acc cag ctg gag cag agc att cag gct ggg cga gtc cag ctg 1584
Glu Glu Thr Gln Leu Glu Gln Ser Ile Gln Ala Gly Arg Val Gln Leu
        515                 520                 525

gaa acc atc atc aag tcc ctg aag tca acg caa gac gaa atc aac cag 1632
Glu Thr Ile Ile Lys Ser Leu Lys Ser Thr Gln Asp Glu Ile Asn Gln
    530                 535                 540

gca agg agc aaa ctt tcc cag ctg cat gaa agc cgc cag gag gcc cac 1680
Ala Arg Ser Lys Leu Ser Gln Leu His Glu Ser Arg Gln Glu Ala His
545                 550                 555                 560

agg agc ctg gag cag tat gac cag gtg ctc gat gga gcc cat ggt gcc 1728
Arg Ser Leu Glu Gln Tyr Asp Gln Val Leu Asp Gly Ala His Gly Ala
                565                 570                 575

agc ctg acc gac ctg gcc aac ctg agc gaa ggc gtc tcc ctg gca gag 1776
Ser Leu Thr Asp Leu Ala Asn Leu Ser Glu Gly Val Ser Leu Ala Glu
            580                 585                 590

agg ggc agt ttt gga gcc atg gat gat cct ttc aaa aat aaa gcc ttg 1824
Arg Gly Ser Phe Gly Ala Met Asp Asp Pro Phe Lys Asn Lys Ala Leu
        595                 600                 605

tta ttt agc aac aac acg caa gag ttg cat ccg gat cct ttc cag aca 1872
Leu Phe Ser Asn Asn Thr Gln Glu Leu His Pro Asp Pro Phe Gln Thr
    610                 615                 620

gaa gac ccc ttc aaa tct gac cca ttt aaa gga gct gac ccc ttc aaa 1920
Glu Asp Pro Phe Lys Ser Asp Pro Phe Lys Gly Ala Asp Pro Phe Lys
625                 630                 635                 640

ggc gac ccg ttc cag aat gac ccc ttt gca gaa cag cag aca act tca 1968
Gly Asp Pro Phe Gln Asn Asp Pro Phe Ala Glu Gln Gln Thr Thr Ser
                645                 650                 655

aca gat cca ttt gga ggg gac cct ttc aaa gaa agt gac cca ttc cgt 2016
Thr Asp Pro Phe Gly Gly Asp Pro Phe Lys Glu Ser Asp Pro Phe Arg
            660                 665                 670

ggc tct gcc act gac gac ttc ttc aag aaa cag aca aag aat gac cca 2064
Gly Ser Ala Thr Asp Asp Phe Phe Lys Lys Gln Thr Lys Asn Asp Pro
        675                 680                 685

ttt acc tcg gat cca ttc acg aaa aac cct tcc tta cct tcg aag ctc 2112
Phe Thr Ser Asp Pro Phe Thr Lys Asn Pro Ser Leu Pro Ser Lys Leu
    690                 695                 700

gac ccc ttt gaa tcc agt gat ccc ttt tca tcc tcc agt gtc tcc tca 2160
Asp Pro Phe Glu Ser Ser Asp Pro Phe Ser Ser Ser Ser Val Ser Ser
705                 710                 715                 720

aaa gga tca gat ccc ttt gga acc tta gat ccc ttc gga agt ggg tcc 2208
Lys Gly Ser Asp Pro Phe Gly Thr Leu Asp Pro Phe Gly Ser Gly Ser
                725                 730                 735

ttc aat agt gct gaa ggc ttt gcc gac ttc agc cag atg tcc aag ccc 2256
Phe Asn Ser Ala Glu Gly Phe Ala Asp Phe Ser Gln Met Ser Lys Pro
            740                 745                 750

cca cct tct ggg cct ttc acc tcc tcc ttg gga ggg gca gga ttc tca 2304
Pro Pro Ser Gly Pro Phe Thr Ser Ser Leu Gly Gly Ala Gly Phe Ser
        755                 760                 765

gat gac ccc ttt aaa agt aaa cag gac act cct gct ctg cct ccg aag 2352
Asp Asp Pro Phe Lys Ser Lys Gln Asp Thr Pro Ala Leu Pro Pro Lys
    770                 775                 780

aaa cct gct cct cca cgg cct aaa ccg ccc agc ggt aaa agt aca cct 2400
Lys Pro Ala Pro Pro Arg Pro Lys Pro Pro Ser Gly Lys Ser Thr Pro
785                 790                 795                 800

gta agc cag ctt ggt tcc gca gac ttt ccc gag gcc ccc gat cca ttc 2448
Val Ser Gln Leu Gly Ser Ala Asp Phe Pro Glu Ala Pro Asp Pro Phe
                805                 810                 815

cag cca ctc ggg gct gac agc ggc gac ccg ttc caa agt aaa aag ggg 2496
Gln Pro Leu Gly Ala Asp Ser Gly Asp Pro Phe Gln Ser Lys Lys Gly
            820                 825                 830

ttt ggg gac ccg ttt agt gga aaa gac cca ttt gtc ccc tcc tct gca 2544
Phe Gly Asp Pro Phe Ser Gly Lys Asp Pro Phe Val Pro Ser Ser Ala
        835                 840                 845

gct aaa cct tct aag gcc tct gcc tcg ggc ttt gca gac ttc acc tct 2592
Ala Lys Pro Ser Lys Ala Ser Ala Ser Gly Phe Ala Asp Phe Thr Ser
    850                 855                 860

gta agt tgagtcctcc gcctccgggc caccccactc ccttccgctt gcagcttccc 2648
Val Ser
865

tgggattttt gtctcctttt aaaggcaaac ctcccagctt ctttagcctc ttggtacctc 2708

acactctctg tccctcgcgt tatttattct acactgccac ttctgtaaga aaaacagttt 2768

ctcaat                                                          2774

<210> 34
<211> 973
<212> DNA
<213> Homo sapiens

<220>
<223> clone name: hfb2063

<220>
<221> CDS
<222> (1)..(807)

<400> 34
gac gcg tgg gcg gtg aag gag gag tgc cgc ctg ctc aat gcc cct cca      48
Asp Ala Trp Ala Val Lys Glu Glu Cys Arg Leu Leu Asn Ala Pro Pro
1               5                   10                  15

gtg cct cct cgg ggt ggc aat ggc agc ggc cgg ctc tcc agc agc ccc      96
Val Pro Pro Arg Gly Gly Asn Gly Ser Gly Arg Leu Ser Ser Ser Pro
            20                  25                  30

ccg gtt ccc cct cgc ttc ccc aag ctg cag ccg gta cat tcc ccc agc      144
Pro Val Pro Pro Arg Phe Pro Lys Leu Gln Pro Val His Ser Pro Ser
        35                  40                  45

tcc agc ctc tcc tac tac tcc tct ggc ctc cag gat ggg gcg ggt tcc      192
Ser Ser Leu Ser Tyr Tyr Ser Ser Gly Leu Gln Asp Gly Ala Gly Ser
    50                  55                  60

cgc agt ggc agt ggc tcc cca tcg ccg gac acc tac tcc ctc tat tgc      240
Arg Ser Gly Ser Gly Ser Pro Ser Pro Asp Thr Tyr Ser Leu Tyr Cys
65                  70                  75                  80

tac cca tgc acc tgg gga gac tgc aag gaa cca gtc ctg gag ccc ttc      288
Tyr Pro Cys Thr Trp Gly Asp Cys Lys Glu Pro Val Leu Glu Pro Phe
                85                  90                  95

gat ccc ttt gag ctg ggg cag ggc agt tct cca gag cct gag ctg ctg 336
Asp Pro Phe Glu Leu Gly Gln Gly Ser Ser Pro Glu Pro Glu Leu Leu
            100                 105                 110

cgt tct cag gag ccc aga gca gtg ggg aca cct ggg cct gga ccc cgc 384
Arg Ser Gln Glu Pro Arg Ala Val Gly Thr Pro Gly Pro Gly Pro Arg
        115                 120                 125

ctt tca cca ctt ggc ccc tcc aag gcc ttt gag cct gaa ggt ttg gtg 432
Leu Ser Pro Leu Gly Pro Ser Lys Ala Phe Glu Pro Glu Gly Leu Val
    130                 135                 140

ctg cac cag gtc ccc acc cca ctg tca cca gct gct ctg cag gga ccc 480
Leu His Gln Val Pro Thr Pro Leu Ser Pro Ala Ala Leu Gln Gly Pro
145                 150                 155                 160

gag gcg gga gga gca ctt ttt cta acc caa ggg cgc ctg gaa ggg cct 528
Glu Ala Gly Gly Ala Leu Phe Leu Thr Gln Gly Arg Leu Glu Gly Pro
                165                 170                 175

cct gcc agt ccc cgg gat gga gcc aca ggc ttt gga gtc cga gat gcc 576
Pro Ala Ser Pro Arg Asp Gly Ala Thr Gly Phe Gly Val Arg Asp Ala
            180                 185                 190

tcc tcc tgg cag ccc cct gct gac ctg tct gca ctc tcc ctg gag gag 624
Ser Ser Trp Gln Pro Pro Ala Asp Leu Ser Ala Leu Ser Leu Glu Glu
        195                 200                 205

gtc tct cgc agt ctg cgt ttc atc ggg ctc tca gag gat gtg gtg agc 672
Val Ser Arg Ser Leu Arg Phe Ile Gly Leu Ser Glu Asp Val Val Ser
    210                 215                 220

ttc ttt gcc cga gaa cgc atc gat ggt agc atc ttt gtg cag ctc agt 720
Phe Phe Ala Arg Glu Arg Ile Asp Gly Ser Ile Phe Val Gln Leu Ser
225                 230                 235                 240

gag gac atc ctg gca gat gac ttc cac ctc acc aag ctg cag gtc aag 768
Glu Asp Ile Leu Ala Asp Asp Phe His Leu Thr Lys Leu Gln Val Lys
                245                 250                 255

aag atc atg cag ttc atc aaa ggc tgg agg ccc aag atc tgaactgccc 817
Lys Ile Met Gln Phe Ile Lys Gly Trp Arg Pro Lys Ile
            260                 265

agctggagct gcacagctgg aatgctggta tgggggcccc aggtacagca ctccggagga 877

gcaggtgctg cctgcaagaa ggatctatgt cgagactgag gctgctcagc agccactggg 937

tggatccagg ggagatgcat gtggaaatgt ggtcct 973

<210> 35
<211> 536
<212> DNA
<213> Homo sapiens

<220>
<223> clone name: hfb2411

<220>
<221> CDS
<222> (1)..(162)

<400> 35
gag gag gaa gag gaa gaa agt gaa aat aaa agt atg gat acg gat ccc 48
Glu Glu Glu Glu Glu Glu Ser Glu Asn Lys Ser Met Asp Thr Asp Pro
1               5                   10                  15

gaa acg ata cga gat gaa gaa gag act gga gat cac tcc atg gac gat 96
Glu Thr Ile Arg Asp Glu Glu Glu Thr Gly Asp His Ser Met Asp Asp
            20                  25                  30

agt tcg gag gat ggg aaa atg gaa acc aaa tca gac cac gag gaa gac 144
Ser Ser Glu Asp Gly Lys Met Glu Thr Lys Ser Asp His Glu Glu Asp
        35                  40                  45

aat atg gaa gat ggc atg taataaacta ctgcatttta agcttcctat 192
Asn Met Glu Asp Gly Met
    50

ttttttttcc agtagtattg ttacctgctt gaaaacactg ctgtgttaag ctgttcatgc 252

acgtgcctga cgcttccagg aagctgtaga gagggacaga aggggcggtt cagccaagac 312

agatgtagac ggagttggag ctgggtattg ttaaaaactg cattatgcaa aaattttgta 372

cagtgttaag gcctaaaaac tgtgtggttc agagactaat tcctgtgttt aatagcattt 432

atactttaag cacaactaga aaattgtaag aattgcactc tacttatgta tcactacaaa 492

ctttaaaaaa ctatgtctaa tttatattaa tacattttaa aaag 536

<210> 36
<211> 1065
<212> DNA
<213> Homo sapiens

<220>
<223> clone name: hfb2436

<220>
<221> CDS
<222> (1)..(672)

<400> 36
tcc tcc gat ccc gct gac ttc gaa cca atc cca tct ttt tct ggg ttt 48
Ser Ser Asp Pro Ala Asp Phe Glu Pro Ile Pro Ser Phe Ser Gly Phe
1               5                   10                  15

ccg tta gat tct ccc aaa acc ttg gtg ctt gac ttt gag aca gag ggt 96
Pro Leu Asp Ser Pro Lys Thr Leu Val Leu Asp Phe Glu Thr Glu Gly
            20                  25                  30

gaa cga aac tca cct aat ccc agg agt gtt agg atc cct tct cct aac 144
Glu Arg Asn Ser Pro Asn Pro Arg Ser Val Arg Ile Pro Ser Pro Asn
        35                  40                  45

att ttg aaa act gga ctt aca gaa aat gtt gac cgt ggc ttg ggg ggc 192
Ile Leu Lys Thr Gly Leu Thr Glu Asn Val Asp Arg Gly Leu Gly Gly

    50                  55                  60

cta gag gga aca cac cag gcc ctt gac ctg tta gca gga gga atg atg 240
Leu Glu Gly Thr His Gln Ala Leu Asp Leu Leu Ala Gly Gly Met Met
65                  70                  75                  80

cct gag gaa gta aaa gaa tct tcc caa tta gac aaa caa gag tca ctc 288
Pro Glu Glu Val Lys Glu Ser Ser Gln Leu Asp Lys Gln Glu Ser Leu
                85                  90                  95

gga ttg gaa tta aaa aca att aat tct gca ggc ctt ggg cca tct cct 336
Gly Leu Glu Leu Lys Thr Ile Asn Ser Ala Gly Leu Gly Pro Ser Pro
            100                 105                 110

tgc ctt cca gac ctt gat gac ttt gtc aca cgg acc tct gga gtt caa 384
Cys Leu Pro Asp Leu Asp Asp Phe Val Thr Arg Thr Ser Gly Val Gln
        115                 120                 125

aaa gat aaa ctg tgt tct cca ctc tct gag cca ggt gac cct tct aaa 432
Lys Asp Lys Leu Cys Ser Pro Leu Ser Glu Pro Gly Asp Pro Ser Lys
    130                 135                 140

tgt agt tcc ctg gag ttg ggg cca tta cag cta gaa ata tcg aat gca 480
Cys Ser Ser Leu Glu Leu Gly Pro Leu Gln Leu Glu Ile Ser Asn Ala
145                 150                 155                 160

tcc acc aca gag gtg gca att ctg caa gta gat gat gac agt ggc gac 528
Ser Thr Thr Glu Val Ala Ile Leu Gln Val Asp Asp Asp Ser Gly Asp
                165                 170                 175

cct ctg aat ttg gtt aaa gct cca gtg tca agg tcc cct cca agg gag 576
Pro Leu Asn Leu Val Lys Ala Pro Val Ser Arg Ser Pro Pro Arg Glu
            180                 185                 190

cag gta att gaa gac aat atg gtc cct cag gga atg cct gaa cag gaa 624
Gln Val Ile Glu Asp Asn Met Val Pro Gln Gly Met Pro Glu Gln Glu
        195                 200                 205

act aca gtt ggt gcc atc cag gac cac aca gaa tcc agt gtt cac aac 672
Thr Thr Val Gly Ala Ile Gln Asp His Thr Glu Ser Ser Val His Asn

210                 215                 220

taagaataaa tacctagagc tacttgtgag tgaatcattg gcttctagaa atcagatgcc 732

cagatgatcc aagactagtt tgttatctca tctggaacct acaccaagag atgcagtcag 792

catcttaaag taaatgttca tggaagctaa aaaatttacc tcctttcctt ttccctttcc 852

ccttaaaaca ccagacaaat cgataacaac ttatatataa tatatatata tctgtaaaaa 912

tgtattttgt taattttttt ggccaattta ttttctccca ctttgtcatt aaaatcacat 972

gtctaactgg ctcaccacat agtgtgcttt gattgtattt tggctttgac tctgccattt 1032

ctgaaatgtg ggggaggatg gaggcagctg cct                              1065

<210> 37
<211> 910
<212> DNA
<213> Homo sapiens

<220>
<223> clone name: hfb2122

<220>
<221> CDS
<222> (1)..(672)

<400> 37
gag cgc agc ctg gca gag atg gag tcc tcg cac cag cag gtg atg gag    48
Glu Arg Ser Leu Ala Glu Met Glu Ser Ser His Gln Gln Val Met Glu
1               5                   10                  15

gag ctg cag cgg cac cac gag cgg gag ctg cag cgc ctg cag cag gag    96
Glu Leu Gln Arg His His Glu Arg Glu Leu Gln Arg Leu Gln Gln Glu
            20                  25                  30

aag gag tgg ctc ctg gct gag gag acg gca gcc acg gcc tca gcc att    144
Lys Glu Trp Leu Leu Ala Glu Glu Thr Ala Ala Thr Ala Ser Ala Ile

        35                  40                  45

gaa gcc atg aag aag gcc tac cag gaa gag ctg agc cga gag ctg agc 192
Glu Ala Met Lys Lys Ala Tyr Gln Glu Glu Leu Ser Arg Glu Leu Ser
    50                  55                  60

aaa aca cgg agt ctc cag cag ggc ccg gat ggc ctc cgg aag cag cac 240
Lys Thr Arg Ser Leu Gln Gln Gly Pro Asp Gly Leu Arg Lys Gln His
65                  70                  75                  80

cag tca gat gtg gag gca ctg aag cga gag ctg cag gtg cta tcg gag 288
Gln Ser Asp Val Glu Ala Leu Lys Arg Glu Leu Gln Val Leu Ser Glu
                85                  90                  95

cag tac tcg cag aag tgc ctg gag att ggg gca ctc atg cgg cag gct 336
Gln Tyr Ser Gln Lys Cys Leu Glu Ile Gly Ala Leu Met Arg Gln Ala
            100                 105                 110

gag gag cgc gag cac acg ctg cgc cgc tgc cag cag gag ggc cag gag 384
Glu Glu Arg Glu His Thr Leu Arg Arg Cys Gln Gln Glu Gly Gln Glu
        115                 120                 125

ctg ctg cgc cac aac cag gag ctg cat ggg ccg cct gtc aga gga gat 432
Leu Leu Arg His Asn Gln Glu Leu His Gly Pro Pro Val Arg Gly Asp
    130                 135                 140

aga cca gct gcg cgg ctt cat tgc ctc gca ggg cat ggg caa tgg ctg 480
Arg Pro Ala Ala Arg Leu His Cys Leu Ala Gly His Gly Gln Trp Leu
145                 150                 155                 160

cgg gcg cag caa cga gcg gag ttc ctg cga gct aga ggt gct gct tcg 528
Arg Ala Gln Gln Arg Ala Glu Phe Leu Arg Ala Arg Gly Ala Ala Ser
                165                 170                 175

cgt aaa aga aaa cga act cca gta cct aaa gaa gga ggt gca gtg cct 576
Arg Lys Arg Lys Arg Thr Pro Val Pro Lys Glu Gly Gly Ala Val Pro
            180                 185                 190

ccg gga cga gct cca gat gat gca gaa gga caa gcg ctt cac ctc ggg 624
Pro Gly Arg Ala Pro Asp Asp Ala Glu Gly Gln Ala Leu His Leu Gly

195                 200                 205

aaa gta cca gga cgt cta tgt gga gct gag cca cat caa gac acg gtc    672
Lys Val Pro Gly Arg Leu Cys Gly Ala Glu Pro His Gln Asp Thr Val
    210                 215                 220

tgagcgggag atcgagcagc tgaaggagca cctgcgtctt gccatggccg ccctccagga 732

gaaggagtcg atgcgcaaca gcctggctga gtagaggtcc cgcccagctg cagaccctcc 792

aggctggagg accagccgcc ctccttccct cctggatgga agtaaaaagc caagctttct 852

ccccaccctc tgtgggccac acgtgcactt gcacccacca cacacacaca cacacaca   910

<210> 38
<211> 1332
<212> DNA
<213> Homo sapiens

<220>
<223> clone name: hfb2157

<220>
<221> CDS
<222> (1)..(1224)

<400> 38
aga aac cca gaa atc agt cac ctg ctc aac aac cca gac ata atg agg    48
Arg Asn Pro Glu Ile Ser His Leu Leu Asn Asn Pro Asp Ile Met Arg
  1               5                  10                  15

cag aca ctc gaa att gcc agg aat cca gcc atg atg caa gag atg atg    96
Gln Thr Leu Glu Ile Ala Arg Asn Pro Ala Met Met Gln Glu Met Met
             20                  25                  30

aga aat caa gac ctg gct ctt agc aat cta gaa agc atc cca ggt ggc    144
Arg Asn Gln Asp Leu Ala Leu Ser Asn Leu Glu Ser Ile Pro Gly Gly
         35                  40                  45

tat aat gct tta cgg cgc atg tac act gac att caa gag ccg atg ctg 192
Tyr Asn Ala Leu Arg Arg Met Tyr Thr Asp Ile Gln Glu Pro Met Leu
    50                  55                  60

aat gcc gca caa gag cag ttt ggg ggt aat cca ttt gcc tcc gtg ggg 240
Asn Ala Ala Gln Glu Gln Phe Gly Gly Asn Pro Phe Ala Ser Val Gly
65                  70                  75                  80

agt agt tcc tcc tct ggg gaa ggt acg cag cct tcc cgc aca gaa aat 288
Ser Ser Ser Ser Ser Gly Glu Gly Thr Gln Pro Ser Arg Thr Glu Asn
                85                  90                  95

cgc gat cca cta ccc aat cca tgg gca cca ccg cca gct acc cag agt 336
Arg Asp Pro Leu Pro Asn Pro Trp Ala Pro Pro Pro Ala Thr Gln Ser
            100                 105                 110

tct gca act acc agc acg acc aca agc act ggt agt ggg tct ggc aat 384
Ser Ala Thr Thr Ser Thr Thr Thr Ser Thr Gly Ser Gly Ser Gly Asn
        115                 120                 125

agt tcc agc aat gct act ggg aac acc gtt gct gcc gct aat tat gtc 432
Ser Ser Ser Asn Ala Thr Gly Asn Thr Val Ala Ala Ala Asn Tyr Val
    130                 135                 140

gcc agc atc ttt agt acc cca ggc atg cag agc ctg ctg caa cag ata 480
Ala Ser Ile Phe Ser Thr Pro Gly Met Gln Ser Leu Leu Gln Gln Ile
145                 150                 155                 160

act gaa aac ccc cag ctg att cag aat atg ctg tcg gcg ccc tac atg 528
Thr Glu Asn Pro Gln Leu Ile Gln Asn Met Leu Ser Ala Pro Tyr Met
                165                 170                 175

aga agc atg atg cag tcg ctg agc cag aat cca gat ttg gct gca cag 576
Arg Ser Met Met Gln Ser Leu Ser Gln Asn Pro Asp Leu Ala Ala Gln
            180                 185                 190

atg atg ctg aat agc ccg ctg ttt act gca aat cct cag ctg cag gag 624
Met Met Leu Asn Ser Pro Leu Phe Thr Ala Asn Pro Gln Leu Gln Glu
        195                 200                 205

cag atg cgg cca cag ctc cca gcc ttc ctg cag cag atg cag aat cca 672
Gln Met Arg Pro Gln Leu Pro Ala Phe Leu Gln Gln Met Gln Asn Pro
    210                 215                 220

gac aca cta tca gcc atg tca aac cca aga gca atg cag gct tta atg 720
Asp Thr Leu Ser Ala Met Ser Asn Pro Arg Ala Met Gln Ala Leu Met
225                 230                 235                 240

cag atc cag cag ggg cta cag aca tta gcc act gaa gca cct ggc ctg 768
Gln Ile Gln Gln Gly Leu Gln Thr Leu Ala Thr Glu Ala Pro Gly Leu
                245                 250                 255

att ccg agc ttc act cca ggt gtg ggg gtg ggg gtg ctg gga acc gct 816
Ile Pro Ser Phe Thr Pro Gly Val Gly Val Gly Val Leu Gly Thr Ala
            260                 265                 270

ata ggc cct gta ggc cca gtc acc ccc ata ggc ccc ata ggc cct ata 864
Ile Gly Pro Val Gly Pro Val Thr Pro Ile Gly Pro Ile Gly Pro Ile
        275                 280                 285

gtc cct ttt acc ccc ata ggc ccc att ggg ccc ata gga ccc act ggc 912
Val Pro Phe Thr Pro Ile Gly Pro Ile Gly Pro Ile Gly Pro Thr Gly
    290                 295                 300

cct gca gcc ccc cct ggc tcc acc ggc tct ggt ggc ccc acg ggg cct 960
Pro Ala Ala Pro Pro Gly Ser Thr Gly Ser Gly Gly Pro Thr Gly Pro
305                 310                 315                 320

act gtg tcc agc gct gca cct aga gaa acc acg agt cct aca tca gaa 1008
Thr Val Ser Ser Ala Ala Pro Arg Glu Thr Thr Ser Pro Thr Ser Glu
                325                 330                 335

tct gga ccc aac cag cag ttc att cag caa atg gtg cag gcc ctg gct 1056
Ser Gly Pro Asn Gln Gln Phe Ile Gln Gln Met Val Gln Ala Leu Ala
            340                 345                 350

gga gca aat gct cca cag ctg ccg aat cca gaa gtc aga ttt cag caa 1104
Gly Ala Asn Ala Pro Gln Leu Pro Asn Pro Glu Val Arg Phe Gln Gln
        355                 360                 365

caa ctg gaa cag ctc aac gca atg ggg ttc tta aac cgt gaa gca aac 1152
Gln Leu Glu Gln Leu Asn Ala Met Gly Phe Leu Asn Arg Glu Ala Asn
    370                 375                 380

ttg cag gcc cta ata gca aca gga ggc gac atc aat gca gcc att gaa 1200
Leu Gln Ala Leu Ile Ala Thr Gly Gly Asp Ile Asn Ala Ala Ile Glu
385                 390                 395                 400

agg ctg ctg ggc tcc cag cca tcg taatcacatt tctgtacctg gaaaaaaaat 1254
Arg Leu Leu Gly Ser Gln Pro Ser
                405

gtatcttatt tttgataatg gctcttaaat ctttaaacac acacacaaaa tcgtctttac 1314

tttcattttg attctttt 1332

<210> 39
<211> 1605
<212> DNA
<213> Homo sapiens

<220>
<223> clone name: hfb2216

<220>
<221> CDS
<222> (1)..(1458)

<400> 39
aat gcc gag agg atg gag agc atc ctg cag gca ctg gag gat att cag 48
Asn Ala Glu Arg Met Glu Ser Ile Leu Gln Ala Leu Glu Asp Ile Gln
1               5                   10                  15

ctg gat ctg gag gca gtg aac atc aag gca ggc aaa gcc ttc ctg cgt 96
Leu Asp Leu Glu Ala Val Asn Ile Lys Ala Gly Lys Ala Phe Leu Arg
            20                  25                  30

ctc aag cgc aag ttc atc cag atg cga aga ccc ttc ctg gag cgc aga 144
Leu Lys Arg Lys Phe Ile Gln Met Arg Arg Pro Phe Leu Glu Arg Arg

        35                  40                  45

gac ctc atc atc cag cat atc cca ggc ttc tgg gtc aaa gca ttc ctc 192
Asp Leu Ile Ile Gln His Ile Pro Gly Phe Trp Val Lys Ala Phe Leu
    50                  55                  60

aac cac ccc aga att tca att ttg atc aac cga cgt gat gaa gac att 240
Asn His Pro Arg Ile Ser Ile Leu Ile Asn Arg Arg Asp Glu Asp Ile
65                  70                  75                  80

ttc cgc tac ttg acc aat ctg cag gta cag gat ctc aga cat atc tcc 288
Phe Arg Tyr Leu Thr Asn Leu Gln Val Gln Asp Leu Arg His Ile Ser
                85                  90                  95

atg ggc tac aaa atg aag ctg tac ttc cag act aac ccc tac ttc aca 336
Met Gly Tyr Lys Met Lys Leu Tyr Phe Gln Thr Asn Pro Tyr Phe Thr
            100                 105                 110

aac atg gtg att gtc aag gag ttc cag cgc aac cgc tca ggc cgg ctg 384
Asn Met Val Ile Val Lys Glu Phe Gln Arg Asn Arg Ser Gly Arg Leu
        115                 120                 125

gtg tct cac tca acc cca atc cgc tgg cac cgg ggc cag gaa ccc cag 432
Val Ser His Ser Thr Pro Ile Arg Trp His Arg Gly Gln Glu Pro Gln
    130                 135                 140

gcc cgt cgt cac ggg aac cag gat gcg agc cac agc ttt ttc agc tgg 480
Ala Arg Arg His Gly Asn Gln Asp Ala Ser His Ser Phe Phe Ser Trp
145                 150                 155                 160

ttc tca aac cat agc ctc cca gag gct gac agg att gct gag att atc 528
Phe Ser Asn His Ser Leu Pro Glu Ala Asp Arg Ile Ala Glu Ile Ile
                165                 170                 175

aag aat gat ctg tgg gtt aac cct cta cgc tac tac ctg aga gaa agg 576
Lys Asn Asp Leu Trp Val Asn Pro Leu Arg Tyr Tyr Leu Arg Glu Arg
            180                 185                 190

ggc tcc agg ata aag aga aag aag caa gaa atg aag aaa cgt aaa acc 624
Gly Ser Arg Ile Lys Arg Lys Lys Gln Glu Met Lys Lys Arg Lys Thr

        195                 200                 205

agg ggc aga tgt gag gtg gtg atc atg gaa gac gcc cct gac tat tat 672
Arg Gly Arg Cys Glu Val Val Ile Met Glu Asp Ala Pro Asp Tyr Tyr
    210                 215                 220

gca gtg gaa gac att ttc agc gag atc tca gac att gat gag aca att 720
Ala Val Glu Asp Ile Phe Ser Glu Ile Ser Asp Ile Asp Glu Thr Ile
225                 230                 235                 240

cat gac atc aag atc tct gac ttc atg gag acc acc gac tac ttc gag 768
His Asp Ile Lys Ile Ser Asp Phe Met Glu Thr Thr Asp Tyr Phe Glu
                245                 250                 255

acc act gac aat gag ata act gac atc aat gag aac atc tgc gac agc 816
Thr Thr Asp Asn Glu Ile Thr Asp Ile Asn Glu Asn Ile Cys Asp Ser
            260                 265                 270

gag aat cct gac cac aat gag gtc ccc aac aac gag acc act gat aac 864
Glu Asn Pro Asp His Asn Glu Val Pro Asn Asn Glu Thr Thr Asp Asn
        275                 280                 285

aac gag agt gct gat gac cac gaa acc act gac aac aat gag agt gca 912
Asn Glu Ser Ala Asp Asp His Glu Thr Thr Asp Asn Asn Glu Ser Ala
    290                 295                 300

gat gac aac aac gag aat cct gaa gac aat aac aag aac act gat gac 960
Asp Asp Asn Asn Glu Asn Pro Glu Asp Asn Asn Lys Asn Thr Asp Asp
305                 310                 315                 320

aac gaa gag aac cct aac aac aac gag aac act tac ggc aac aac ttc 1008
Asn Glu Glu Asn Pro Asn Asn Asn Glu Asn Thr Tyr Gly Asn Asn Phe
                325                 330                 335

ttc aaa ggt ggc ttc tgg ggc agc cat ggc aac aac cag gac agc agc 1056
Phe Lys Gly Gly Phe Trp Gly Ser His Gly Asn Asn Gln Asp Ser Ser
            340                 345                 350

gac agt gac aat gaa gca gat gag gcc agt gat gat gaa gat aat gat 1104
Asp Ser Asp Asn Glu Ala Asp Glu Ala Ser Asp Asp Glu Asp Asn Asp

        355                 360                 365

ggc aac gaa ggt gac aat gag ggc agt gat gat gat ggc aat gaa ggt 1152
Gly Asn Glu Gly Asp Asn Glu Gly Ser Asp Asp Asp Gly Asn Glu Gly
    370                 375                 380

gac aat gaa ggc agc gat gat gac gac aga gac att gag tac tat gag 1200
Asp Asn Glu Gly Ser Asp Asp Asp Asp Arg Asp Ile Glu Tyr Tyr Glu
385                 390                 395                 400

aaa ggt att gaa gac ttt gac agg gat cag gct gac tac gag gac gtg 1248
Lys Gly Ile Glu Asp Phe Asp Arg Asp Gln Ala Asp Tyr Glu Asp Val
                405                 410                 415

ata gag atc atc tca gac gaa tca gtg gaa gaa gag ggc att gag gaa 1296
Ile Glu Ile Ile Ser Asp Glu Ser Val Glu Glu Glu Gly Ile Glu Glu
            420                 425                 430

ggc atc cag caa gat gag gac atc tat gag gaa gga aac tat gag gag 1344
Gly Ile Gln Gln Asp Glu Asp Ile Tyr Glu Glu Gly Asn Tyr Glu Glu
        435                 440                 445

gaa gga agt gaa gat gtc tgg gaa gaa ggg gaa gat tcg gac gac tct 1392
Glu Gly Ser Glu Asp Val Trp Glu Glu Gly Glu Asp Ser Asp Asp Ser
    450                 455                 460

gac cta gag gat gtg ctt cag gtc cca aac ggt tgg gcc aat ccg ggg 1440
Asp Leu Glu Asp Val Leu Gln Val Pro Asn Gly Trp Ala Asn Pro Gly
465                 470                 475                 480

aag agg ggg aaa acc gga taagggtttt cccctttgg ggatcacctc 1488
Lys Arg Gly Lys Thr Gly
                485

tctgtatccc ccacccacta tcccatttgc cctcctcctc agctagggcc acgcggaccc 1548

acattgcact tctggggggt gaccgacttc gtacacgggt ttaaagttta ttttttt 1605

<210> 40

<211> 532
<212> DNA
<213> Homo sapiens

<220>
<223> clone name: hfb2025

<220>
<221> CDS
<222> (1)..(201)

<400> 40
ata gca gag aca ggg gtt agt ggc ggt gtg gac cgg agg gag gtt cag 48
Ile Ala Glu Thr Gly Val Ser Gly Gly Val Asp Arg Arg Glu Val Gln
1               5                   10                  15

cgc ctt cgc agg cgg aac cag cag ttg gag gaa gag aac aat ctc ttg 96
Arg Leu Arg Arg Arg Asn Gln Gln Leu Glu Glu Glu Asn Asn Leu Leu
            20                  25                  30

cgg ctg aaa gtg gac atc tta tta gac atg ctt tca gag tcc act gcc 144
Arg Leu Lys Val Asp Ile Leu Leu Asp Met Leu Ser Glu Ser Thr Ala
        35                  40                  45

gaa tcc cac tta atg gag aag gaa ctg gat gaa ctg agg atc agc cgg 192
Glu Ser His Leu Met Glu Lys Glu Leu Asp Glu Leu Arg Ile Ser Arg
    50                  55                  60

aag aga aaa tgaagacccc agagacattt attggggagt aggatgtggc 241
Lys Arg Lys
65

tgagtgcttt ttttttggcc agactagcgg attcagtcct ggaagagagt atcatataat 301

gagacccaca ggcactggca cccttgggtt ggcaatagaa ggtgacatgg aatggagaaa 361

accaagattc cagatgggga tagtaactag aaggtgcttc agatgcactg cctgcgggtg 421

ccagtctgaa aaccagaccg cacagaggcc tggggctgct gatgagcttt tcggtgctct 481

cacacacaag ctcgcaaaca cacatgtccc agaatagctc tgtgggtgtg t          532

<210> 41
<211> 519
<212> DNA
<213> Homo sapiens

<220>
<223> clone name: hfb2281

<220>
<221> CDS
<222> (1)..(324)

<400> 41
gag ggc ccc ttg gac agc ccc tcg gag ggc cac agt tat cag aac agc     48
Glu Gly Pro Leu Asp Ser Pro Ser Glu Gly His Ser Tyr Gln Asn Ser
1               5                   10                  15

ggt ctg gac cac ttc caa aac agc aac ata gac cag agc ttc tgg gag     96
Gly Leu Asp His Phe Gln Asn Ser Asn Ile Asp Gln Ser Phe Trp Glu
            20                  25                  30

acc ttt gga agt gct gag ccc acc aag acc cgc aag tcc ccg agc agc     144
Thr Phe Gly Ser Ala Glu Pro Thr Lys Thr Arg Lys Ser Pro Ser Ser
        35                  40                  45

gac agc tgg acg tgc gcg gac acc tcc acc gag agg agg agc tcg gac     192
Asp Ser Trp Thr Cys Ala Asp Thr Ser Thr Glu Arg Arg Ser Ser Asp
    50                  55                  60

agc tgg gag gtg tgg ggc tcg gcc tcc acc aac agg aac agc aac agc     240
Ser Trp Glu Val Trp Gly Ser Ala Ser Thr Asn Arg Asn Ser Asn Ser
65                  70                  75                  80

gac ggc ggg gag ggc ggg gag ggc acc aag aag gca gtg ccg ccg gcc     288
Asp Gly Gly Glu Gly Gly Glu Gly Thr Lys Lys Ala Val Pro Pro Ala
                85                  90                  95

gtg ccc act gat gat ggc tgg gac aac cag aac tgg tagggcccac     334
Val Pro Thr Asp Asp Gly Trp Asp Asn Gln Asn Trp
            100                 105

tgcgcccccg tccccagcgc ccccgggcga cttcgtgttt gcactctgcc ctcgtcgttc 394

ctcctccttc catttgaccc aagaatcagc aactgcagtg tgaggacagc gtctcgggag 454

gcacgaccct agggagaccc gggtgtgcgc ccgcctgcgc gttggggagt cttcggtgcg 514

tgggg                                                             519

<210> 42
<211> 1630
<212> DNA
<213> Homo sapiens

<220>
<223> clone name: hfb2060

<220>
<221> CDS
<222> (1)..(1293)

<400> 42
aat tct cca tat tct ctc tta gag gtg ctg tgt cct cga tgt gat att     48
Asn Ser Pro Tyr Ser Leu Leu Glu Val Leu Cys Pro Arg Cys Asp Ile
  1               5                  10                  15

ggc ctt ttg gga ttg aaa agg aag cag aga aag tta aag gag ccc ttg     96
Gly Leu Leu Gly Leu Lys Arg Lys Gln Arg Lys Leu Lys Glu Pro Leu
             20                  25                  30

cca aat gga aag aaa aaa agc acc aaa tta gag agt ctg agt gac ctg     144
Pro Asn Gly Lys Lys Lys Ser Thr Lys Leu Glu Ser Leu Ser Asp Leu
         35                  40                  45

gag aga gct tta act gaa agc aag gag act gct tca gct atg gca act     192
Glu Arg Ala Leu Thr Glu Ser Lys Glu Thr Ala Ser Ala Met Ala Thr

50                  55                  60

ctt ggg gaa gct cgc cta agg gaa atg gaa gcc ttg cag tct ctc aga    240
Leu Gly Glu Ala Arg Leu Arg Glu Met Glu Ala Leu Gln Ser Leu Arg
65                  70                  75                  80

ctc gcc aat gaa ggg aaa ttg ctg tcg cct ctc caa gat gtg gat ata    288
Leu Ala Asn Glu Gly Lys Leu Leu Ser Pro Leu Gln Asp Val Asp Ile
                85                  90                  95

aaa atc tgc cta tgt cag aag gcc cca gct gcc cct atg att caa tgt    336
Lys Ile Cys Leu Cys Gln Lys Ala Pro Ala Ala Pro Met Ile Gln Cys
            100                 105                 110

gaa ctc tgc agg gat gct ttc cac acc agt tgt gtg gcg gta ccc agt    384
Glu Leu Cys Arg Asp Ala Phe His Thr Ser Cys Val Ala Val Pro Ser
        115                 120                 125

att tca cag ggc ctg cga atc tgg ctt tgt ccc cat tgt cgg agg tca    432
Ile Ser Gln Gly Leu Arg Ile Trp Leu Cys Pro His Cys Arg Arg Ser
    130                 135                 140

gag aaa cct cca tta gag aaa att ctg ccc ctg ctc gcc tcc ctt cag    480
Glu Lys Pro Pro Leu Glu Lys Ile Leu Pro Leu Leu Ala Ser Leu Gln
145                 150                 155                 160

cgt atc cga gtt cgc ctt cct gag gga gat gca ctt cga tat atg att    528
Arg Ile Arg Val Arg Leu Pro Glu Gly Asp Ala Leu Arg Tyr Met Ile
                165                 170                 175

gaa aga acc gtg aac tgg cag cac aga gcc cag caa ctg ctt tcg tca    576
Glu Arg Thr Val Asn Trp Gln His Arg Ala Gln Gln Leu Leu Ser Ser
            180                 185                 190

ggg aat ctt aaa ttt gtg caa gat cga gtg ggc tca gga ctg tta tat    624
Gly Asn Leu Lys Phe Val Gln Asp Arg Val Gly Ser Gly Leu Leu Tyr
        195                 200                 205

agc aga tgg caa gcc tca gca gga cag gtg tca gac aca aac aag gta    672
Ser Arg Trp Gln Ala Ser Ala Gly Gln Val Ser Asp Thr Asn Lys Val

    210                 215                 220

tct caa cct cct ggc aca aca tca ttt tct ttg cct gat gac tgg gac 720
Ser Gln Pro Pro Gly Thr Thr Ser Phe Ser Leu Pro Asp Asp Trp Asp
225                 230                 235                 240

aac aga acc tca tat ttg cac tcc ccc ttc tca act gga cga agt tgt 768
Asn Arg Thr Ser Tyr Leu His Ser Pro Phe Ser Thr Gly Arg Ser Cys
                245                 250                 255

atc ccc ctc cat ggt gtt agt cca gaa gtg aat gaa cta ttg atg gaa 816
Ile Pro Leu His Gly Val Ser Pro Glu Val Asn Glu Leu Leu Met Glu
            260                 265                 270

gcc cag ctg ctc cag gta tcc ctt cct gaa att cag gaa ctt tac cag 864
Ala Gln Leu Leu Gln Val Ser Leu Pro Glu Ile Gln Glu Leu Tyr Gln
        275                 280                 285

act tta ctt gca aag cca agc cct gct cag cag act gac cga agc tca 912
Thr Leu Leu Ala Lys Pro Ser Pro Ala Gln Gln Thr Asp Arg Ser Ser
    290                 295                 300

cca gtg aga ccc agc agt gag aag aat gac tgt tgc cga ggg aag cga 960
Pro Val Arg Pro Ser Ser Glu Lys Asn Asp Cys Cys Arg Gly Lys Arg
305                 310                 315                 320

gat gga att aac agt ctt gag aga aaa ctg aag aga cgc ctg gaa aga 1008
Asp Gly Ile Asn Ser Leu Glu Arg Lys Leu Lys Arg Arg Leu Glu Arg
                325                 330                 335

gag ggc ctc tcc agt gag cgg tgg gaa cga gtt aag aaa atg cgg acc 1056
Glu Gly Leu Ser Ser Glu Arg Trp Glu Arg Val Lys Lys Met Arg Thr
            340                 345                 350

ccc aaa aag aag aaa atc aaa ctg agc cac ccc aag gac atg aac aat 1104
Pro Lys Lys Lys Lys Ile Lys Leu Ser His Pro Lys Asp Met Asn Asn
        355                 360                 365

ttc aag tta gag aga gag cgt agc tat gaa act cat tcc ctg ccc tca 1152
Phe Lys Leu Glu Arg Glu Arg Ser Tyr Glu Thr His Ser Leu Pro Ser

370                 375                 380

gac aca tcc tat tcc gaa cag gaa gac tct gag gat gaa gat gcc atc    1200
Asp Thr Ser Tyr Ser Glu Gln Glu Asp Ser Glu Asp Glu Asp Ala Ile
385                 390                 395                 400

tgc cca gct gtg agc tgc ctg cag cca gaa gga gat gag gtc agt gag    1248
Cys Pro Ala Val Ser Cys Leu Gln Pro Glu Gly Asp Glu Val Ser Glu
                405                 410                 415

gtt tgg gcc ata gag gac gtg ctt tcc ctt aac tca gaa acc ctg        1293
Val Trp Ala Ile Glu Asp Val Leu Ser Leu Asn Ser Glu Thr Leu
            420                 425                 430

taagctagac ctcatctcgc tgggtctttc tcctctgctt atctgctttg agctgatctg 1353

aatggattgc tcagcccttc ccccactgat atctgtgctc ttaccatcgt tcctgggtct 1413

cctccctttt cttgaagaaa ctcttggtct tccctttttc cttttcttgc tcttccagct 1473

ttcaccttcc cggcgctaag aaggtgggag ggatgctgat gtgatgccct ttggcttact 1533

tatcacaggg cagcgctggc ttaaccccaa agcagacacg acttgaagaa tgactttgac 1593

tcggttgctt tggctagggg gccagctaca ctcaatt                          1630

<210> 43
<211> 30
<212> DNA
<213> Artificial Sequence

<220>
<223> Description of Artificial Sequence: Artificially
      synthesized primer sequence

<400> 43
tttgaattcg ccaattttaa tcaaagtggg                                    30

<210> 44
<211> 32
<212> DNA
<213> Artificial Sequence

<220>
<223> Description of Artificial Sequence: Artificially
      synthesized primer sequence

<400> 44
tagcatctat gactttttgg ggcgttcaag tg                                32


<210> 45
<211> 342
<212> DNA
<213> Saccharomyces cerevisiae

<220>
<221> CDS
<222> (1)..(342)

<220>
<221> domain
<222> (1)..(342)

<400> 45
gcc aat ttt aat caa agt ggg aat att gct gat agc tca ttg tcc ttc    48
Ala Asn Phe Asn Gln Ser Gly Asn Ile Ala Asp Ser Ser Leu Ser Phe
 1               5                  10                  15

act ttc act aac agt agc aac ggt ccg aac ctc ata aca act caa aca    96
Thr Phe Thr Asn Ser Ser Asn Gly Pro Asn Leu Ile Thr Thr Gln Thr
            20                  25                  30

aat tct caa gcg ctt tca caa cca att gcc tcc tct aac gtt cat gat    144
Asn Ser Gln Ala Leu Ser Gln Pro Ile Ala Ser Ser Asn Val His Asp
        35                  40                  45

aac ttc atg aat aat gaa atc acg gct agt aaa att gat gat ggt aat    192

Asn Phe Met Asn Asn Glu Ile Thr Ala Ser Lys Ile Asp Asp Gly Asn
    50                  55                  60

aat tca aaa cca ctg tca cct ggt tgg acg gac caa act gcg tat aac 240
Asn Ser Lys Pro Leu Ser Pro Gly Trp Thr Asp Gln Thr Ala Tyr Asn
65                  70                  75                  80

gcg ttt gga atc act aca ggg atg ttt aat acc act aca atg gat gat 288
Ala Phe Gly Ile Thr Thr Gly Met Phe Asn Thr Thr Thr Met Asp Asp
                85                  90                  95

gta tat aac tat cta ttc gat gat gaa gat acc cca cca aac cca aaa 336
Val Tyr Asn Tyr Leu Phe Asp Asp Glu Asp Thr Pro Pro Asn Pro Lys
            100                 105                 110

aaa gag 342
Lys Glu

<210> 46
<211> 609
<212> DNA
<213> Escherichia coli

<220>
<221> CDS
<222> (1)..(606)

<400> 46
atg aaa gcg tta acg gcc agg caa caa gag gtg ttt gat ctc atc cgt 48
Met Lys Ala Leu Thr Ala Arg Gln Gln Glu Val Phe Asp Leu Ile Arg
1               5                   10                  15

gat cac atc agc cag aca ggt atg ccg ccg acg cgt gcg gaa atc gcg 96
Asp His Ile Ser Gln Thr Gly Met Pro Pro Thr Arg Ala Glu Ile Ala
            20                  25                  30

cag cgt ttg ggg ttc cgt tcc cca aac gcg gct gaa gaa cat ctg aag 144
Gln Arg Leu Gly Phe Arg Ser Pro Asn Ala Ala Glu Glu His Leu Lys
        35                  40                  45

gcg ctg gca cgc aaa ggc gtt att gaa att gtt tcc ggc gca tca cgc     192
Ala Leu Ala Arg Lys Gly Val Ile Glu Ile Val Ser Gly Ala Ser Arg
     50                  55                  60

ggg att cgt ctg ttg cag gaa gag gaa gaa ggg ttg ccg ctg gta ggt     240
Gly Ile Arg Leu Leu Gln Glu Glu Glu Glu Gly Leu Pro Leu Val Gly
 65                  70                  75                  80

cgt gtg gct gcc ggt gaa cca ctt ctg gcg caa cag cat att gaa ggt     288
Arg Val Ala Ala Gly Glu Pro Leu Leu Ala Gln Gln His Ile Glu Gly
                 85                  90                  95

cat tat cag gtc gat cct tcc tta ttc aag ccg aat gct gat ttc ctg     336
His Tyr Gln Val Asp Pro Ser Leu Phe Lys Pro Asn Ala Asp Phe Leu
            100                 105                 110

ctg cgc gtc agc ggg atg tcg atg aaa gat atc ggc att atg gat ggt     384
Leu Arg Val Ser Gly Met Ser Met Lys Asp Ile Gly Ile Met Asp Gly
        115                 120                 125

gac ttg ctg gca gtg cat aaa act cag gat gta cgt aac ggt cag gtc     432
Asp Leu Leu Ala Val His Lys Thr Gln Asp Val Arg Asn Gly Gln Val
    130                 135                 140

gtt gtc gca cgt att gat gac gaa gtt acc gtt aag cgc ctg aaa aaa     480
Val Val Ala Arg Ile Asp Asp Glu Val Thr Val Lys Arg Leu Lys Lys
145                 150                 155                 160

cag ggc aat aaa gtc gaa ctg ttg cca gaa aat agc gag ttt aaa cca     528
Gln Gly Asn Lys Val Glu Leu Leu Pro Glu Asn Ser Glu Phe Lys Pro
                165                 170                 175

att gtc gtt gac ctt cgt cag cag agc ttc acc att gaa ggg ctg gcg     576
Ile Val Val Asp Leu Arg Gln Gln Ser Phe Thr Ile Glu Gly Leu Ala
            180                 185                 190

gtt ggg gtt att cgc aac ggc gac tgg ctg taa                         609
Val Gly Val Ile Arg Asn Gly Asp Trp Leu
        195                 200

<210> 47
<211> 10
<212> PRT
<213> Human immunodeficiency virus

<400> 47
Gln Leu Pro Pro Leu Glu Arg Leu Thr Leu
1               5                   10

<210> 48
<211> 30
<212> DNA
<213> Artificial Sequence

<220>
<223> Description of Artificial Sequence: Artificially
      synthesized oligonucleotide sequence

<400> 48
acagctgcca ccgattgaga gacttacgtt                                    30

<210> 49
<211> 30
<212> DNA
<213> Artificial Sequence

<220>
<223> Description of Artificial Sequence: Artificially
      synthesized oligonucleotide sequence

<400> 49
tgtcgacggt ggctaactct ctgaatgcaa                                    30

<210> 50
<211> 1080

<212> DNA
<213> Artificial Sequence

<220>
<223> Description of Artificial Sequence: DNA cording
      for LexA-GAL4 fusion protein

<220>
<221> CDS
<222> (1)..(1077)

<400> 50
atg aaa gcg tta cag ctg cca ccg att gag aga ctt acg tta acg gcc     48
Met Lys Ala Leu Gln Leu Pro Pro Ile Glu Arg Leu Thr Leu Thr Ala
  1               5                  10                  15

agg caa caa gag gtg ttt gat ctc atc cgt gat cac atc agc cag aca     96
Arg Gln Gln Glu Val Phe Asp Leu Ile Arg Asp His Ile Ser Gln Thr
             20                  25                  30

ggt atg ccg ccg acg cgt gcg gaa atc gcg cag cgt ttg ggg ttc cgt    144
Gly Met Pro Pro Thr Arg Ala Glu Ile Ala Gln Arg Leu Gly Phe Arg
         35                  40                  45

tcc cca aac gcg gct gaa gaa cat ctg aag gcg ctg gca cgc aaa ggc    192
Ser Pro Asn Ala Ala Glu Glu His Leu Lys Ala Leu Ala Arg Lys Gly
     50                  55                  60

gtt att gaa att gtt tcc ggc gca tca cgc ggg att cgt ctg ttg cag    240
Val Ile Glu Ile Val Ser Gly Ala Ser Arg Gly Ile Arg Leu Leu Gln
 65                  70                  75                  80

gaa gag gaa gaa ggg ttg ccg ctg gta ggt cgt gtg gct gcc ggt gaa    288
Glu Glu Glu Glu Gly Leu Pro Leu Val Gly Arg Val Ala Ala Gly Glu
                 85                  90                  95

cca ctt ctg gcg caa cag cat att gaa ggt cat tat cag gtc gat cct    336
Pro Leu Leu Ala Gln Gln His Ile Glu Gly His Tyr Gln Val Asp Pro
            100                 105                 110

tcc tta ttc aag ccg aat gct gat ttc ctg ctg cgc gtc agc ggg atg 384
Ser Leu Phe Lys Pro Asn Ala Asp Phe Leu Leu Arg Val Ser Gly Met
        115                 120                 125

tcg atg aaa gat atc ggc att atg gat ggt gac ttg ctg gca gtg cat 432
Ser Met Lys Asp Ile Gly Ile Met Asp Gly Asp Leu Leu Ala Val His
    130                 135                 140

aaa act cag gat gta cgt aac ggt cag gtc gtt gtc gca cgt att gat 480
Lys Thr Gln Asp Val Arg Asn Gly Gln Val Val Val Ala Arg Ile Asp
145                 150                 155                 160

gac gaa gtt acc gtt aag cgc ctg aaa aaa cag ggc aat aaa gtc gaa 528
Asp Glu Val Thr Val Lys Arg Leu Lys Lys Gln Gly Asn Lys Val Glu
                165                 170                 175

ctg ttg cca gaa aat agc gag ttt aaa cca att gtc gtt gac ctt cgt 576
Leu Leu Pro Glu Asn Ser Glu Phe Lys Pro Ile Val Val Asp Leu Arg
            180                 185                 190

cag cag agc ttc acc att gaa ggg ctg gcg gtt ggg gtt att cgc aac 624
Gln Gln Ser Phe Thr Ile Glu Gly Leu Ala Val Gly Val Ile Arg Asn
        195                 200                 205

ggc gac tgg ctg gaa ttc gcc aat ttt aat caa agt ggg aat att gct 672
Gly Asp Trp Leu Glu Phe Ala Asn Phe Asn Gln Ser Gly Asn Ile Ala
    210                 215                 220

gat agc tca ttg tcc ttc act ttc act aac agt agc aac ggt ccg aac 720
Asp Ser Ser Leu Ser Phe Thr Phe Thr Asn Ser Ser Asn Gly Pro Asn
225                 230                 235                 240

ctc ata aca act caa aca aat tct caa gcg ctt tca caa cca att gcc 768
Leu Ile Thr Thr Gln Thr Asn Ser Gln Ala Leu Ser Gln Pro Ile Ala
                245                 250                 255

tcc tct aac gtt cat gat aac ttc atg aat aat gaa atc acg gct agt 816
Ser Ser Asn Val His Asp Asn Phe Met Asn Asn Glu Ile Thr Ala Ser
            260                 265                 270

aaa att gat gat ggt aat aat tca aaa cca ctg tca cct ggt tgg acg     864
Lys Ile Asp Asp Gly Asn Asn Ser Lys Pro Leu Ser Pro Gly Trp Thr
        275                 280                 285

gac caa act gcg tat aac gcg ttt gga atc act aca ggg atg ttt aat     912
Asp Gln Thr Ala Tyr Asn Ala Phe Gly Ile Thr Thr Gly Met Phe Asn
    290                 295                 300

acc act aca atg gat gat gta tat aac tat cta ttc gat gat gaa gat     960
Thr Thr Thr Met Asp Asp Val Tyr Asn Tyr Leu Phe Asp Asp Glu Asp
305                 310                 315                 320

acc cca cca aac cca aaa aaa gag atc tct atg gct tac cca tac gat     1008
Thr Pro Pro Asn Pro Lys Lys Glu Ile Ser Met Ala Tyr Pro Tyr Asp
                325                 330                 335

gtt cca gat tac gct agc ttg ggt ggt cat atg gcc atg gcg gcc gct     1056
Val Pro Asp Tyr Ala Ser Leu Gly Gly His Met Ala Met Ala Ala Ala
            340                 345                 350

cga gtc gac ctg cag cca agc taa                                     1080
Arg Val Asp Leu Gln Pro Ser
        355

<210> 51
<211> 1152
<212> DNA
<213> Artificial Sequence

<220>
<223> Description of Artificial Sequence: DNA
      cording for 'BraC protein derived from
      Pseudomonas aeruginosa BraC   (修正しました)

<220>
<221> CDS
<222> (1)..(1149)

<400> 51

atg gct aag atc tct ccc ggg ctc gag ctc atg aag aag ggt act cag 48
Met Ala Lys Ile Ser Pro Gly Leu Glu Leu Met Lys Lys Gly Thr Gln
1               5                   10                  15

cgt cta tcc cgc ctg ttc gcc gcg atg gcc att gcc ggg ttc gcc agc 96
Arg Leu Ser Arg Leu Phe Ala Ala Met Ala Ile Ala Gly Phe Ala Ser
            20                  25                  30

tac tcc atg gcc gcc gac acc atc aag atc gcc ctg gct ggc ccg gtc 144
Tyr Ser Met Ala Ala Asp Thr Ile Lys Ile Ala Leu Ala Gly Pro Val
        35                  40                  45

acc ggt ccg gta gcc cag tac ggc gac atg cag cgc gcc ggt gcg ctg 192
Thr Gly Pro Val Ala Gln Tyr Gly Asp Met Gln Arg Ala Gly Ala Leu
    50                  55                  60

atg gca atc gaa cag atc aac aag gca ggc ggc gtg aac ggc gcg caa 240
Met Ala Ile Glu Gln Ile Asn Lys Ala Gly Gly Val Asn Gly Ala Gln
65                  70                  75                  80

ctc gaa ggc gtg atc tac gac gac gcc tgc gat ccc aag cag gcc gtg 288
Leu Glu Gly Val Ile Tyr Asp Asp Ala Cys Asp Pro Lys Gln Ala Val
                85                  90                  95

gcg gtc gcc aac aag gtg gtc aac gac ggc gtc aag ttc gtg gtc ggt 336
Ala Val Ala Asn Lys Val Val Asn Asp Gly Val Lys Phe Val Val Gly
            100                 105                 110

cat gtc tgc tcc agc tcc acc caa ccc gcc acc gac atc tac gaa gac 384
His Val Cys Ser Ser Ser Thr Gln Pro Ala Thr Asp Ile Tyr Glu Asp
        115                 120                 125

gaa ggc gtg ctg atg atc acc ccg tcg gcc acc gcc ccg gaa atc acc 432
Glu Gly Val Leu Met Ile Thr Pro Ser Ala Thr Ala Pro Glu Ile Thr
    130                 135                 140

tcg cgc ggc tac aag ctg atc ttc cgc acc atc ggc ctg gac aac atg 480
Ser Arg Gly Tyr Lys Leu Ile Phe Arg Thr Ile Gly Leu Asp Asn Met
145                 150                 155                 160

cag ggc ccg gtg gcc ggc aag ttc atc gcc gaa cgc tac aag gac aag 528
Gln Gly Pro Val Ala Gly Lys Phe Ile Ala Glu Arg Tyr Lys Asp Lys
                165                 170                 175

acc atc gcg gta ctg cac gac aag cag cag tac ggc gaa ggc atc gcc 576
Thr Ile Ala Val Leu His Asp Lys Gln Gln Tyr Gly Glu Gly Ile Ala
            180                 185                 190

acc gag gtg aag aag acc gtg gaa gac gcc ggc atc aag gtt gcc gtc 624
Thr Glu Val Lys Lys Thr Val Glu Asp Ala Gly Ile Lys Val Ala Val
        195                 200                 205

ttc gaa ggc ctg aac gcc ggc gac aag gac ttc aac gcg ctg atc agc 672
Phe Glu Gly Leu Asn Ala Gly Asp Lys Asp Phe Asn Ala Leu Ile Ser
    210                 215                 220

aag ctg aag aaa gcc ggc gtg cag ttc gtc tac ttc ggc ggc tac cac 720
Lys Leu Lys Lys Ala Gly Val Gln Phe Val Tyr Phe Gly Gly Tyr His
225                 230                 235                 240

cca gaa atg ggc ctg ctg ctg cgc cag gcc aag cag gcc ggg ctg gac 768
Pro Glu Met Gly Leu Leu Leu Arg Gln Ala Lys Gln Ala Gly Leu Asp
                245                 250                 255

gcg cgc ttc atg ggc ccg gaa ggg gtc ggc aac agc gaa atc acc gcg 816
Ala Arg Phe Met Gly Pro Glu Gly Val Gly Asn Ser Glu Ile Thr Ala
            260                 265                 270

atc gcc ggc gac gct tcg gaa ggc atg ctg gcg acc ctg ccg cgc gcc 864
Ile Ala Gly Asp Ala Ser Glu Gly Met Leu Ala Thr Leu Pro Arg Ala
        275                 280                 285

ttc gag cag gat ccg aag aac aag gcc ctg atc gac gcc ttc aag gcg 912
Phe Glu Gln Asp Pro Lys Asn Lys Ala Leu Ile Asp Ala Phe Lys Ala
    290                 295                 300

aag aac cag gat ccg agc ggc atc ttc gtc ctg ccc gcc tac tcc gcg 960
Lys Asn Gln Asp Pro Ser Gly Ile Phe Val Leu Pro Ala Tyr Ser Ala
305                 310                 315                 320

gtc aca gtg atc gcc aag ggc atc gag aaa gcc ggc gag gcc gat ccg     1008
Val Thr Val Ile Ala Lys Gly Ile Glu Lys Ala Gly Glu Ala Asp Pro
                325                 330                 335

gag aag gtc gcc gag gcc ctg cgc gcc aac acc ttc gag act ccc acc     1056
Glu Lys Val Ala Glu Ala Leu Arg Ala Asn Thr Phe Glu Thr Pro Thr
            340                 345                 350

ggg aac ctc ggg ttc gac gag aag ggc gac ctg aag aac ttc gac ttc     1104
Gly Asn Leu Gly Phe Asp Glu Lys Gly Asp Leu Lys Asn Phe Asp Phe
        355                 360                 365

acc gtc tac gag tgg cac aag gac gcc acc cgg acc gag gtc aag taa     1152
Thr Val Tyr Glu Trp His Lys Asp Ala Thr Arg Thr Glu Val Lys
    370                 375                 380

<210> 52
<211> 12
<212> PRT
<213> Simian virus 40

<400> 52
Ser Glu Pro Pro Lys Lys Lys Arg Lys Val Glu Thr
  1               5                  10

<210> 53
<211> 37
<212> DNA
<213> Artificial Sequence

<220>
<223> Description of Artificial Sequence: Artificially
      synthesized oligonucleotide sequence

<400> 53
ctagcgagcc tccaaaaaag aagagaaagg tcgaaac                            37

<210> 54
<211> 37
<212> DNA
<213> Artificial Sequence

<220>
<223> Description of Artificial Sequence: Artificially
      synthesized oligonucleotide sequence

<220>
<223> Number of chain: single-strand, topology: linear

<400> 54
gctcggaggt tttttcttct ctttccagct ttggtac                    37


<210> 55
<211> 419
<212> DNA
<213> Artificial Sequence

<220>
<223> Description of Artificial Sequence: Artificially
      synthesized promoter sequence with LexA operator

<220>
<223> genomic DNA

<400> 55
tcgactgctg tatataaaac cagtggttat atgtacagta ctgctgtata taaaaccagt 60

ggttatatgt acagtacgtc gagggaatca aattaacaac cataggatga taatgcgatt 120

agttttttag ccttatttct ggggtaatta atcagcgaag cgatgatttt tgatctatta 180

acagatatat aaatgcaaaa actgcataac cactttaact aatactttca acattttcgg 240

tttgtattac ttcttattca aatgtaataa aagtatcaac aaaaaattgt taatatacct 300

ctatacttta acgtcaagga gaaaaaacta taatgactaa atctcattca gaagaagtga 360

ttgtacctga gttcaattct agcgcaaagg aattaccaag accattggcc gaaaagtgc 419

<210> 56
<211> 12
<212> DNA
<213> Artificial Sequence

<220>
<223> Description of Artificial Sequence: Artificially
      synthesized oligonucleotide sequence

<400> 56
aattgaccac cc                                                          12

<210> 57
<211> 12
<212> DNA
<213> Artificial Sequence

<220>
<223> Description of Artificial Sequence: Artificially
      synthesized oligonucleotide sequence

<400> 57
ctggtgggtt aa                                                          12

<210> 58
<211> 25
<212> DNA
<213> Artificial Sequence

<220>
<223> Description of Artificial Sequence: Artificially
      synthesized oligonucleotide sequence

<400> 58

ctagcttggg tggaattcat atggc                                                25

<210> 59
<211> 24
<212> DNA
<213> Artificial Sequence

<220>
<223> Description of Artificial Sequence: Artificially
      synthesized oligonucleotide sequence

<400> 59
gaacccacct taagtatacg gtac                                                 24

<210> 60
<211> 11
<212> DNA
<213> Artificial Sequence

<220>
<223> Description of Artificial Sequence: Artificially
      synthesized oligonucleotide sequence

<400> 60
ctgcatgcac c                                                               11

<210> 61
<211> 14
<212> DNA
<213> Artificial Sequence

<220>
<223> Description of Artificial Sequence: Artificially
      synthesized oligonucleotide sequence

<400> 61
atggacgtac gtgg                                                            14

<210> 62
<211> 32
<212> DNA
<213> Artificial Sequence

<220>
<223> Description of Artificial Sequence: Artificially
      synthesized primer sequence

<400> 62
ctattcgatg atgaagatac cccaccaaac cc                                32

<210> 63
<211> 30
<212> DNA
<213> Artificial Sequence

<220>
<223> Description of Artificial Sequence: Artificially
      synthesized primer sequence

<400> 63
gaaattcgcc cggaattagc ttggctgcag                                   30

<210> 64
<211> 32
<212> DNA
<213> Artificial Sequence

<220>
<223> Description of Artificial Sequence: Artificially
      synthesized primer sequence

<400> 64
ctattcgatg atgaagatac cccaccaaac cc                                32

<210> 65
<211> 30
<212> DNA
<213> Artificial Sequence

<220>
<223> Description of Artificial Sequence: Artificially
      synthesized primer sequence

<400> 65
gaaattcgcc cggaattagc ttggctgcag                                        30


<210> 66
<211> 539
<212> DNA
<213> Homo sapiens

<400> 66
gcaattttgc cttctgtact ttgtgcaggt tgacctacca tggggtctcc ccatgtaagg 60

tgactgcaga gaaattaatg gacttacgaa atgaatacct gccagcggat gaggctaata 120

aaagactttt ggatcaaagg tatggtaaga gagtgattca gaaggcactg gaagagatgg 180

aaagtaagga gtggctagag aagaactcca agagctgccc atgttgtgga actcccatag 240

agaaattaga cggatgttac aagatgacat gttctggctg tatgcaatat ttctgttgga 300

tttgcatggg ttctctctct agaacaaacc cttacaaaca tttcaatgac cctggttcac 360

catgttttaa ccggctgttt tatgctgtgg atgttgacaa caatatttgg gaagatgaag 420

tagaagacta gttaactact gctcnagata tggaagtgga ttgtttttcc cnaatctccg 480

tccagtncca aagtaacttt gcgggatatt tagggtctat cnttcactct cctgcgtaa  539


<210> 67

<211> 534
<212> DNA
<213> Homo sapiens

<400> 67
gaattcctga tgaggtttta caaagtattt tggatcaata ctccaacaaa tcagaaagcc 60

agaaagagga tcctttcaat attgcagaac cacgagtgga tttacacacc tcaggagaac 120

actcagaatt ggttcaagaa gaaaatttga gcccaggcac ccaaacacct tcaaatgata 180

aagcaagtat gttgcaagaa tactccaaat acctccaaca ggcttttgaa aaatccacta 240

atgcaagttt tactcttgga cacggtttcc aatttgtcag tttgtcttca cctctccaca 300

accacacttt gtttccagaa aaacaaatat acactacgtc tcctttggag tgtggtttcg 360

gccaatctgt tacctcagtg ttgccatctt cattgccaaa gcctcctttt gggatgttgt 420

ttggatctca gccaggtctt tatttgtctg ctttggatgc tacacatcag cagttgacac 480

cttcccagga gctggatgat ctgatagatt ctcagaagaa cttagagact tcat 534


<210> 68
<211> 453
<212> DNA
<213> Homo sapiens

<400> 68
ccttgctggg ggttcgggga gcgcttggac cccggcttct gggacgcgtc aggagaaagg 60

agcactggct ttgctttcat caggccaaag atgcctttct ttgggaatac gttcagtccg 120

aagaagacac ctcctcggaa gtcggcatct ctctccaacc tgcattcttt ggatcgatca 180

acccgggagg tggagctggg cttggaatac ggatccccga ctatgaacct ggcagggcaa 240

agcctgaagt ttgaaaatgg ccagtggata gcagagacag gggttagtgg cggtgtggac 300

cggagggaag ttcagcgcct tcgcaggcgg aaccagcagt tggaggaaga gaacaatctc 360

ttgcggctga aagtggacat cttattagac atgctttcag agtccactgc cgaatcccac 420

ttaatggaga aggaactgga tgaactgagg atc 453

<210> 69
<211> 535
<212> DNA
<213> Homo sapiens

<400> 69
ggatccgctc agaaatgggc cagcttcggc aggagaacga gctgctgcag aacaagttac 60

ataatgctgt gcaaatgaag caaaaagaca agcagaatat cagccagttg gagaaaaagc 120

taaaagctga gcaggaagcc cgaagttttg tagagaaaca gttaatggaa gagaaaaaga 180

ggaagaagtt agaagaagcc actgctgccc gggctgttgc gtttgctgct gcatctaggg 240

gagaatgcac cgaaacctta cggaatcgga tcagagaact agaagcagag ggcaagaagc 300

tcacgatgga catgaaggtg aaagaagacc aaatcagaga actagaacta aaagtccagg 360

agcttcggaa atataaggaa aatgagaagg acactgaggt gttaatgtca gccctctcag 420

ccatgcaaga caaaacacag cacctggaga acagcttaag tgcagagacg agaatcaagc 480

tggacctgtt ctccgcactg ggcgatgcaa agcggcagct cgagattgcc caagg 535

<210> 70
<211> 578
<212> DNA
<213> Homo sapiens

<400> 70
gaattcctca agacaccatt ccagagtatc ctgagctcca gctggaccct aaattggatc 60

ctctttttgc tgagagtccc ctaatgaaca ttgaggttgt tgaggtcctc acactgaacc 120

aggaggtggc tggtccccgg aatgcccaga tccaggccct atatgctgaa gatggaagcc 180

tgagtgcaga tgcccccagt gagcaggtcc aacagcaggg caagcatcca ggtgaccctg 240

aggccgcgcg ccagaagttc cggcagttcc gttataagga catgacaggt ccccgggagg 300

ccctggacca gctccgagag ctgtgtcacc agtggctaca gcctaaggca cgctccaagg 360

agcagatcct ggagctgctg gtgctggagc agttcctaag tgcactgcct gtgaaactcc 420

cgacatgggt ggaatcgcng cacccaaaaa atgccaanaa gtggtggccc tggtaaaagg 480

tgtgacctgg attctgaaga agaagtactt cctgcaggan aactgccnag ggcaccnctg 540

ctgctcaagg tcnctgcccn caggaagaaa aacanaag 578

<210> 71
<211> 564
<212> DNA
<213> Homo sapiens

<400> 71
gtttcaacta ttcccttccc aaaaaagcct gtagaacatg caagaaaaaa ttccattcag 60

cctgcttgta caaatggttt acatctagca acaaatccac ttgtccactg tgtcgtgaga 120

cgtttttctg agattttttc actggaaggg tccctgaagt acatcaaaca aaggcattgg 180

gtttggtccg tctgaaagtg tggtgtgggg aagccagtga gcattacttt aaataggacc 240

tctctggaaa atattttggt aatgtaataa tcctaaaatc aggttactaa cttagattgc 300

ttgtaaatgt ttggaacttc ttttacaaaa gaatattaca tatttgagag aaaatatttc 360

attaatggtt aatttcttgt atatttaaaa ataaatatga aaaaccccta aattacagaa 420

ttnggnattt gtgaaaagnt gcacaactat attattgana aacccattcc gtttgttgat 480

ccgtntttan aatatccaag tgaagcaaaa cgaactgaga gagctnttta catctataga 540

tatttaagcc tattattagt atng 564

<210> 72
<211> 21
<212> DNA
<213> Artificial Sequence

<220>
<223> Description of Artificial Sequence: Artificially
      synthesized primer sequence

<400> 72
gccttctgta ctttgtgcag g 21

<210> 73
<211> 20
<212> DNA
<213> Artificial Sequence

<220>
<223> Description of Artificial Sequence: Artificially
      synthesized primer sequence

<400> 73
tttaaccggc tgttttatgc 20

<210> 74
<211> 30
<212> DNA
<213> Artificial Sequence

<220>
<223> Description of Artificial Sequence: Artificially
      synthesized primer sequence

<400> 74
cactccttac tttccatctc ttccagtgcc                                        30

<210> 75
<211> 22
<212> DNA
<213> Artificial Sequence

<220>
<223> Description of Artificial Sequence: Artificially
      synthesized primer sequence

<400> 75
cagaaagcca gaaagaggat cc                                                22

<210> 76
<211> 20
<212> DNA
<213> Artificial Sequence

<220>
<223> Description of Artificial Sequence: Artificially
      synthesized primer sequence

<400> 76
gtcttcacct ctccacaacc                                                   20

<210> 77
<211> 30
<212> DNA
<213> Artificial Sequence

<220>
<223> Description of Artificial Sequence: Artificially
      synthesized primer sequence

<400> 77
gcctgttgga ggtatttgga gtattcttgc                                        30

<210> 78
<211> 20
<212> DNA
<213> Artificial Sequence

<220>
<223> Description of Artificial Sequence: Artificially
      synthesized primer sequence

<400> 78
caggagaaag gagcactggc                                                   20

<210> 79
<211> 20
<212> DNA
<213> Artificial Sequence

<220>
<223> Description of Artificial Sequence: Artificially
      synthesized primer sequence

<400> 79
ccaaagatgc ctttctttgg                                                   20

<210> 80
<211> 26
<212> DNA
<213> Artificial Sequence

<220>
<223> Description of Artificial Sequence: Artificially
      synthesized primer sequence

<400> 80

ggttcatagt cggggatccg tattcc                                    26


<210> 81
<211> 21
<212> DNA
<213> Artificial Sequence

<220>
<223> Description of Artificial Sequence: Artificially
      synthesized primer sequence

<400> 81
ataatgctgt gcaaatgaag c                                         21


<210> 82
<211> 21
<212> DNA
<213> Artificial Sequence

<220>
<223> Description of Artificial Sequence: Artificially
      synthesized primer sequence

<400> 82
agcagaatat cagccagttg g                                         21


<210> 83
<211> 30
<212> DNA
<213> Artificial Sequence

<220>
<223> Description of Artificial Sequence: Artificially
      synthesized primer sequence

<400> 83
gcttctagtt ctctgatccg attccgtaag                                30

<210> 84
<211> 21
<212> DNA
<213> Artificial Sequence

<220>
<223> Description of Artificial Sequence: Artificially
      synthesized primer sequence

<400> 84
ttgaggtcct cacactgaac c                                                21


<210> 85
<211> 22
<212> DNA
<213> Artificial Sequence

<220>
<223> Description of Artificial Sequence: Artificially
      synthesized primer sequence

<400> 85
tccgttataa ggacatgaca gg                                               22


<210> 86
<211> 30
<212> DNA
<213> Artificial Sequence

<220>
<223> Description of Artificial Sequence: Artificially
      synthesized primer sequence

<400> 86
cactcaggct tccatcttca gcatataggg                                       30

<210> 87
<211> 21
<212> DNA
<213> Artificial Sequence

<220>
<223> Description of Artificial Sequence: Artificially
      synthesized primer sequence

<400> 87
ctattccctt cccaaaaaag c                                                21


<210> 88
<211> 20
<212> DNA
<213> Artificial Sequence

<220>
<223> Description of Artificial Sequence: Artificially
      synthesized primer sequence

<400> 88
cccattccgt ttgttgatcc                                                  20


<210> 89
<211> 26
<212> DNA
<213> Artificial Sequence

<220>
<223> Description of Artificial Sequence: Artificially
      synthesized primer sequence

<400> 89
cgtctcacga cacagtggac aagtgg                                           26

<210> 90
<211> 28
<212> DNA
<213> Artificial Sequence

<220>
<223> Description of Artificial Sequence: Artificially
      synthesized primer sequence

<400> 90
cgctgttttg acctccatag aagacacc                                        28


<210> 91
<211> 215
<212> DNA
<213> Artificial Sequence

<220>
<223> Description of Artificial Sequence: Upstream
      region of cloning site of pCMV SPORT2

<400> 91
cgcctggaga cgccatccac gctgttttga cctccataga agacaccggg accgatccag 60

cctccggact ctagcctagg ccgcggagcg gataacaatt tcacacagga aacagctatg 120

accactaggc ttttgcaaaa agctatttag gtgacactat agaaggtacg cctgcaggta 180

ccggtccgga attcccgggt cgacccacgc gtccg                                215


<210> 92
<211> 3092
<212> DNA
<213> Homo sapiens

<220>
<223> clone name: hfb030

<220>
<221> CDS
<222> (237)..(1658)

<400> 92
gaagaggcgg cgcgccggtt gagcagggcg tttctaggcc tggtcggctg gcggcgatgg 60

caggattttc ataatatatg tagtatgagt tccacatctt ggcctcttac ccagcttcag 120

cagtctcagc tccaccagtt agagaataaa tgggatttgc atgaactcca ctctgagctg 180

aaatacagac tgcggtgtta atatcctttt cttctgattg ttattaacag gtcctt atg 239
Met
1

tcg tca gaa gat cga gaa gct cag gag gat gaa ttg ctg gcc ctg gca 287
Ser Ser Glu Asp Arg Glu Ala Gln Glu Asp Glu Leu Leu Ala Leu Ala
            5                   10                  15

agt att tac gat gga gat gaa ttt aga aaa gca gag tct gtc caa ggt 335
Ser Ile Tyr Asp Gly Asp Glu Phe Arg Lys Ala Glu Ser Val Gln Gly
        20                  25                  30

gga gaa acc agg atc tat ttg gat ttg cca cag aat ttc aag ata ttt 383
Gly Glu Thr Arg Ile Tyr Leu Asp Leu Pro Gln Asn Phe Lys Ile Phe
    35                  40                  45

gtg agc ggc aat tca aat gag tgt ctc cag aat agt ggc ttt gaa tac 431
Val Ser Gly Asn Ser Asn Glu Cys Leu Gln Asn Ser Gly Phe Glu Tyr
50                  55                  60                  65

acc att tgc ttt ctg cct cca ctt gtg ctg aac ttt gaa ctg cca cca 479
Thr Ile Cys Phe Leu Pro Pro Leu Val Leu Asn Phe Glu Leu Pro Pro
                70                  75                  80

gat tat cca tcc tct tcc cca cct tca ttc aca ctt agt ggc aaa tgg 527
Asp Tyr Pro Ser Ser Ser Pro Pro Ser Phe Thr Leu Ser Gly Lys Trp
            85                  90                  95

ctg tca cca act cag cta tct gct cta tgc aag cac tta gac aac cta 575

Leu Ser Pro Thr Gln Leu Ser Ala Leu Cys Lys His Leu Asp Asn Leu
        100                 105                 110

tgg gaa gaa cac cgt ggc agc gtg gtc ctg ttt gcc tgg atg caa ttt 623
Trp Glu Glu His Arg Gly Ser Val Val Leu Phe Ala Trp Met Gln Phe
    115                 120                 125

ctt aag gaa gag acc cta gca tac ttg aat att gtc tct cct ttt gag 671
Leu Lys Glu Glu Thr Leu Ala Tyr Leu Asn Ile Val Ser Pro Phe Glu
130                 135                 140                 145

ctc aag att ggt tct cag aaa aaa gtg cag aga agg aca gct caa gct 719
Leu Lys Ile Gly Ser Gln Lys Lys Val Gln Arg Arg Thr Ala Gln Ala
                150                 155                 160

tct ccc aac aca gag cta gat ttt gga gga gct gct gga tct gat gta 767
Ser Pro Asn Thr Glu Leu Asp Phe Gly Gly Ala Ala Gly Ser Asp Val
            165                 170                 175

gac caa gag gaa att gtg gat gag aga gca gtg cag gat gtg gaa tca 815
Asp Gln Glu Glu Ile Val Asp Glu Arg Ala Val Gln Asp Val Glu Ser
        180                 185                 190

ctg tca aat ctg atc cag gaa atc ttg gac ttt gat caa gct cag cag 863
Leu Ser Asn Leu Ile Gln Glu Ile Leu Asp Phe Asp Gln Ala Gln Gln
    195                 200                 205

ata aaa tgc ttt aat agt aaa ttg ttc ctg tgc agt atc tgt ttc tgt 911
Ile Lys Cys Phe Asn Ser Lys Leu Phe Leu Cys Ser Ile Cys Phe Cys
210                 215                 220                 225

gag aag ctg ggt agt gaa tgc atg tac ttc ttg gag tgc agg cat gtg 959
Glu Lys Leu Gly Ser Glu Cys Met Tyr Phe Leu Glu Cys Arg His Val
                230                 235                 240

tac tgc aaa gcc tgt ctg aag gac tac ttt gaa atc cag atc aga gat 1007
Tyr Cys Lys Ala Cys Leu Lys Asp Tyr Phe Glu Ile Gln Ile Arg Asp
            245                 250                 255

ggc cag gtt caa tgc ctc aac tgc cca gaa cca aag tgc cct tcg gtg 1055

Gly Gln Val Gln Cys Leu Asn Cys Pro Glu Pro Lys Cys Pro Ser Val
        260                 265                 270

gcc act cct ggt cag gtc aaa gag tta gtg gaa gca gag tta ttt gcc     1103
Ala Thr Pro Gly Gln Val Lys Glu Leu Val Glu Ala Glu Leu Phe Ala
    275                 280                 285

cgt tat gac cgc ctt ctc ctc cag tcc tcc ttg gac ctg atg gca gat     1151
Arg Tyr Asp Arg Leu Leu Leu Gln Ser Ser Leu Asp Leu Met Ala Asp
290                 295                 300                 305

gtg gtg tac tgc ccc cgg ccg tgc tgc cag ctg cct gtg atg cag gaa     1199
Val Val Tyr Cys Pro Arg Pro Cys Cys Gln Leu Pro Val Met Gln Glu
                310                 315                 320

cct ggc tgc acc atg ggt atc tgc tcc agc tgc aat ttt gcc ttc tgt     1247
Pro Gly Cys Thr Met Gly Ile Cys Ser Ser Cys Asn Phe Ala Phe Cys
            325                 330                 335

act ttg tgc agg ttg acc tac cat ggg gtc tcc cca tgt aag gtg act     1295
Thr Leu Cys Arg Leu Thr Tyr His Gly Val Ser Pro Cys Lys Val Thr
        340                 345                 350

gca gag aaa tta atg gac tta cga aat gaa tac ctg caa gcg gat gag     1343
Ala Glu Lys Leu Met Asp Leu Arg Asn Glu Tyr Leu Gln Ala Asp Glu
    355                 360                 365

gct aat aaa aga ctt ttg gat caa agg tat ggt aag aga gtg att cag     1391
Ala Asn Lys Arg Leu Leu Asp Gln Arg Tyr Gly Lys Arg Val Ile Gln
370                 375                 380                 385

aag gca ctg gaa gag atg gaa agt aag gag tgg cta gag aag aac tca     1439
Lys Ala Leu Glu Glu Met Glu Ser Lys Glu Trp Leu Glu Lys Asn Ser
                390                 395                 400

aag agc tgc cca tgt tgt gga act ccc ata gag aaa tta gac gga tgt     1487
Lys Ser Cys Pro Cys Cys Gly Thr Pro Ile Glu Lys Leu Asp Gly Cys
            405                 410                 415

aac aag atg aca tgt act ggc tgt atg caa tat ttc tgt tgg att tgc     1535

Asn Lys Met Thr Cys Thr Gly Cys Met Gln Tyr Phe Cys Trp Ile Cys
        420                 425                 430

atg ggt tct ctc tct aga gca aac cct tac aaa cat ttc aat gac cct    1583
Met Gly Ser Leu Ser Arg Ala Asn Pro Tyr Lys His Phe Asn Asp Pro
    435                 440                 445

ggt tca cca tgt ttt aac cgg ctg ttt tat gct gtg gat gtt gac gac    1631
Gly Ser Pro Cys Phe Asn Arg Leu Phe Tyr Ala Val Asp Val Asp Asp
450                 455                 460                 465

gat att tgg gaa gat gag gta gaa gac tagttaacta ctgctcaaga          1678
Asp Ile Trp Glu Asp Glu Val Glu Asp
                470

tatggaagtg gattgttttt ccctaatctt ccgtcaagta cacaaagtaa ctttgcggga 1738

tatttagggt actattcatt cactcttcct gcgtagaaga tatggaagaa cgaggtttat 1798

attttcatgt ggtactactg aagaaggtgc attgatacat ttttaaatgt aagttgagaa 1858

aaatttataa gccaaaggtt cagaaaatta aactacagaa tattaaatat tataatgtgc 1918

ccaaagctct gaatagttaa aaattaaata tttattttct tccccaagct ttaggtaagg 1978

agaagagggg tcaagagtta aacttagaga ccctttgtct ctgagaagca tccttctaag 2038

acattctgtt ggagttccct cagtactatt ccttacaact ggagtgggta gaagccttat 2098

gaaaattata ctgacaacct gatctcgttt actccatgtt aatcacattc ctaccaacct 2158

aaatttctgc caagtcagtt ctctttggag gaagcccatt actccataac tgactggatg 2218

gtccagtgtc attttgatct gcttttcaga atggaaattt ataatataaa tatatgtttt 2278

aataccacaa acactgtggg gcacttgata tttattagtg gtgtatatgg tccactggtt 2338

tcaggccaaa tctagaattt agtgatactg gctcagaaga atttaagttc tattcagctt 2398

tcctgggtgt tagaacctag attcaaaatg gcttgtcttt gctacttttg ttccacattc 2458

tctctcttta cctttgccct accttctgtt tgtaaggatg attttaatat taattccagg 2518

aacacatgtt tttatttcct ctgctggaat aggagaaagc ttaatatatt cccaagctga 2578

atgttcttga attatatctg catttaccac tgtatatgca tatagtgaca gtataacctg 2638

tctatactac agtagttcca gtcctaactt tctgaattat ttttaaagat cttctctaac 2698

aagctatggg aatttggctt catactcttt ctttgcaaca gcagtgttct gggtgataat 2758

tttgaattga tacctgttcc tttttctggg ttttgttggc tttttgaaaa attgtctttc 2818

cttatcattg gtgggaggct tggtagcaaa gtaacatttt ttggaaaaga ggacagaaaa 2878

attgaactac agcttgagaa cgtattcttt ttttcctact ttgttattgc aaattgagga 2938

atcactttta actgttttag gtgtgtgtgt ccagagtgag caaggattat gtttttggat 2998

tgtcaaagag gatgcttagt cttaaaataa aaataaattt aaaaatcatc ttataattaa 3058

aaaaaaaaaa aaaaaaaaaa aaaaaaaaaa aaaa 3092

<210> 93
<211> 3041
<212> DNA
<213> Homo sapiens

<220>
<223> clone name: hfb044-1

<220>
<221> CDS
<222> (48)..(2732)

<400> 93
cgacggcggc tgcggcggcg gcgggacccc caggcctcct ccggggt atg aaa atc 56
Met Lys Ile
1

ggc agt ggg ttc ctg agt ggc ggc gga ggt acc ggc agt agc ggt ggt 104
Gly Ser Gly Phe Leu Ser Gly Gly Gly Gly Thr Gly Ser Ser Gly Gly
    5                   10                  15

agc ggc tcc ggc ggc ggt ggt agt ggc ggc ggc ggc ggc ggc ggc agc 152
Ser Gly Ser Gly Gly Gly Gly Ser Gly Gly Gly Gly Gly Gly Gly Ser
20                  25                  30                  35

agc ggc agg agg gca gag atg gaa ccc acc ttt ccc cag ggt atg gtt 200
Ser Gly Arg Arg Ala Glu Met Glu Pro Thr Phe Pro Gln Gly Met Val
                40                  45                  50

atg ttc aac cac cgt ctt ccc ccg gtc acc agc ttc acc cgg ccg gcg 248
Met Phe Asn His Arg Leu Pro Pro Val Thr Ser Phe Thr Arg Pro Ala
            55                  60                  65

ggg tcg gcc gcc cct ccc ccg caa tgc gtg tta tcc tcc tct acc tcc 296
Gly Ser Ala Ala Pro Pro Pro Gln Cys Val Leu Ser Ser Ser Thr Ser
        70                  75                  80

gca gcc ccg gcc gct gag ccc ccc cct ccg cca gcc ccg gac atg act 344
Ala Ala Pro Ala Ala Glu Pro Pro Pro Pro Pro Ala Pro Asp Met Thr
    85                  90                  95

ttc aag aag gag ccg gcg gcg tca gcc gcg gcc ttc ccc tcg cag agg 392
Phe Lys Lys Glu Pro Ala Ala Ser Ala Ala Ala Phe Pro Ser Gln Arg
100                 105                 110                 115

acc tcc tgg ggg ttc ttg cag tct ttg gtt acc atc aaa cag gag aaa 440
Thr Ser Trp Gly Phe Leu Gln Ser Leu Val Thr Ile Lys Gln Glu Lys
                120                 125                 130

ccc gcg gat cct gag gag cag cag tcc cac cac cac cat cac cac cac 488
Pro Ala Asp Pro Glu Glu Gln Gln Ser His His His His His His His
            135                 140                 145

cac tat ggg ggg ctg ttc gct gga gct gaa gag agg tct cca ggc cta 536
His Tyr Gly Gly Leu Phe Ala Gly Ala Glu Glu Arg Ser Pro Gly Leu
        150                 155                 160

gga ggc ggt gaa ggg ggg agt cac ggc gtc atc cag gac ctc agt att 584
Gly Gly Gly Glu Gly Gly Ser His Gly Val Ile Gln Asp Leu Ser Ile
    165                 170                 175

ctc cac cag cat gtc cag cag caa cca gcc cag cac cac cgt gac gta 632
Leu His Gln His Val Gln Gln Gln Pro Ala Gln His His Arg Asp Val
180                 185                 190                 195

tta ctc agc agc agt agc agg act gat gac cac cat ggc act gag gag 680
Leu Leu Ser Ser Ser Ser Arg Thr Asp Asp His His Gly Thr Glu Glu
                200                 205                 210

cca aag cag gac act aat gtc aaa aag gca aaa agg cca aag cca gaa 728
Pro Lys Gln Asp Thr Asn Val Lys Lys Ala Lys Arg Pro Lys Pro Glu
            215                 220                 225

tct cag gga atc aaa gcc aag agg aag cca agt gca tct tcc aaa cct 776
Ser Gln Gly Ile Lys Ala Lys Arg Lys Pro Ser Ala Ser Ser Lys Pro
        230                 235                 240

tct ttg gtt gga gat gga gaa ggt gcc atc ctc tcc cca agt cag aaa 824
Ser Leu Val Gly Asp Gly Glu Gly Ala Ile Leu Ser Pro Ser Gln Lys
    245                 250                 255

cct cat atc tgt gat cac tgt agt gct gct ttc cga agc tcc tat cac 872
Pro His Ile Cys Asp His Cys Ser Ala Ala Phe Arg Ser Ser Tyr His
260                 265                 270                 275

ctg cgg aga cat gtc ctc att cat aca gga gaa aga cct ttc cag tgc 920
Leu Arg Arg His Val Leu Ile His Thr Gly Glu Arg Pro Phe Gln Cys
                280                 285                 290

agc cag tgt agt atg ggt ttc att cag aaa tac cta cta cag aga cat 968
Ser Gln Cys Ser Met Gly Phe Ile Gln Lys Tyr Leu Leu Gln Arg His
            295                 300                 305

gag aaa att cat agt aga gag aag cca ttt gga tgt gat cag tgc agc 1016
Glu Lys Ile His Ser Arg Glu Lys Pro Phe Gly Cys Asp Gln Cys Ser
        310                 315                 320

atg aag ttt att cag aag tac cat atg gag aga cac aag agg aca cat 1064
Met Lys Phe Ile Gln Lys Tyr His Met Glu Arg His Lys Arg Thr His
    325                 330                 335

agt gga gaa aag cca tat aag tgt gac act tgc caa cag tat ttt tca 1112
Ser Gly Glu Lys Pro Tyr Lys Cys Asp Thr Cys Gln Gln Tyr Phe Ser
340                 345                 350                 355

agg act gat aga ttg ttg aag cac agg cgc aca tgt ggt gaa gtc ata 1160
Arg Thr Asp Arg Leu Leu Lys His Arg Arg Thr Cys Gly Glu Val Ile
                360                 365                 370

gtt aaa gga gcc act agt gca gaa cct ggg tca tca aac cat acc aat 1208
Val Lys Gly Ala Thr Ser Ala Glu Pro Gly Ser Ser Asn His Thr Asn
            375                 380                 385

atg ggt aat ctg gct gtg ttg tct cag gga aat aca agt tct tca agg 1256
Met Gly Asn Leu Ala Val Leu Ser Gln Gly Asn Thr Ser Ser Ser Arg
        390                 395                 400

aga aaa aca aag tca aaa agc ata gct att gaa aat aag gaa cag aag 1304
Arg Lys Thr Lys Ser Lys Ser Ile Ala Ile Glu Asn Lys Glu Gln Lys
    405                 410                 415

acc ggt aaa aca aat gaa tcg caa att tca aat aat ata aac atg cag 1352
Thr Gly Lys Thr Asn Glu Ser Gln Ile Ser Asn Asn Ile Asn Met Gln
420                 425                 430                 435

agt tac tca gta gaa atg cct acc gtg tct tcc agt gga ggc ata att 1400
Ser Tyr Ser Val Glu Met Pro Thr Val Ser Ser Ser Gly Gly Ile Ile
                440                 445                 450

ggc act gga ata gat gaa ctg cag aag agg gtg cca aaa ttg atc ttt 1448
Gly Thr Gly Ile Asp Glu Leu Gln Lys Arg Val Pro Lys Leu Ile Phe
            455                 460                 465

aag aaa gga agc aga aag aat aca gat aaa aac tac ctt aac ttt gtg 1496
Lys Lys Gly Ser Arg Lys Asn Thr Asp Lys Asn Tyr Leu Asn Phe Val
        470                 475                 480

tca cca tta cca gac ata gta gga cag aaa tcc ttg tct gga aaa cca 1544
Ser Pro Leu Pro Asp Ile Val Gly Gln Lys Ser Leu Ser Gly Lys Pro
    485                 490                 495

agt ggc tca ctt ggc ata gta tca aat aat agt gtg gag acc att ggt 1592
Ser Gly Ser Leu Gly Ile Val Ser Asn Asn Ser Val Glu Thr Ile Gly
500                 505                 510                 515

ctt ctc caa agt aca agt ggc aaa caa ggt cag ata agt agt aat tat 1640
Leu Leu Gln Ser Thr Ser Gly Lys Gln Gly Gln Ile Ser Ser Asn Tyr
                520                 525                 530

gat gat gcc atg cag ttt tca aag aaa aga aga tat tta cca act gcc 1688
Asp Asp Ala Met Gln Phe Ser Lys Lys Arg Arg Tyr Leu Pro Thr Ala
            535                 540                 545

agc agc aac agt gcc ttt tct ata aac gta gga cac atg gtc tcc caa 1736
Ser Ser Asn Ser Ala Phe Ser Ile Asn Val Gly His Met Val Ser Gln
        550                 555                 560

cag tct gtc att cag tct gca ggt gtc agt gtt ttg gac aat gag gca 1784
Gln Ser Val Ile Gln Ser Ala Gly Val Ser Val Leu Asp Asn Glu Ala
    565                 570                 575

cca ttg tca ctt att gac tcc tca gct cta aat gct gaa att aaa tct 1832
Pro Leu Ser Leu Ile Asp Ser Ser Ala Leu Asn Ala Glu Ile Lys Ser
580                 585                 590                 595

tgt cat gac aag tct gga att cct gat gag gtt tta caa agt att ttg 1880
Cys His Asp Lys Ser Gly Ile Pro Asp Glu Val Leu Gln Ser Ile Leu
                600                 605                 610

gat caa tac tcc aac aaa tca gaa agc cag aaa gag gat cct ttc aat 1928
Asp Gln Tyr Ser Asn Lys Ser Glu Ser Gln Lys Glu Asp Pro Phe Asn
            615                 620                 625

att gca gaa cca cga gtg gat tta cac acc tca gga gaa cac tca gaa 1976
Ile Ala Glu Pro Arg Val Asp Leu His Thr Ser Gly Glu His Ser Glu
        630                 635                 640

ttg gtt caa gaa gaa aat ttg agc cca ggc acc caa aca cct tca aat 2024
Leu Val Gln Glu Glu Asn Leu Ser Pro Gly Thr Gln Thr Pro Ser Asn
    645                 650                 655

gat aaa gca agt atg ttg caa gaa tac tcc aaa tac ctc caa cag gct 2072
Asp Lys Ala Ser Met Leu Gln Glu Tyr Ser Lys Tyr Leu Gln Gln Ala
660                 665                 670                 675

ttt gaa aaa tcc act aat gca agt ttt act ctt gga cac ggt ttc caa 2120
Phe Glu Lys Ser Thr Asn Ala Ser Phe Thr Leu Gly His Gly Phe Gln
                680                 685                 690

ttt gtc agt ttg tct tca cct ctc cac aac cac act ttg ttt cca gaa 2168
Phe Val Ser Leu Ser Ser Pro Leu His Asn His Thr Leu Phe Pro Glu
            695                 700                 705

aaa caa ata tac act acg tct cct ttg gag tgt ggt ttc ggc caa tct 2216
Lys Gln Ile Tyr Thr Thr Ser Pro Leu Glu Cys Gly Phe Gly Gln Ser
        710                 715                 720

gtt acc tca gtg ttg cca tct tca ttg cca aag cct cct ttt ggg atg 2264
Val Thr Ser Val Leu Pro Ser Ser Leu Pro Lys Pro Pro Phe Gly Met
    725                 730                 735

ttg ttt gga tct cag cca ggt ctt tat ttg tct gct ttg gat gct aca 2312
Leu Phe Gly Ser Gln Pro Gly Leu Tyr Leu Ser Ala Leu Asp Ala Thr
740                 745                 750                 755

cat cag cag ttg aca cct tcc cag gag ctg gat gat ctg ata gat tct 2360
His Gln Gln Leu Thr Pro Ser Gln Glu Leu Asp Asp Leu Ile Asp Ser
                760                 765                 770

cag aag aac tta gag act tca tca gcc ttc cag tcc tca tct cag aaa 2408
Gln Lys Asn Leu Glu Thr Ser Ser Ala Phe Gln Ser Ser Ser Gln Lys
            775                 780                 785

ttg act agc cag aag gaa cag aaa aac tta gag tct tca aca ggc ttt 2456
Leu Thr Ser Gln Lys Glu Gln Lys Asn Leu Glu Ser Ser Thr Gly Phe
        790                 795                 800

cag att cca tct cag gag tta gct agc cag ata gat cct cag aaa gac 2504
Gln Ile Pro Ser Gln Glu Leu Ala Ser Gln Ile Asp Pro Gln Lys Asp
    805                 810                 815

ata gag cct aga aca acg tat cag att gag aac ttt gca caa gcg ttt 2552
Ile Glu Pro Arg Thr Thr Tyr Gln Ile Glu Asn Phe Ala Gln Ala Phe
820                 825                 830                 835

ggt tct cag ttt aag tcg ggc agc agg gtg cca atg acc ttt atc act 2600
Gly Ser Gln Phe Lys Ser Gly Ser Arg Val Pro Met Thr Phe Ile Thr
                840                 845                 850

aac tct aat gga gaa gtg gac cat aga gta agg act tca gtg tca gat 2648
Asn Ser Asn Gly Glu Val Asp His Arg Val Arg Thr Ser Val Ser Asp
            855                 860                 865

ttc tca ggg tat aca aat atg atg tct gat gta agt gag cca tgt agt 2696
Phe Ser Gly Tyr Thr Asn Met Met Ser Asp Val Ser Glu Pro Cys Ser
        870                 875                 880

aca aga gta aag aca ccc acc agc cag agt tac agg taaggtccca 2742
Thr Arg Val Lys Thr Pro Thr Ser Gln Ser Tyr Arg
    885                 890                 895

aaagtggcca ggctggaggt cttctaatgt aattttgttt tattttgaga acactgccat 2802

tggaatgttt ctacacgatc ctattaagaa taatgtgatg ccctttcaat gcaacttttc 2862

atatttagtt tattttgtta gcgtgatttt agctctgttt gtattatgat ttttaatcaa 2922

aatcaataga ttaaaaatag tttgacattc aaagtgacaa tgtttagcaa tcaaatttac 2982

atgtatagat cgtcagggaa tagcccaaat gttttaaacg caaaaaaaaa aacaaaaaa 3041

<210> 94
<211> 3060
<212> DNA
<213> Homo sapiens

<220>
<223> clone name: hfb044-2

<220>
<221> CDS
<222> (69)..(2753)

<400> 94
   cggcgttatt tttattaact ggaggcgacg gcggctgcgg cggcggcggg acccgcctcc 60
   tccggggt atg aaa atc ggc agt ggg ttc ctg agt ggc ggc gga ggt acc 110
   Met Lys Ile Gly Ser Gly Phe Leu Ser Gly Gly Gly Gly Thr
   1 5 10
   ggc agt agc ggt ggt agc ggc tcc ggc ggc ggt ggt agt ggc ggc ggc 158
   Gly Ser Ser Gly Gly Ser Gly Ser Gly Gly Gly Gly Ser Gly Gly Gly
   15 20 25 30
   ggc ggc ggc ggc agc agc ggc agg agg gca gag atg gaa ccc acc ttt 206
   Gly Gly Gly Gly Ser Ser Gly Arg Arg Ala Glu Met Glu Pro Thr Phe
   35 40 45
   ccc cag ggt atg gtt atg ttc aac cac cgt ctt ccc ccg gtc acc agc 254
   Pro Gln Gly Met Val Met Phe Asn His Arg Leu Pro Pro Val Thr Ser
   50 55 60
   ttc acc cgg ccg gcg ggg tcg gcc gcc cct ccc ccg caa tgc gtg tta 302
   Phe Thr Arg Pro Ala Gly Ser Ala Ala Pro Pro Pro Gln Cys Val Leu
   65 70 75
   tcc tcc tct acc tcc gca gcc ccg gcc gct gag ccc ccc cct ccg cca 350
   Ser Ser Ser Thr Ser Ala Ala Pro Ala Ala Glu Pro Pro Pro Pro Pro
   80 85 90
   gcc ccg gac atg act ttc aag aag gag ccg gcg gcg tca gcc gcg gcc 398
   Ala Pro Asp Met Thr Phe Lys Lys Glu Pro Ala Ala Ser Ala Ala Ala
   95 100 105 110
   ttc ccc tcg cag agg acc tcc tgg ggg ttc ttg cag tct ttg ggt agc 446
   Phe Pro Ser Gln Arg Thr Ser Trp Gly Phe Leu Gln Ser Leu Gly Ser
   115 120 125
   atc aaa cag gag aaa ccc gcg gat cct gag gag cag cag tcc cac cac 494
   Ile Lys Gln Glu Lys Pro Ala Asp Pro Glu Glu Gln Gln Ser His His
   130 135 140
   cac cat cac cac cac cac tat ggg ggg ctg ttc gct gga gct gaa gag 542
   His His His His His His Tyr Gly Gly Leu Phe Ala Gly Ala Glu Glu
   145 150 155
   agg tct cca ggc cta gga ggc ggt gaa ggg ggg agt cac ggc gtc atc 590
   Arg Ser Pro Gly Leu Gly Gly Gly Glu Gly Gly Ser His Gly Val Ile
   160 165 170
   cag gac ctc agt att ctc cac cag cat gtc cag cag caa cca gcc cag 638
   Gln Asp Leu Ser Ile Leu His Gln His Val Gln Gln Gln Pro Ala Gln
   175 180 185 190
   cac cac cgt gac gta tta ctc agc agc agt agc agg act gat gac cac 686
   His His Arg Asp Val Leu Leu Ser Ser Ser Ser Arg Thr Asp Asp His
   195 200 205
   cat ggc act gag gag cca aag cag gac act aat gtc aaa aag gca aaa 734
   His Gly Thr Glu Glu Pro Lys Gln Asp Thr Asn Val Lys Lys Ala Lys
   210 215 220
   agg cca aag cca gaa tct cag gga atc aaa gcc aag agg aag cca agt 782
   Arg Pro Lys Pro Glu Ser Gln Gly Ile Lys Ala Lys Arg Lys Pro Ser
   225 230 235
   gca tct tcc aaa cct tct ttg gtt gga gat gga gaa ggt gcc atc ctc 830
   Ala Ser Ser Lys Pro Ser Leu Val Gly Asp Gly Glu Gly Ala Ile Leu
   240 245 250
   tcc cca agt cag aaa cct cat atc tgt gat cac tgt agt gct gct ttc 878
   Ser Pro Ser Gln Lys Pro His Ile Cys Asp His Cys Ser Ala Ala Phe
   255 260 265 270
   cga agc tcc tat cac ctg cgg aga cat gtc ctc att cat aca gga gaa 926
   Arg Ser Ser Tyr His Leu Arg Arg His Val Leu Ile His Thr Gly Glu
   275 280 285
   aga cct ttc cag tgc agc cag tgt agt atg ggt ttc att cag aaa tac 974
   Arg Pro Phe Gln Cys Ser Gln Cys Ser Met Gly Phe Ile Gln Lys Tyr
   290 295 300
   cta cta cag aga cat gag aaa att cat agt aga gag aag cca ttt gga 1022
   Leu Leu Gln Arg His Glu Lys Ile His Ser Arg Glu Lys Pro Phe Gly
   305 310 315
   tgt gat cag tgc agc atg aag ttt att cag aag tac cat atg gag aga 1070
   Cys Asp Gln Cys Ser Met Lys Phe Ile Gln Lys Tyr His Met Glu Arg
   320 325 330
   cac aag agg aca cat agt gga gaa aag cca tat aag tgt gac act tgc 1118
   His Lys Arg Thr His Ser Gly Glu Lys Pro Tyr Lys Cys Asp Thr Cys
   335 340 345 350
   caa cag tat ttt tca agg act gat aga ttg ttg aag cac agg cgc aca 1166
   Gln Gln Tyr Phe Ser Arg Thr Asp Arg Leu Leu Lys His Arg Arg Thr
   355 360 365
   tgt ggt gaa gtc ata gtt aaa gga gcc act agt gca gaa cct ggg tca 1214
   Cys Gly Glu Val Ile Val Lys Gly Ala Thr Ser Ala Glu Pro Gly Ser
   370 375 380
   tca aac cat acc aat atg ggt aat ctg gct gtg ttg tct cag gga aat 1262
   Ser Asn His Thr Asn Met Gly Asn Leu Ala Val Leu Ser Gln Gly Asn
   385 390 395
   aca agt tct tca agg aga aaa aca aag tca aaa agc ata gct att gaa 1310
   Thr Ser Ser Ser Arg Arg Lys Thr Lys Ser Lys Ser Ile Ala Ile Glu
   400 405 410
   aat aag gaa cag aag acc ggt aaa aca aat gaa tcg caa att tca aat 1358
   Asn Lys Glu Gln Lys Thr Gly Lys Thr Asn Glu Ser Gln Ile Ser Asn
   415 420 425 430
   aat ata aac atg cag agt tac tca gta gaa atg cct acc gtg tct tcc 1406
   Asn Ile Asn Met Gln Ser Tyr Ser Val Glu Met Pro Thr Val Ser Ser
                   435                 440                 445
   agt gga ggc ata att ggc act gga ata gat gaa ctg cag aag agg gtg     1454
   Ser Gly Gly Ile Ile Gly Thr Gly Ile Asp Glu Leu Gln Lys Arg Val
               450                 455                 460
   cca aaa ttg atc ttt aag aaa gga agc aga aag aat aca gat aaa aac     1502
   Pro Lys Leu Ile Phe Lys Lys Gly Ser Arg Lys Asn Thr Asp Lys Asn
           465                 470                 475
   tac ctt aac ttt gtg tca cca tta cca gac ata gta gga cag aaa tcc     1550
   Tyr Leu Asn Phe Val Ser Pro Leu Pro Asp Ile Val Gly Gln Lys Ser
       480                 485                 490
   ttg tct gga aaa cca agt ggc tca ctt ggc ata gta tca aat aat agt     1598
   Leu Ser Gly Lys Pro Ser Gly Ser Leu Gly Ile Val Ser Asn Asn Ser
   495                 500                 505                 510
   gtg gag acc att ggt ctt ctc caa agt aca agt ggc aaa caa ggt cag     1646
   Val Glu Thr Ile Gly Leu Leu Gln Ser Thr Ser Gly Lys Gln Gly Gln
                   515                 520                 525
   ata agt agt aat tat gat gat gcc atg cag ttt tca aag aaa aga aga     1694
   Ile Ser Ser Asn Tyr Asp Asp Ala Met Gln Phe Ser Lys Lys Arg Arg
               530                 535                 540
   tat tta cca act gcc agc agc aac agt gcc ttt tct ata aac gta gga     1742
   Tyr Leu Pro Thr Ala Ser Ser Asn Ser Ala Phe Ser Ile Asn Val Gly
           545                 550                 555
   cac atg gtc tcc caa cag tct gtc att cag tct gca ggt gtc agt gtt     1790
   His Met Val Ser Gln Gln Ser Val Ile Gln Ser Ala Gly Val Ser Val
       560                 565                 570
   ttg gac aat gag gca cca ttg tca ctt att gac tcc tca gct cta aat     1838
   Leu Asp Asn Glu Ala Pro Leu Ser Leu Ile Asp Ser Ser Ala Leu Asn
   575                 580                 585                 590
   gct gaa att aaa tct tgt cat gac aag tct gga att cct gat gag gtt     1886
   Ala Glu Ile Lys Ser Cys His Asp Lys Ser Gly Ile Pro Asp Glu Val
                   595                 600                 605
   tta caa agt att ttg gat caa tac tcc aac aaa tca gaa agc cag aaa      1934
   Leu Gln Ser Ile Leu Asp Gln Tyr Ser Asn Lys Ser Glu Ser Gln Lys
               610                 615                 620
   gag gat cct ttc aat att gca gaa cca cga gtg gat tta cac acc tca      1982
   Glu Asp Pro Phe Asn Ile Ala Glu Pro Arg Val Asp Leu His Thr Ser
           625                 630                 635
   gga gaa cac tca gaa ttg gtt caa gaa gaa aat ttg agc cca ggc acc      2030
   Gly Glu His Ser Glu Leu Val Gln Glu Glu Asn Leu Ser Pro Gly Thr
       640                 645                 650
   caa aca cct tca aat gat aaa gca agt atg ttg caa gaa tac tcc aaa      2078
   Gln Thr Pro Ser Asn Asp Lys Ala Ser Met Leu Gln Glu Tyr Ser Lys
   655                 660                 665                 670
   tac ctc caa cag gct ttt gaa aaa tcc act aat gca agt ttt act ctt      2126
   Tyr Leu Gln Gln Ala Phe Glu Lys Ser Thr Asn Ala Ser Phe Thr Leu
                   675                 680                 685
   gga cac ggt ttc caa ttt gtc agt ttg tct tca cct ctc cac aac cac      2174
   Gly His Gly Phe Gln Phe Val Ser Leu Ser Ser Pro Leu His Asn His
               690                 695                 700
   act ttg ttt cca gaa aaa caa ata tac act acg tct cct ttg gag tgt      2222
   Thr Leu Phe Pro Glu Lys Gln Ile Tyr Thr Thr Ser Pro Leu Glu Cys
           705                 710                 715
   ggt ttc ggc caa tct gtt acc tca gtg ttg cca tct tca ttg cca aag      2270
   Gly Phe Gly Gln Ser Val Thr Ser Val Leu Pro Ser Ser Leu Pro Lys
       720                 725                 730
   cct cct ttt ggg atg ttg ttt gga tct cag cca ggt ctt tat ttg tct      2318
   Pro Pro Phe Gly Met Leu Phe Gly Ser Gln Pro Gly Leu Tyr Leu Ser
   735                 740                 745                 750
   gct ttg gat gct aca cat cag cag ttg aca cct tcc cag gag ctg gat      2366
   Ala Leu Asp Ala Thr His Gln Gln Leu Thr Pro Ser Gln Glu Leu Asp
   755 760 765
   gat ctg ata gat tct cag aag aac tta gag act tca tca gcc ttc cag 2414
   Asp Leu Ile Asp Ser Gln Lys Asn Leu Glu Thr Ser Ser Ala Phe Gln
   770 775 780
   tcc tca tct cag aaa ttg act agc cag aag gaa cag aaa aac tta gag 2462
   Ser Ser Ser Gln Lys Leu Thr Ser Gln Lys Glu Gln Lys Asn Leu Glu
   785 790 795
   tct tca aca ggc ttt cag att cca tct cag gag tta gct agc cag ata 2510
   Ser Ser Thr Gly Phe Gln Ile Pro Ser Gln Glu Leu Ala Ser Gln Ile
   800 805 810
   gat cct cag aaa gac ata gag cct aga aca acg tat cag att gag aac 2558
   Asp Pro Gln Lys Asp Ile Glu Pro Arg Thr Thr Tyr Gln Ile Glu Asn
   815 820 825 830
   ttt gca caa gcg ttt ggt tct cag ttt aag tcg ggc agc agg gtg cca 2606
   Phe Ala Gln Ala Phe Gly Ser Gln Phe Lys Ser Gly Ser Arg Val Pro
   835 840 845
   atg acc ttt atc act aac tct aat gga gaa gtg gac cat aga gta agg 2654
   Met Thr Phe Ile Thr Asn Ser Asn Gly Glu Val Asp His Arg Val Arg
   850 855 860
   act tca gtg tca gat ttc tca ggg tat aca aat atg atg tct gat gta 2702
   Thr Ser Val Ser Asp Phe Ser Gly Tyr Thr Asn Met Met Ser Asp Val
   865 870 875
   agt gag cca tgt agt aca aga gta aag aca ccc acc agc cag agt tac 2750
   Ser Glu Pro Cys Ser Thr Arg Val Lys Thr Pro Thr Ser Gln Ser Tyr
   880 885 890
   agg taaggtccca aaagtggcca ggctggaggt cttctaatgt aattttgttt 2803
   Arg
   895
   tattttgaga acactgccat tggaatgttt ctacacgatc ctattaagaa taatgtgatg 2863
   ccctttcaat gcaacttttc atatttagtt tattttgtta gcgtgatttt agctctgttt 2923
   gtattatgat ttttaatcaa aatcaataga ttaaaaatag tttgacattc aaagtgacaa 2983
   tgtttagcaa tcaaatttac atgtatagat cgtcagggaa tagcccaaat gttttaaacg 3043
   caaaaaaaaa aacaaaa 3060

<210> 95
<211> 984
<212> DNA
<213> Homo sapiens

<220>
<223> clone name: hfb060-1

<400> 95
attggcgcag gctggcgctg ggaacccccg cgccggttac ccctttcctc ggtggtagag 60

gccgggcggg gcagcgcatg cgcgtggcgg gcaggcggca ggaggcgggt gggtcaaggt 120

aactctgggc tacagagtcc ttgctggggg ttcggggagc gcttggaccc cggcttctgg 180

gacgcgtcag gagaagggag cactggcttt gctttcatca ggccaaagat gcctttcttt 240

gggaatacgt tcagtccgaa gaagacacct cctcggaagt cggcatctct ctccaacctg 300

cattctttgg atcgatcaac ccgggaggtg gagctgggct tggaatacgg atccccgact 360

atgaacctgg cagggcaaag cctgaagttt gaaaatggcc agtggatagc agagacaggg 420

gttagtggcg gtgtggaccg gagggaggtt cagcgccttc gcaggcggaa ccagcagttg 480

gaggaagaga acaatctctt gcggctgaaa gtggacatct tattagacat gctttcagag 540

tccactgccg aatcccactt aatggagaag gaactggatg aactgaggat cagccggaag 600

agaaaatgaa gaccccagag acatttattg gggagtagga tgtggctgag tgcttttttt 660

ttggccagac tagcggattc agtcctggaa gagagtatca tataatgaga cccacaggca 720

ctggcaccct tgggttggca atagaaggtg acatggaatg gagaaaacca agattccaga 780

tggggatagt aactagaagg tgcttcagat gcactgcctg cgggtgccag tctgaaaacc 840

agaccgcaca gaggcctggg gctgctgatg agcttttcgg tgctctccac acacaagctc 900

gcaaacacac atgtcccaga atagctctgt tgggttgtgt tgggagaagc ggctggagtt 960

cattctctca accccttatg ttgg 984

<210> 96
<211> 982
<212> DNA
<213> Homo sapiens

<220>
<223> clone name: hfb060-2

<400> 96
gcggcaggag gcgggtgggt caaggtaact ctgggctaca gagtccttgc tgggggttcg 60

gggagcgctt ggaccccggc ttctgggacg cgtcagagac gggccctgct aagttgccca 120

ggctggtctt gaactcctgt gctcaagtaa tcctccctcc ctggcctccc aaagtgctgg 180

gattacagga gaagggagca ctggctttgc tttcatcagg ccaaagatgc ctttctttgg 240

gaatacgttc agtccgaaga agacacctcc tcggaagtcg gcatctctct ccaacctgca 300

ttctttggat cgatcaaccc gggaggtgga gctgggcttg gaatacggat ccccgactat 360

gaacctggca gggcaaagcc tgaagtttga aaatggccag tggatagcag agacaggggt 420

tagtggcggt gtggaccgga gggaggttca gcgccttcgc aggcggaacc agcagttgga 480

ggaagagaac aatctcttgc ggctgaaagt ggacatctta ttagacatgc tttcagagtc 540

cactgccgaa tcccacttaa tggagaagga actggatgaa ctgaggatca gccggaagag 600

aaaatgaaga ccccagagac atttattggg gagtaggatg tggctgagtg cttttttttt 660

ggccagacta gcggattcag tcctggaaga gagtatcata taatgagacc cacaggcact 720

ggcacccttg ggttggcaat agaaggtgac atggaatgga gaaaaccaag attccagatg 780

gggatagtaa ctagaaggtg cttcagatgc actgcctgcg ggtgccagtc tgaaaaccag 840

accgcacaga ggcctggggc tgctgatgag cttttcggtg ctctccacac acaagctcgc 900

aaacacacat gtcccagaat agctctgttg ggttgtgttg ggagaagcgg ctggagttca 960

ttctctcaac cccttatgtt gg 982

<210> 97
<211> 1101
<212> DNA
<213> Homo sapiens

<220>
<223> clone name: hfb066

<220>
<221> CDS
<222> (1)..(1101)

<400> 97
aaa aat tac aaa aat gcc agt gga gtt gtg aac tct tca cct cga agt 48
Lys Asn Tyr Lys Asn Ala Ser Gly Val Val Asn Ser Ser Pro Arg Ser
1               5                   10                  15

cat agc gcc aca aat ggg agc att cct tcc tca tct agt aaa aat gag 96
His Ser Ala Thr Asn Gly Ser Ile Pro Ser Ser Ser Ser Lys Asn Glu
            20                  25                  30

aag aag cag aaa tgc act agc aag agc cca agt aca cac aag gac tta 144

Lys Lys Gln Lys Cys Thr Ser Lys Ser Pro Ser Thr His Lys Asp Leu
        35                  40                  45

atg gaa aac tgt att cct aat aac cag cta agc aaa cca gac gca ctg 192
Met Glu Asn Cys Ile Pro Asn Asn Gln Leu Ser Lys Pro Asp Ala Leu
    50                  55                  60

gtc agg ctg gaa caa gac att aaa aag tta aag gct gac ctg caa gcc 240
Val Arg Leu Glu Gln Asp Ile Lys Lys Leu Lys Ala Asp Leu Gln Ala
65                  70                  75                  80

agc aga caa gtg gaa caa gag ctc cgc agt cag atc agc tcc ctt tcg 288
Ser Arg Gln Val Glu Gln Glu Leu Arg Ser Gln Ile Ser Ser Leu Ser
                85                  90                  95

agc acc gag cga ggg atc cgc tca gaa atg ggc cag ctt cgg cag gag 336
Ser Thr Glu Arg Gly Ile Arg Ser Glu Met Gly Gln Leu Arg Gln Glu
            100                 105                 110

aac gag ctg ctg cag aac aag tta cat aat gct gtg caa atg aag caa 384
Asn Glu Leu Leu Gln Asn Lys Leu His Asn Ala Val Gln Met Lys Gln
        115                 120                 125

aaa gac aag cag aat atc agc cag ttg gag aaa aag cta aaa gct gag 432
Lys Asp Lys Gln Asn Ile Ser Gln Leu Glu Lys Lys Leu Lys Ala Glu
    130                 135                 140

cag gaa gcc cga agt ttt gta gag aaa cag tta atg gaa gag aaa aag 480
Gln Glu Ala Arg Ser Phe Val Glu Lys Gln Leu Met Glu Glu Lys Lys
145                 150                 155                 160

agg aag aag tta gaa gaa gcc act gct gcc cgg gct gtt gcg ttt gct 528
Arg Lys Lys Leu Glu Glu Ala Thr Ala Ala Arg Ala Val Ala Phe Ala
                165                 170                 175

gct gca tct agg gga gaa tgc acc gaa acc tta cgg aat cgg atc aga 576
Ala Ala Ser Arg Gly Glu Cys Thr Glu Thr Leu Arg Asn Arg Ile Arg
            180                 185                 190

gaa cta gaa gca gag ggc aag aag ctc acg atg gac atg aag gtg aaa 624

Glu Leu Glu Ala Glu Gly Lys Lys Leu Thr Met Asp Met Lys Val Lys
        195                 200                 205

gaa gac caa atc aga gaa cta gaa cta aaa gtc cag gag ctt cgg aaa     672
Glu Asp Gln Ile Arg Glu Leu Glu Leu Lys Val Gln Glu Leu Arg Lys
    210                 215                 220

tat aag gaa aat gag aag gac act gag gtg tta atg tca gcc ctc tca     720
Tyr Lys Glu Asn Glu Lys Asp Thr Glu Val Leu Met Ser Ala Leu Ser
225                 230                 235                 240

gcc atg caa gac aaa aca cag cac ctg gag aac agc tta agt gca gag     768
Ala Met Gln Asp Lys Thr Gln His Leu Glu Asn Ser Leu Ser Ala Glu
                245                 250                 255

acg aga atc aag ctg gac ctg ttc tcc gca ctg ggc gat gca aag cgg     816
Thr Arg Ile Lys Leu Asp Leu Phe Ser Ala Leu Gly Asp Ala Lys Arg
            260                 265                 270

cag ctc gag att gcc caa gga caa atc ctt cag aaa gat cag gga aat     864
Gln Leu Glu Ile Ala Gln Gly Gln Ile Leu Gln Lys Asp Gln Gly Asn
        275                 280                 285

caa gga cct aaa aca gaa gat agc cga agt cat ggc cgt cat gcc cag     912
Gln Gly Pro Lys Thr Glu Asp Ser Arg Ser His Gly Arg His Ala Gln
    290                 295                 300

cat aac ata cag tgc cgc cac cag ccc cct gag ccc tgt ttc ccc cca     960
His Asn Ile Gln Cys Arg His Gln Pro Pro Glu Pro Cys Phe Pro Pro
305                 310                 315                 320

cta ctc ttc caa att tgt gga gac cag ccc ctc tgg act ttg acc cca     1008
Leu Leu Phe Gln Ile Cys Gly Asp Gln Pro Leu Trp Thr Leu Thr Pro
                325                 330                 335

atg gcc tct gtt tac cag ccc ctg aag aaa ttg aag gcc agc ctg tgt     1056
Met Ala Ser Val Tyr Gln Pro Leu Lys Lys Leu Lys Ala Ser Leu Cys
            340                 345                 350

gtt gtg ccc aaa aaa ttt ggg tta ccg gga aag gca ttg caa agg         1101

Val Val Pro Lys Lys Phe Gly Leu Pro Gly Lys Ala Leu Gln Arg
        355                 360                 365

<210> 98
<211> 2565
<212> DNA
<213> Homo sapiens

<220>
<223> clone name: hfb101-1

<220>
<221> CDS
<222> (474)..(2117)

<400> 98
cgcccccacc gccacccaac caagcatcac ccattccagc acttccggcc tgacccctgt 60
ccagcgcctt tcctttctcc agcttctgct ctgccccaag agtggtcgcc ttcgtggcag 120
ggcacgactc tctcccagct cgggtcgccg cccacattag tgtggggccc tgcggcctag 180
cgtccctcac cagaggcctc cccttgccta gctggaccgc cgagggacat cgacgagtat 240
cctcctcctg ctgtccccgg cttcgcctgc cgcccctaac cggccagtca agatggccgc 300
cgctgggtga ggcaagctgg cgcgccgcgg gggcgtctgg gagttgtagt tcgggacggc 360
gggctgacgc acttcgccgt cggccgacgg gcgccattgt gcggcgcgcg ccggagtatc 420
ctgagcccca gctggaccct aaattggatc ctcttcctgc tgagagtccc cta atg 476
aac att gag gtt gtt gag gtc ctc aca ctg aac cag gag gtg gct ggt 524
ccc cgg aat gcc cag atc cag gcc cta tat gct gaa gat gga agc ctg 572
agt gca gat gcc ccc agt gag cag atc caa cag cag ggc aag cat cca 620
ggt gac cct gag gcc gcg cgc cag agg ttc cgg cag ttc cgt tat aag 668
gac atg aca ggt ccc cgg gag gcc ctg gac cag ctc cga gag ctg tgt 716
cac cag tgg cta cag cct aag gca cgc tcc aag gag cag atc ctg gag 764
ctg ctg gtg ctg gag cag ttc cta ggt gca ctg cct gtg aag ctc cgg 812
aca tgg gtg gaa tcg cag cac cca gag aac tgc caa gag gtg gtg gcc 860
ctg gta gag ggt gtg acc tgg atg tct gag gag gaa gta ctt cct gca 908
gga caa cct gcc gag ggc acc acc tgc tgc ctc gag gtc act gcc cag 956
cag gag gag aag cag gag gat gca gcc atc tgc cca gtg aca gtg ctc 1004
cct gag gag cca gtg acc ttc cag gat gtg gct gtg gac ttc agc cgg 1052
gag gag tgg ggg ctg ctg ggc ccg aca cag agg acc gag tac cgc gat     1100
gtg atg ctg gag acc ttt ggg cac ctg gtc tct gtg ggg tgg gag act     1148
aca ctg gaa aat aaa gag tta gct cca aat tct gac att cct gag gaa     1196
gaa cca gcc ccc agc ctg aaa gta caa gaa tcc tca agg gat tgt gcc     1244
ttg tcc tct aca tta gaa gat acc ttg cag ggt ggg gtc cag gaa gtc     1292
caa gac aca gtg ttg aag cag atg gag tct gct cag gaa aaa gac ctt     1340
cct cag aag aag cac ttt gac aac cgt gag tcc cag gca aac agt ggt     1388
gct ctt gac aca aac caa gtt tcg ctc cag aaa att gac aac cct gag     1436
tcc cag gca aac agt ggc gct ctt gac aca aac caa gtt ttg ctc cac     1484
aaa att cct cct aga aaa cga ttg cgc aaa cgt gac tca caa gtt aaa     1532
agt atg aaa cat aat tca cgt gta aaa att cat cag aag agc tgt gaa 1580
agg caa aag gcc aag gaa ggc aat ggt tgt agg aaa acc ttc agt cgg 1628
agt act aaa cag att acg ttt ata aga att cac aag ggg agc caa gtt 1676
tgc cga tgc agt gaa tgt ggt aaa ata ttc cgg aac cca aga tac ttt 1724
tct gtg cat aag aaa atc cat acc gga gag agg ccc tat gtg tgt caa 1772
gac tgt ggg aaa gga ttt gtt cag agc tct tcc ctc aca cag cat cag 1820
aga gtt cat tct gga gag aga cca ttt gaa tgt cag gag tgt ggg agg 1868
acc ttc aat gat cgc tca gcc atc tcc cag cac ctg agg act cac act 1916
ggc gct aag ccc tac aag tgt cag gac tgt gga aaa gcc ttc cgc cag 1964
agc tcc cac ctc atc aga cat cag agg act cac acc ggg gag cgc cca 2012
tat gca tgc aac aaa tgt gga aag gcc ttc acc cag agc tca cac ctt    2060
att tgg cac cag aga acc cac aat agg aca aag cga aag aag aaa cag    2108
cct acc tca tagctctcaa gccagttgaa gaaaccttgc cttttcagct            2157
tgaccctgca atataacatg cacaggcctg cttgtgaatc aggactgaat gtgaaaggga 2217
agtattgagt gaggacattc ccaaaaccaa aggacaactg aggagactgc ccagcacata 2277
atgaataaat aagaaaatga gtgaggagtt attaacatca tttggaaaaa agatttccca 2337
ttcacttgat attgtttgtt cactcattta gtcattaaaa gtgagattaa taaaatctga 2397
aaatgttata taataacttt aaaaagccag gtaattaata atctgcactg atattacatc 2457
cacagtacca cagtatttat gtgtatgaat taaggattaa aagataatgt ggataaataa 2517
actattgatc tatgtctgtg taaaaaaaaa aaaaaaaaaa aaaaaaaa              2565

<210> 99
<211> 2694
<212> DNA
<213> Homo sapiens

<220>
<223> clone name: hfb101-2

<220>
<221> CDS
<222> (402)..(2264)

<400> 99
gtccagcgcc tttcctttct ccagcttctg ctctgcccca agagtggtcg ccttcgtggc 60

agggcacgac tctctcccag ctcgggtcgc cgcccacatt agtgtggggc cctgcggcct 120

agcgtccctc accagaggcc tccccttgcc tagctggacc gccgagggac atcgacgagt 180

atcctcctcc tgctgtcccc ggcttcgcct gccgccccta accggccagt caagatggcc 240

gccgctgggt gaggcaagct ggcgcgccgc gggggcgtct gggagttgta gttcgggacg 300

gcgggctgac gcacttcgcc gccggccgac gggcgccatt gtgcggcgcg cgccgggact 360

ctgcccactt ccaccagaga cacattgaga aggaggaaac t atg gcc tcc agg ctt 416
Met Ala Ser Arg Leu
1               5

ccg acg gcc tgg tcc tgt gaa cca gtg acc ttt gaa gat gta aca ctg 464
Pro Thr Ala Trp Ser Cys Glu Pro Val Thr Phe Glu Asp Val Thr Leu
                10                  15                  20

ggt ttt acc ccg gaa gag tgg gga ctg ctg gac ctc aaa cag aag tcc 512
Gly Phe Thr Pro Glu Glu Trp Gly Leu Leu Asp Leu Lys Gln Lys Ser
            25                  30                  35

ctg tac agg gaa gtg atg ctg gag aac tac agg aac ctg gtc tca gtg 560
Leu Tyr Arg Glu Val Met Leu Glu Asn Tyr Arg Asn Leu Val Ser Val
        40                  45                  50

gag tat cct gag ctc cag ctg gac cct aaa ttg gat cct ctt cct gct 608
Glu Tyr Pro Glu Leu Gln Leu Asp Pro Lys Leu Asp Pro Leu Pro Ala
    55                  60                  65

gag agt ccc cta atg aac att gag gtt gtt gag gtc ctc aca ctg aac 656
Glu Ser Pro Leu Met Asn Ile Glu Val Val Glu Val Leu Thr Leu Asn
70                  75                  80                  85

cag gag gtg gct ggt ccc cgg aat gcc cag atc cag gcc cta tat gct 704
Gln Glu Val Ala Gly Pro Arg Asn Ala Gln Ile Gln Ala Leu Tyr Ala

                90                  95                  100

gaa gat gga agc ctg agt gca gat gcc ccc agt gag cag gtc caa cag 752
Glu Asp Gly Ser Leu Ser Ala Asp Ala Pro Ser Glu Gln Val Gln Gln
            105                 110                 115

cag ggc aag cat cca ggt gac cct gag gcc gcg cgc cag agg ttc cgg 800
Gln Gly Lys His Pro Gly Asp Pro Glu Ala Ala Arg Gln Arg Phe Arg
        120                 125                 130

cag ttc cgt tat aag gac atg aca ggt ccc cgg gag gcc ctg gac cag 848
Gln Phe Arg Tyr Lys Asp Met Thr Gly Pro Arg Glu Ala Leu Asp Gln
    135                 140                 145

ctc cga gag ctg tgt cac cag tgg cta cag cct aag gca cgc tcc aag 896
Leu Arg Glu Leu Cys His Gln Trp Leu Gln Pro Lys Ala Arg Ser Lys
150                 155                 160                 165

gag cag atc ctg gag ctg ctg gtg ctg gag cag ttc cta ggt gca ctg 944
Glu Gln Ile Leu Glu Leu Leu Val Leu Glu Gln Phe Leu Gly Ala Leu
                170                 175                 180

cct gtg aag ctc cgg aca tgg gtg gaa tcg cag cac cca gag aac tgc 992
Pro Val Lys Leu Arg Thr Trp Val Glu Ser Gln His Pro Glu Asn Cys
            185                 190                 195

caa gag gtg gtg gcc ctg gta gag ggt gtg acc tgg atg tct gag gag 1040
Gln Glu Val Val Ala Leu Val Glu Gly Val Thr Trp Met Ser Glu Glu
        200                 205                 210

gaa gta ctt cct gca gga caa cct gcc gag ggc acc acc tgc tgc ctc 1088
Glu Val Leu Pro Ala Gly Gln Pro Ala Glu Gly Thr Thr Cys Cys Leu
    215                 220                 225

gag gtc act gcc cag cag gag gag aag cag gag gat gca gcc atc tgc 1136
Glu Val Thr Ala Gln Gln Glu Glu Lys Gln Glu Asp Ala Ala Ile Cys
230                 235                 240                 245

cca gtg aca gtg ctc cct gag gag cca gtg acc ttc cag gat gtg gct 1184
Pro Val Thr Val Leu Pro Glu Glu Pro Val Thr Phe Gln Asp Val Ala

                250                 255                 260

gtg gac ttc agc cgg gag gag tgg ggg ctg ctg ggc ccg aca cag agg 1232
Val Asp Phe Ser Arg Glu Glu Trp Gly Leu Leu Gly Pro Thr Gln Arg
            265                 270                 275

acc gag tac cgc gat gtg atg ctg gag acc ttt ggg cac ctg gtc tct 1280
Thr Glu Tyr Arg Asp Val Met Leu Glu Thr Phe Gly His Leu Val Ser
        280                 285                 290

gtg ggg tgg gag act aca ctg gaa aat aaa gag tta gct cca aat tct 1328
Val Gly Trp Glu Thr Thr Leu Glu Asn Lys Glu Leu Ala Pro Asn Ser
    295                 300                 305

gac att cct gag gaa gaa cca gcc ccc agc ctg aaa gta caa gaa tcc 1376
Asp Ile Pro Glu Glu Glu Pro Ala Pro Ser Leu Lys Val Gln Glu Ser
310                 315                 320                 325

tca agg gat tgt gcc ttg tcc tct aca tta gaa gat acc ttg cag ggt 1424
Ser Arg Asp Cys Ala Leu Ser Ser Thr Leu Glu Asp Thr Leu Gln Gly
                330                 335                 340

ggg gtc cag gaa gtc caa gac aca gtg ttg aag cag atg gag tct gct 1472
Gly Val Gln Glu Val Gln Asp Thr Val Leu Lys Gln Met Glu Ser Ala
            345                 350                 355

cag gaa aaa gac ctt cct cag aag aag cac ttt gac aac cgt gag tcc 1520
Gln Glu Lys Asp Leu Pro Gln Lys Lys His Phe Asp Asn Arg Glu Ser
        360                 365                 370

cag gca aac agt ggt gct ctt gac aca aac caa gtt tcg ctc cag aaa 1568
Gln Ala Asn Ser Gly Ala Leu Asp Thr Asn Gln Val Ser Leu Gln Lys
    375                 380                 385

att gac aac cct gag tcc cag gca aac agt ggc gct ctt gac aca aac 1616
Ile Asp Asn Pro Glu Ser Gln Ala Asn Ser Gly Ala Leu Asp Thr Asn
390                 395                 400                 405

caa gtt ttg ctc cac aaa att cct cct aga aaa cga ttg cgc aaa cgt 1664
Gln Val Leu Leu His Lys Ile Pro Pro Arg Lys Arg Leu Arg Lys Arg

                410                 415                 420

gac tca caa gtt aaa agt atg aaa cat aat tca cgt gta aaa att cat 1712
Asp Ser Gln Val Lys Ser Met Lys His Asn Ser Arg Val Lys Ile His
            425                 430                 435

cag aag agc tgt gaa agg caa aag gcc aag gaa ggc aat ggt tgt agg 1760
Gln Lys Ser Cys Glu Arg Gln Lys Ala Lys Glu Gly Asn Gly Cys Arg
        440                 445                 450

aaa acc ttc agt cgg agt act aaa cag att acg ttt ata aga att cac 1808
Lys Thr Phe Ser Arg Ser Thr Lys Gln Ile Thr Phe Ile Arg Ile His
    455                 460                 465

aag ggg agc caa gtt tgc cga tgc agt gaa tgt ggt aaa ata ttc cgg 1856
Lys Gly Ser Gln Val Cys Arg Cys Ser Glu Cys Gly Lys Ile Phe Arg
470                 475                 480                 485

aac cca aga tac ttt tct gtg cat aag aaa atc cat acc gga gag agg 1904
Asn Pro Arg Tyr Phe Ser Val His Lys Lys Ile His Thr Gly Glu Arg
                490                 495                 500

ccc tat gtg tgt caa gac tgt ggg aaa gga ttt gtt cag agc tct tcc 1952
Pro Tyr Val Cys Gln Asp Cys Gly Lys Gly Phe Val Gln Ser Ser Ser
            505                 510                 515

ctc aca cag cat cag aga gtt cat tct gga gag aga cca ttt gaa tgt 2000
Leu Thr Gln His Gln Arg Val His Ser Gly Glu Arg Pro Phe Glu Cys
        520                 525                 530

cag gag tgt ggg agg acc ttc aat gat cgc tca gcc atc tcc cag cac 2048
Gln Glu Cys Gly Arg Thr Phe Asn Asp Arg Ser Ala Ile Ser Gln His
    535                 540                 545

ctg agg act cac act ggc gct aag ccc tac aag tgt cag gac tgt gga 2096
Leu Arg Thr His Thr Gly Ala Lys Pro Tyr Lys Cys Gln Asp Cys Gly
550                 555                 560                 565

aaa gcc ttc cgc cag agc tcc cac ctc atc aga cat cag agg act cac 2144
Lys Ala Phe Arg Gln Ser Ser His Leu Ile Arg His Gln Arg Thr His

                570                 575                 580

acc ggg gag cgc cca tat gca tgc aac aaa tgt gga aag gcc ttc acc 2192
Thr Gly Glu Arg Pro Tyr Ala Cys Asn Lys Cys Gly Lys Ala Phe Thr
            585                 590                 595

cag agc tca cac ctt att ggg cac cag aga acc cac aat agg aca aag 2240
Gln Ser Ser His Leu Ile Gly His Gln Arg Thr His Asn Arg Thr Lys
        600                 605                 610

cga aag aag aaa cag cct acc tca tagctctcaa gccagttgaa gaaaccttgc 2294
Arg Lys Lys Lys Gln Pro Thr Ser
    615                 620

cttttcagct tgaccctgca atataacatg cacaggcctg cttgtgaatc aggactgaat 2354

gtgaaaggga agtattgagt gaggacattc ccaaaaccaa aggacaactg aggagactgc 2414

ccagcacata atgaataaat aagaaaatga gtgaggagtt attaacatca tttggaaaaa 2474

agatttccca ttcacttgat attgtttgtt cactcattta gtcattaaaa gtgagattaa 2534

taaaatctga aaatgttata taataacttt aaaaagccag gtaattaata atctgcactg 2594

atattacatc cacagtacca cagtatttat gtgtatgaat taaggattaa aagataatgt 2654

ggataaataa actattgatc tatgaaaaaa aaaaaaaaaa 2694

<210> 100
<211> 6193
<212> DNA
<213> Homo sapiens

<220>
<223> clone name: hfb341

<220>
<221> CDS
<222> (466)..(3870)

<400> 100
gaaaaaacag ctcacaactt ggagaacgta ctgatacatt tctgggaaag actgtcagag 60
atctgtgttg cgaaaatcag tgagccagaa gctgatgttg agtccgtttt gggtgtatct 120
aacctattac aggtgcttca gaagccgaag agctcattga agtcaagtaa aaaaaaaaat 180
ggtaaggtta gatttgctga tgagatactt gaaagcaata aagagaatga aaaatgtgta 240
tcttcagaag gagagaagat tgaaggctgg gaattaacaa ctgaaccttc tctcactcat 300
aattcttcag gccttttgtc tcctctaagg aaaaaacctt tggaagactt agtctgtaaa 360
ctcgcagata taagtattaa ttatgtcaat gaacgaaagt cagagcaaca tctaaggttt 420
ctttctactc tgcttgactc cttttcttca agccgagtat ttaaa atg cta ctt ggt 477
gat gaa aaa cag agt att gtc caa gcc aaa cct ctt gaa ata gcc aag 525
ctt gta caa aaa aat cct gcg gtg cag ttt tta tac cag aaa ctg ata 573
ggt tgg cta aat gaa gat caa agg aag gat ttt ggt ttc ctg gtg gac 621
att ttg tac agt gct ctc cgg tgc tgt gac aat gat atg gaa aga aaa 669
aaa gtc ttg gat gat cta acc aag gtg gac ttg aaa tgg aat tct ctt 717
ctt aag att att gaa aag gca tgt cct agt tca gat aaa cat gct tta 765
gta act cct tgg ctc aaa ggc gat atc ctt ggt gag aaa ttg gtc aac 813
ttg gca gat tgt ctt tgt aat gag gac ttg gaa tcc agg gta tct tca 861
gaa tct cac ttc tca gaa aga tgg act ctt cta agc ttg gta tta tcc 909
caa cat gtt aaa aat gat tac ttg att gga gac gta tat gtt gaa aga 957
atc att gtt aga ctt cat gaa act tta ttc aaa aca aag aaa tta tca 1005
gaa gct gaa agc agt gac tca tca gtg tct ttt atc tgt gat gtg gcc 1053
tat aac tat ttc agc tca gcg aaa gga tgc ttg cta atg cca tca tct 1101
gaa gat tta tta tta act ctc ttt cag tta tgt gct cag agc aaa gaa 1149
aaa aca cat ttg cca gat ttt ctt atc tgt aaa ctg aaa aat act tgg 1197
ctc tct ggt gta aat tta ttg gtt cat caa act gac agt tca tat aaa 1245
gag agt acc ttc cta cat ttg tct gct ctg tgg ctg aag aac caa gtt 1293
cag gct tca tct ttg gat atc aac agt ctc caa gtc ctc ttg tct gct 1341
gtt gat gat ttg cta aat aca ctt cta gag agt gaa gat tct tat ctt 1389
atg gga gtt tat att gga agt gta atg ccg aac gac agt gaa tgg gaa 1437
aag atg agg cag tct ctt cct atg cag tgg tta cat aga cct ctt tta 1485
gag gga aga ttg agt ttg aat tat gaa tgt ttc aaa aca gat ttt aag 1533
gaa cag gac ata aag aca ctt ccc agc cat ttg tgt act tca gca tta 1581
ttg agc aaa atg gtc tta att gca ctg aga aag gaa aca gtc tta gaa 1629
aat aat gag ctt gag aaa ata att gca gaa ctg ctt tat tca ctg cag 1677
tgg tgt gaa gaa tta gat aac cca cct att ttt cta att gga ttt tgt 1725
gaa ata ctt caa aaa atg aat att acg tat gat aac tta cgt gta ctt 1773
ggt aat acg tcg ggc ctt ttg cag ctg tta ttt aac agg tcc aga gaa 1821
cat ggc aca ctg tgg tct ctt att att gct aag ttg atc ctt tcc cga 1869
agc att tca tct gat gaa gta aaa cca cat tat aag aga aaa gaa agt 1917
ttt ttt cca cta act gaa ggc aat ttg cat acc att caa agt cta tgt 1965
cca ttt ttg tca aaa gaa gaa aag aaa gaa ttt agt gct caa tgt ata 2013
cct gct ctt ttg ggc tgg act aag aaa gat ctt tgc agc act aat gga 2061
ggt ttt gga cat ctt gcc att ttc aat tct tgt ctg caa acc aaa agt 2109
ata gat gat gga gag cta tta cat gga ata tta aaa atc ata ata tcc 2157
tgg aag aaa gag cat gaa gat att ttt ctt ttc agt tgt aat cta tca 2205
gaa gca agt cca gag gta ctg ggt gta aat ata gaa ata atc cgg ttt 2253
ctt tcc cta ttt ctg aaa tac tgc tca tcc cct ttg gca gag agt gag 2301
tgg gac ttc atc atg tgc tcc atg ttg gct tgg ctg gag aca aca agt 2349
gag aat cag gca ttg tat tct att cca ctt gtg caa ctg ttt gcc tgt 2397
gtc agc tgt gat ttg gcc tgt gac ctc agt gct ttc ttt gat tcc aca 2445
act ctg gat acc att ggc aat ctt cct gta aat cta atc agt gaa tgg 2493
aaa gaa ttt ttt tcc caa ggc atc cac agt ttg ctt tta cct att ttg 2541
gtg act gtt aca gga gaa aac aaa gat gtg tct gaa aca tcc ttt cag 2589
aat gca atg ctg aaa ccc atg tgt gaa aca tta acg tat atc tca aag 2637
gaa cag cta ttg agt cac aaa ctt cct gca aga tta gtt gct gac caa 2685
aaa aca aac tta cca gaa tat ctc cag act ttg tta aat aca ttg gcc 2733
cca tta ctc ctc ttc aga gct agg cct gtg caa att gct gtt tat cat 2781
atg cta tac aaa ttg atg cct gaa tta cca cag tat gat cag gat aat 2829
cta aag tca tac gga gat gaa gaa gaa gag cca gcc ttg tca cca cca 2877
gca gca ctg atg tct ctt ctt agc att caa gag gac tta cta gaa aat 2925
gtt ttg ggg tgt att cct gtt gga cag ata gtt act att aaa cca ctg 2973
agt gaa gac ttc tgt tat gtt ctg gga tac ctt ctc act tgg aaa tta 3021
ata cta act ttc ttc aaa gct gca tca tca cag ctt cgg gct ttg tat 3069
tcc atg tat ctt cgg aaa aca aag agt ttg aat aaa ttg ctc tat cac 3117
ctg ttc agg ctt atg cca gaa aat cca acc tat gca gag aca gca gtt 3165
gag gtc cca aat aag gac cct aaa aca ttc ttt act gag gag ctc cag 3213
ctg agt att aga gaa aca aca atg ctt cca tac cac att cca cac ttg 3261
gct tgt tca gtc tat cat atg aca tta aaa gac ttg cct gcc atg gtt 3309
agg ttg tgg tgg aat agc agt gag aag cgt gtt ttc aat att gtg gat 3357
aga ttt aca agc aag tat gtc agc agt gtt ctt tct ttt caa gaa ata 3405
tct tct gta caa aca agt aca caa cta ttt aat ggc atg acg gtt aaa 3453
gct cga gct act act cga gag gta atg gct act tat act att gag gac 3501
ata gtt att gaa ctt ata ata caa ctg cct tca aat tat cca ctg ggt 3549
tca ata ata gta gaa agt ggg aaa aga gta gga gta gct gtt cag cag 3597
tgg cgg aac tgg atg ctg cag tta agc act tac ctc acc cat cag aat 3645
gga agt att atg gaa ggc tta gct tta tgg aaa aat aac gta gac aaa 3693
cgt ttt gag ggt gtt gaa gat tgc atg atc tgt ttc tca gtc att cac 3741
ggt ttc aac tat tcc ctt ccc aaa aaa gcc tgt aga aca tgc aag aaa 3789
aaa ttc cat tca gcc tgc ttg tac aaa tgg ttt aca tct agc aac aaa 3837
tcc act tgt cca ctg tgt cgt gag acg ttt ttc tgagattttt ttcactggaa 3890
gggatccctg aagtacatca aacaaaggca ttggatttgg atccgtctga aagtgtggat 3950
gtggggaagc cagtgagcat tacttttaaa taggaccttc tctggaaaat tattttggtt 4010
aatgtaataa tcctaaaatc aggtttactt aactttagat tgctttgtaa atgtttggaa 4070
actttctttt acaaaagaat attacatatt tgagagaaaa tatttctatt aatggtttaa 4130
tttctttgta tatttaaaaa taaatatgaa aaacccctaa attacagaat tggaatttgt 4190
gaaaagattg cacaactata ttattgataa acccattccg tttgttgatc tgtgttttag 4250
aatagtccaa gtgaagcaaa acgaactgag agagctgatt tacatctata gatattttaa 4310
gccttattta ttagttatga caataaatta gtattctgat atttgtagat tattttaatg 4370
ataaaaatga cactaagcta ttaatacggc ttgtcttaag atatctggag gcatgctctg 4430
aaatcctata taaaagagtt taaactgaac tttaaggtgc ctccatttat caagggttat 4490
gaaactcaga aaattaaatt cttgatgctg acctttttgc ttgtgggaga attatttcta 4550
tatattagtg aaatttgaat tgtaaacttg tcagtgaatt tatcactctg agcgagtctc 4610
tagagggtga gaatttgaga gaaatagtaa tttgattatt tcattcatga agcggagtgg 4670
tagttatcct tctattgaga gggaagaaat aaatgagtat tactgaaact taaagatcag 4730
tggaaaaaaa aaacatttaa aaatgttgta ggagactttc aattttggaa gcaaactgag 4790
ctattcttac cagccaaagt tatactaaat aagagacttg ggggagcaaa tgtttttcag 4850
ccttcagaaa tgaaagttaa ggattttagc actaggtaaa attcagtata ataggctgaa 4910
gtggagtaag tgaaaacctg ccttttgcca ctcttaaaaa ttgtgcccaa aatataaaag 4970
tgtggaactt tagaacttgg aataatttta ttgcagtctt ccattacatg gaaatagcat 5030
atctaatatc taggttactt gagagaccag ctaatcatct ctgttgcaca tgtttaattg 5090
gcaaaaagca attcatgata aataaaatta ctgctttcca tctactgggt aaaatgacta 5150
ttgaaatagt atgaatgtgg tcagaggatt atagttgaga gtgaagtact atgtgtgagt 5210
tatagatctc tcgaattata tttatagatg cagtgtcctg cccagttttg tttgcctcct 5270
acattttact gtaaaatatt tattgtcttc tagccttgag cctctgaggg tcagtaagtg 5330
aagtacagat agcaaaattt tactacctcg ttaacccttt tcttaaaata ttctctatct 5390
ttctatgtct ctttctgaca tagtaatccc aaaggattgt gttactcccc gtgaaagtta 5450
ttacttttcc tttaaaaatg gttttataat aagactgttt aaaacctttc cagtattggt 5510
acatcttggc ttctggccca aaccccaagc agaaaagaaa atggaataat ggagcattgt 5570
ttttccacat tagtattagg gcatcagatt cttggtgaaa cactataatt aagtagttat 5630
aattaaataa ctgttcttca tacttaggac tcttaataca tttctttaag acttgtaagt 5690
ataattgtaa agcttgttaa ctgtttatat actaaagaaa aagctttaca agataagact 5750
gaaattcaga ataccacatc tgtacagcgt tgacaactct aactaaaagg attacagccc 5810
ttaaaatttt agcttaagga cattttaaaa ttgcatgaga ttagcaaaat tattaattta 5870
aaaccccaaa gtaaattgca aattttaaaa tttatttttg actcttttat cacatgctcc 5930
aatgtatgta cataagtgtc tgtggcttaa ataatatgtt gttatttgat acatttttgt 5990
ggtgcaagta attaatgtta ttatttgatt gttataccat atgtgcccat taaaattctt 6050
tttaattaaa aatatttcaa atatgcagaa aaataataat ataaatgccc ttgtaccctt 6110
cacatactat tttgccatat ttgcttctga tttttaaata aataaaatgt tactcttttg 6170
taaaaaaaaa aaaaaaaaaa aaa 6193

<210> 101
<211> 474
<212> PRT
<213> Homo sapiens

<220>
<223> Designation: HFB030

<400> 101
Met Ser Ser Glu Asp Arg Glu Ala Gln Glu Asp Glu Leu Leu Ala Leu
1               5                   10                  15

Ala Ser Ile Tyr Asp Gly Asp Glu Phe Arg Lys Ala Glu Ser Val Gln
            20                  25                  30

Gly Gly Glu Thr Arg Ile Tyr Leu Asp Leu Pro Gln Asn Phe Lys Ile
        35                  40                  45

Phe Val Ser Gly Asn Ser Asn Glu Cys Leu Gln Asn Ser Gly Phe Glu
    50                  55                  60

Tyr Thr Ile Cys Phe Leu Pro Pro Leu Val Leu Asn Phe Glu Leu Pro
65                  70                  75                  80

Pro Asp Tyr Pro Ser Ser Ser Pro Pro Ser Phe Thr Leu Ser Gly Lys
                85                  90                  95

Trp Leu Ser Pro Thr Gln Leu Ser Ala Leu Cys Lys His Leu Asp Asn
            100                 105                 110

Leu Trp Glu Glu His Arg Gly Ser Val Val Leu Phe Ala Trp Met Gln
        115                 120                 125

Phe Leu Lys Glu Glu Thr Leu Ala Tyr Leu Asn Ile Val Ser Pro Phe
    130                 135                 140

Glu Leu Lys Ile Gly Ser Gln Lys Lys Val Gln Arg Arg Thr Ala Gln
145                 150                 155                 160

Ala Ser Pro Asn Thr Glu Leu Asp Phe Gly Gly Ala Ala Gly Ser Asp
                165                 170                 175

Val Asp Gln Glu Glu Ile Val Asp Glu Arg Ala Val Gln Asp Val Glu
            180                 185                 190

Ser Leu Ser Asn Leu Ile Gln Glu Ile Leu Asp Phe Asp Gln Ala Gln
        195                 200                 205

Gln Ile Lys Cys Phe Asn Ser Lys Leu Phe Leu Cys Ser Ile Cys Phe
    210                 215                 220

Cys Glu Lys Leu Gly Ser Glu Cys Met Tyr Phe Leu Glu Cys Arg His
225                 230                 235                 240

Val Tyr Cys Lys Ala Cys Leu Lys Asp Tyr Phe Glu Ile Gln Ile Arg
                245                 250                 255

Asp Gly Gln Val Gln Cys Leu Asn Cys Pro Glu Pro Lys Cys Pro Ser
            260                 265                 270

Val Ala Thr Pro Gly Gln Val Lys Glu Leu Val Glu Ala Glu Leu Phe
        275                 280                 285

Ala Arg Tyr Asp Arg Leu Leu Leu Gln Ser Ser Leu Asp Leu Met Ala
    290                 295                 300

Asp Val Val Tyr Cys Pro Arg Pro Cys Cys Gln Leu Pro Val Met Gln
305                 310                 315                 320

Glu Pro Gly Cys Thr Met Gly Ile Cys Ser Ser Cys Asn Phe Ala Phe
                325                 330                 335

Cys Thr Leu Cys Arg Leu Thr Tyr His Gly Val Ser Pro Cys Lys Val
            340                 345                 350

Thr Ala Glu Lys Leu Met Asp Leu Arg Asn Glu Tyr Leu Gln Ala Asp
        355                 360                 365

Glu Ala Asn Lys Arg Leu Leu Asp Gln Arg Tyr Gly Lys Arg Val Ile
    370                 375                 380

Gln Lys Ala Leu Glu Glu Met Glu Ser Lys Glu Trp Leu Glu Lys Asn
385                 390                 395                 400

Ser Lys Ser Cys Pro Cys Cys Gly Thr Pro Ile Glu Lys Leu Asp Gly
                405                 410                 415

Cys Asn Lys Met Thr Cys Thr Gly Cys Met Gln Tyr Phe Cys Trp Ile
            420                 425                 430

Cys Met Gly Ser Leu Ser Arg Ala Asn Pro Tyr Lys His Phe Asn Asp
        435                 440                 445

Pro Gly Ser Pro Cys Phe Asn Arg Leu Phe Tyr Ala Val Asp Val Asp
    450                 455                 460

Asp Asp Ile Trp Glu Asp Glu Val Glu Asp

465                 470

<210> 102
<211> 895
<212> PRT
<213> Homo sapiens

<220>
<223> Designation: HFB044-1

<400> 102
Met Lys Ile Gly Ser Gly Phe Leu Ser Gly Gly Gly Gly Thr Gly Ser
1               5                   10                  15

Ser Gly Gly Ser Gly Ser Gly Gly Gly Gly Ser Gly Gly Gly Gly Gly
            20                  25                  30

Gly Gly Ser Ser Gly Arg Arg Ala Glu Met Glu Pro Thr Phe Pro Gln
        35                  40                  45

Gly Met Val Met Phe Asn His Arg Leu Pro Pro Val Thr Ser Phe Thr
    50                  55                  60

Arg Pro Ala Gly Ser Ala Ala Pro Pro Pro Gln Cys Val Leu Ser Ser
65                  70                  75                  80

Ser Thr Ser Ala Ala Pro Ala Ala Glu Pro Pro Pro Pro Pro Ala Pro
                85                  90                  95

Asp Met Thr Phe Lys Lys Glu Pro Ala Ala Ser Ala Ala Ala Phe Pro
            100                 105                 110

Ser Gln Arg Thr Ser Trp Gly Phe Leu Gln Ser Leu Val Thr Ile Lys
        115                 120                 125

Gln Glu Lys Pro Ala Asp Pro Glu Glu Gln Gln Ser His His His His
    130                 135                 140

His His His His Tyr Gly Gly Leu Phe Ala Gly Ala Glu Glu Arg Ser

145                 150                 155                 160

Pro Gly Leu Gly Gly Gly Glu Gly Gly Ser His Gly Val Ile Gln Asp
                165                 170                 175

Leu Ser Ile Leu His Gln His Val Gln Gln Gln Pro Ala Gln His His
            180                 185                 190

Arg Asp Val Leu Leu Ser Ser Ser Ser Arg Thr Asp Asp His His Gly
        195                 200                 205

Thr Glu Glu Pro Lys Gln Asp Thr Asn Val Lys Lys Ala Lys Arg Pro
    210                 215                 220

Lys Pro Glu Ser Gln Gly Ile Lys Ala Lys Arg Lys Pro Ser Ala Ser
225                 230                 235                 240

Ser Lys Pro Ser Leu Val Gly Asp Gly Glu Gly Ala Ile Leu Ser Pro
                245                 250                 255

Ser Gln Lys Pro His Ile Cys Asp His Cys Ser Ala Ala Phe Arg Ser
            260                 265                 270

Ser Tyr His Leu Arg Arg His Val Leu Ile His Thr Gly Glu Arg Pro
        275                 280                 285

Phe Gln Cys Ser Gln Cys Ser Met Gly Phe Ile Gln Lys Tyr Leu Leu
    290                 295                 300

Gln Arg His Glu Lys Ile His Ser Arg Glu Lys Pro Phe Gly Cys Asp
305                 310                 315                 320

Gln Cys Ser Met Lys Phe Ile Gln Lys Tyr His Met Glu Arg His Lys
                325                 330                 335

Arg Thr His Ser Gly Glu Lys Pro Tyr Lys Cys Asp Thr Cys Gln Gln
            340                 345                 350

Tyr Phe Ser Arg Thr Asp Arg Leu Leu Lys His Arg Arg Thr Cys Gly
        355                 360                 365

Glu Val Ile Val Lys Gly Ala Thr Ser Ala Glu Pro Gly Ser Ser Asn
    370                 375                 380

His Thr Asn Met Gly Asn Leu Ala Val Leu Ser Gln Gly Asn Thr Ser
385                 390                 395                 400

Ser Ser Arg Arg Lys Thr Lys Ser Lys Ser Ile Ala Ile Glu Asn Lys
                405                 410                 415

Glu Gln Lys Thr Gly Lys Thr Asn Glu Ser Gln Ile Ser Asn Asn Ile
            420                 425                 430

Asn Met Gln Ser Tyr Ser Val Glu Met Pro Thr Val Ser Ser Ser Gly
        435                 440                 445

Gly Ile Ile Gly Thr Gly Ile Asp Glu Leu Gln Lys Arg Val Pro Lys
    450                 455                 460

Leu Ile Phe Lys Lys Gly Ser Arg Lys Asn Thr Asp Lys Asn Tyr Leu
465                 470                 475                 480

Asn Phe Val Ser Pro Leu Pro Asp Ile Val Gly Gln Lys Ser Leu Ser
                485                 490                 495

Gly Lys Pro Ser Gly Ser Leu Gly Ile Val Ser Asn Asn Ser Val Glu
            500                 505                 510

Thr Ile Gly Leu Leu Gln Ser Thr Ser Gly Lys Gln Gly Gln Ile Ser
        515                 520                 525

Ser Asn Tyr Asp Asp Ala Met Gln Phe Ser Lys Lys Arg Arg Tyr Leu
    530                 535                 540

Pro Thr Ala Ser Ser Asn Ser Ala Phe Ser Ile Asn Val Gly His Met
545                 550                 555                 560

Val Ser Gln Gln Ser Val Ile Gln Ser Ala Gly Val Ser Val Leu Asp
                565                 570                 575

Asn Glu Ala Pro Leu Ser Leu Ile Asp Ser Ser Ala Leu Asn Ala Glu
            580                 585                 590

Ile Lys Ser Cys His Asp Lys Ser Gly Ile Pro Asp Glu Val Leu Gln
        595                 600                 605

Ser Ile Leu Asp Gln Tyr Ser Asn Lys Ser Glu Ser Gln Lys Glu Asp
    610                 615                 620

Pro Phe Asn Ile Ala Glu Pro Arg Val Asp Leu His Thr Ser Gly Glu
625                 630                 635                 640

His Ser Glu Leu Val Gln Glu Glu Asn Leu Ser Pro Gly Thr Gln Thr
                645                 650                 655

Pro Ser Asn Asp Lys Ala Ser Met Leu Gln Glu Tyr Ser Lys Tyr Leu
            660                 665                 670

Gln Gln Ala Phe Glu Lys Ser Thr Asn Ala Ser Phe Thr Leu Gly His
        675                 680                 685

Gly Phe Gln Phe Val Ser Leu Ser Ser Pro Leu His Asn His Thr Leu
    690                 695                 700

Phe Pro Glu Lys Gln Ile Tyr Thr Thr Ser Pro Leu Glu Cys Gly Phe
705                 710                 715                 720

Gly Gln Ser Val Thr Ser Val Leu Pro Ser Ser Leu Pro Lys Pro Pro
                725                 730                 735

Phe Gly Met Leu Phe Gly Ser Gln Pro Gly Leu Tyr Leu Ser Ala Leu
            740                 745                 750

Asp Ala Thr His Gln Gln Leu Thr Pro Ser Gln Glu Leu Asp Asp Leu
        755                 760                 765

Ile Asp Ser Gln Lys Asn Leu Glu Thr Ser Ser Ala Phe Gln Ser Ser
    770                 775                 780

Ser Gln Lys Leu Thr Ser Gln Lys Glu Gln Lys Asn Leu Glu Ser Ser

785                 790                 795                 800

Thr Gly Phe Gln Ile Pro Ser Gln Glu Leu Ala Ser Gln Ile Asp Pro
                805                 810                 815

Gln Lys Asp Ile Glu Pro Arg Thr Thr Tyr Gln Ile Glu Asn Phe Ala
            820                 825                 830

Gln Ala Phe Gly Ser Gln Phe Lys Ser Gly Ser Arg Val Pro Met Thr
        835                 840                 845

Phe Ile Thr Asn Ser Asn Gly Glu Val Asp His Arg Val Arg Thr Ser
    850                 855                 860

Val Ser Asp Phe Ser Gly Tyr Thr Asn Met Met Ser Asp Val Ser Glu
865                 870                 875                 880

Pro Cys Ser Thr Arg Val Lys Thr Pro Thr Ser Gln Ser Tyr Arg
                885                 890                 895


<210> 103
<211> 895
<212> PRT
<213> Homo sapiens

<220>
<223> Designation: HFB044-2

<400> 103
Met Lys Ile Gly Ser Gly Phe Leu Ser Gly Gly Gly Gly Thr Gly Ser
 1               5                  10                  15

Ser Gly Gly Ser Gly Ser Gly Gly Gly Gly Ser Gly Gly Gly Gly Gly
            20                  25                  30

Gly Gly Ser Ser Gly Arg Arg Ala Glu Met Glu Pro Thr Phe Pro Gln
        35                  40                  45

Gly Met Val Met Phe Asn His Arg Leu Pro Pro Val Thr Ser Phe Thr

    50                  55                  60

Arg Pro Ala Gly Ser Ala Ala Pro Pro Pro Gln Cys Val Leu Ser Ser
65                  70                  75                  80

Ser Thr Ser Ala Ala Pro Ala Ala Glu Pro Pro Pro Pro Pro Ala Pro
                85                  90                  95

Asp Met Thr Phe Lys Lys Glu Pro Ala Ala Ser Ala Ala Ala Phe Pro
            100                 105                 110

Ser Gln Arg Thr Ser Trp Gly Phe Leu Gln Ser Leu Gly Ser Ile Lys
        115                 120                 125

Gln Glu Lys Pro Ala Asp Pro Glu Glu Gln Gln Ser His His His His
    130                 135                 140

His His His His Tyr Gly Gly Leu Phe Ala Gly Ala Glu Glu Arg Ser
145                 150                 155                 160

Pro Gly Leu Gly Gly Gly Glu Gly Gly Ser His Gly Val Ile Gln Asp
                165                 170                 175

Leu Ser Ile Leu His Gln His Val Gln Gln Gln Pro Ala Gln His His
            180                 185                 190

Arg Asp Val Leu Leu Ser Ser Ser Ser Arg Thr Asp Asp His His Gly
        195                 200                 205

Thr Glu Glu Pro Lys Gln Asp Thr Asn Val Lys Lys Ala Lys Arg Pro
    210                 215                 220

Lys Pro Glu Ser Gln Gly Ile Lys Ala Lys Arg Lys Pro Ser Ala Ser
225                 230                 235                 240

Ser Lys Pro Ser Leu Val Gly Asp Gly Glu Gly Ala Ile Leu Ser Pro
                245                 250                 255

Ser Gln Lys Pro His Ile Cys Asp His Cys Ser Ala Ala Phe Arg Ser
            260                 265                 270

Ser Tyr His Leu Arg Arg His Val Leu Ile His Thr Gly Glu Arg Pro
        275                 280                 285

Phe Gln Cys Ser Gln Cys Ser Met Gly Phe Ile Gln Lys Tyr Leu Leu
    290                 295                 300

Gln Arg His Glu Lys Ile His Ser Arg Glu Lys Pro Phe Gly Cys Asp
305                 310                 315                 320

Gln Cys Ser Met Lys Phe Ile Gln Lys Tyr His Met Glu Arg His Lys
                325                 330                 335

Arg Thr His Ser Gly Glu Lys Pro Tyr Lys Cys Asp Thr Cys Gln Gln
            340                 345                 350

Tyr Phe Ser Arg Thr Asp Arg Leu Leu Lys His Arg Arg Thr Cys Gly
        355                 360                 365

Glu Val Ile Val Lys Gly Ala Thr Ser Ala Glu Pro Gly Ser Ser Asn
    370                 375                 380

His Thr Asn Met Gly Asn Leu Ala Val Leu Ser Gln Gly Asn Thr Ser
385                 390                 395                 400

Ser Ser Arg Arg Lys Thr Lys Ser Lys Ser Ile Ala Ile Glu Asn Lys
                405                 410                 415

Glu Gln Lys Thr Gly Lys Thr Asn Glu Ser Gln Ile Ser Asn Asn Ile
            420                 425                 430

Asn Met Gln Ser Tyr Ser Val Glu Met Pro Thr Val Ser Ser Ser Gly
        435                 440                 445

Gly Ile Ile Gly Thr Gly Ile Asp Glu Leu Gln Lys Arg Val Pro Lys
    450                 455                 460

Leu Ile Phe Lys Lys Gly Ser Arg Lys Asn Thr Asp Lys Asn Tyr Leu
465                 470                 475                 480

Asn Phe Val Ser Pro Leu Pro Asp Ile Val Gly Gln Lys Ser Leu Ser
                485                 490                 495

Gly Lys Pro Ser Gly Ser Leu Gly Ile Val Ser Asn Asn Ser Val Glu
            500                 505                 510

Thr Ile Gly Leu Leu Gln Ser Thr Ser Gly Lys Gln Gly Gln Ile Ser
        515                 520                 525

Ser Asn Tyr Asp Asp Ala Met Gln Phe Ser Lys Lys Arg Arg Tyr Leu
    530                 535                 540

Pro Thr Ala Ser Ser Asn Ser Ala Phe Ser Ile Asn Val Gly His Met
545                 550                 555                 560

Val Ser Gln Gln Ser Val Ile Gln Ser Ala Gly Val Ser Val Leu Asp
                565                 570                 575

Asn Glu Ala Pro Leu Ser Leu Ile Asp Ser Ser Ala Leu Asn Ala Glu
            580                 585                 590

Ile Lys Ser Cys His Asp Lys Ser Gly Ile Pro Asp Glu Val Leu Gln
        595                 600                 605

Ser Ile Leu Asp Gln Tyr Ser Asn Lys Ser Glu Ser Gln Lys Glu Asp
    610                 615                 620

Pro Phe Asn Ile Ala Glu Pro Arg Val Asp Leu His Thr Ser Gly Glu
625                 630                 635                 640

His Ser Glu Leu Val Gln Glu Glu Asn Leu Ser Pro Gly Thr Gln Thr
                645                 650                 655

Pro Ser Asn Asp Lys Ala Ser Met Leu Gln Glu Tyr Ser Lys Tyr Leu
            660                 665                 670

Gln Gln Ala Phe Glu Lys Ser Thr Asn Ala Ser Phe Thr Leu Gly His
        675                 680                 685

Gly Phe Gln Phe Val Ser Leu Ser Ser Pro Leu His Asn His Thr Leu

690                 695                 700

Phe Pro Glu Lys Gln Ile Tyr Thr Thr Ser Pro Leu Glu Cys Gly Phe
705                 710                 715                 720

Gly Gln Ser Val Thr Ser Val Leu Pro Ser Ser Leu Pro Lys Pro Pro
                725                 730                 735

Phe Gly Met Leu Phe Gly Ser Gln Pro Gly Leu Tyr Leu Ser Ala Leu
            740                 745                 750

Asp Ala Thr His Gln Gln Leu Thr Pro Ser Gln Glu Leu Asp Asp Leu
        755                 760                 765

Ile Asp Ser Gln Lys Asn Leu Glu Thr Ser Ser Ala Phe Gln Ser Ser
    770                 775                 780

Ser Gln Lys Leu Thr Ser Gln Lys Glu Gln Lys Asn Leu Glu Ser Ser
785                 790                 795                 800

Thr Gly Phe Gln Ile Pro Ser Gln Glu Leu Ala Ser Gln Ile Asp Pro
                805                 810                 815

Gln Lys Asp Ile Glu Pro Arg Thr Thr Tyr Gln Ile Glu Asn Phe Ala
            820                 825                 830

Gln Ala Phe Gly Ser Gln Phe Lys Ser Gly Ser Arg Val Pro Met Thr
        835                 840                 845

Phe Ile Thr Asn Ser Asn Gly Glu Val Asp His Arg Val Arg Thr Ser
    850                 855                 860

Val Ser Asp Phe Ser Gly Tyr Thr Asn Met Met Ser Asp Val Ser Glu
865                 870                 875                 880

Pro Cys Ser Thr Arg Val Lys Thr Pro Thr Ser Gln Ser Tyr Arg
                885                 890                 895

<210> 104

<211> 367
<212> PRT
<213> Homo sapiens

<220>
<223> Designation: HFB066

<400> 104
Lys Asn Tyr Lys Asn Ala Ser Gly Val Val Asn Ser Ser Pro Arg Ser
1               5                   10                  15

His Ser Ala Thr Asn Gly Ser Ile Pro Ser Ser Ser Ser Lys Asn Glu
            20                  25                  30

Lys Lys Gln Lys Cys Thr Ser Lys Ser Pro Ser Thr His Lys Asp Leu
        35                  40                  45

Met Glu Asn Cys Ile Pro Asn Asn Gln Leu Ser Lys Pro Asp Ala Leu
    50                  55                  60

Val Arg Leu Glu Gln Asp Ile Lys Lys Leu Lys Ala Asp Leu Gln Ala
65                  70                  75                  80

Ser Arg Gln Val Glu Gln Glu Leu Arg Ser Gln Ile Ser Ser Leu Ser
                85                  90                  95

Ser Thr Glu Arg Gly Ile Arg Ser Glu Met Gly Gln Leu Arg Gln Glu
            100                 105                 110

Asn Glu Leu Leu Gln Asn Lys Leu His Asn Ala Val Gln Met Lys Gln
        115                 120                 125

Lys Asp Lys Gln Asn Ile Ser Gln Leu Glu Lys Lys Leu Lys Ala Glu
    130                 135                 140

Gln Glu Ala Arg Ser Phe Val Glu Lys Gln Leu Met Glu Glu Lys Lys
145                 150                 155                 160

Arg Lys Lys Leu Glu Glu Ala Thr Ala Ala Arg Ala Val Ala Phe Ala
                165                 170                 175

Ala Ala Ser Arg Gly Glu Cys Thr Glu Thr Leu Arg Asn Arg Ile Arg
            180                 185                 190

Glu Leu Glu Ala Glu Gly Lys Lys Leu Thr Met Asp Met Lys Val Lys
        195                 200                 205

Glu Asp Gln Ile Arg Glu Leu Glu Leu Lys Val Gln Glu Leu Arg Lys
    210                 215                 220

Tyr Lys Glu Asn Glu Lys Asp Thr Glu Val Leu Met Ser Ala Leu Ser
225                 230                 235                 240

Ala Met Gln Asp Lys Thr Gln His Leu Glu Asn Ser Leu Ser Ala Glu
                245                 250                 255

Thr Arg Ile Lys Leu Asp Leu Phe Ser Ala Leu Gly Asp Ala Lys Arg
            260                 265                 270

Gln Leu Glu Ile Ala Gln Gly Gln Ile Leu Gln Lys Asp Gln Gly Asn
        275                 280                 285

Gln Gly Pro Lys Thr Glu Asp Ser Arg Ser His Gly Arg His Ala Gln
    290                 295                 300

His Asn Ile Gln Cys Arg His Gln Pro Pro Glu Pro Cys Phe Pro Pro
305                 310                 315                 320

Leu Leu Phe Gln Ile Cys Gly Asp Gln Pro Leu Trp Thr Leu Thr Pro
                325                 330                 335

Met Ala Ser Val Tyr Gln Pro Leu Lys Lys Leu Lys Ala Ser Leu Cys
            340                 345                 350

Val Val Pro Lys Lys Phe Gly Leu Pro Gly Lys Ala Leu Gln Arg
        355                 360                 365

<210> 105
<211> 548

<212> PRT
<213> Homo sapiens

<220>
<223> Designation: HFB101-1

<400> 105

<210> 106
<211> 621
<212> PRT
<213> Homo sapiens

<220>
<223> Designation: HFB101-2

<400> 106
Met Ala Ser Arg Leu Pro Thr Ala Trp Ser Cys Glu Pro Val Thr Phe
1               5                   10                  15

Glu Asp Val Thr Leu Gly Phe Thr Pro Glu Glu Trp Gly Leu Leu Asp
            20                  25                  30

Leu Lys Gln Lys Ser Leu Tyr Arg Glu Val Met Leu Glu Asn Tyr Arg
        35                  40                  45

Asn Leu Val Ser Val Glu Tyr Pro Glu Leu Gln Leu Asp Pro Lys Leu
    50                  55                  60

Asp Pro Leu Pro Ala Glu Ser Pro Leu Met Asn Ile Glu Val Val Glu
65                  70                  75                  80

Val Leu Thr Leu Asn Gln Glu Val Ala Gly Pro Arg Asn Ala Gln Ile
                85                  90                  95

Gln Ala Leu Tyr Ala Glu Asp Gly Ser Leu Ser Ala Asp Ala Pro Ser
            100                 105                 110

Glu Gln Val Gln Gln Gln Gly Lys His Pro Gly Asp Pro Glu Ala Ala
        115                 120                 125

Arg Gln Arg Phe Arg Gln Phe Arg Tyr Lys Asp Met Thr Gly Pro Arg
    130                 135                 140

Glu Ala Leu Asp Gln Leu Arg Glu Leu Cys His Gln Trp Leu Gln Pro
145                 150                 155                 160

Lys Ala Arg Ser Lys Glu Gln Ile Leu Glu Leu Leu Val Leu Glu Gln
                165                 170                 175

Phe Leu Gly Ala Leu Pro Val Lys Leu Arg Thr Trp Val Glu Ser Gln
            180                 185                 190

His Pro Glu Asn Cys Gln Glu Val Val Ala Leu Val Glu Gly Val Thr
        195                 200                 205

Trp Met Ser Glu Glu Glu Val Leu Pro Ala Gly Gln Pro Ala Glu Gly
    210                 215                 220

Thr Thr Cys Cys Leu Glu Val Thr Ala Gln Gln Glu Glu Lys Gln Glu
225                 230                 235                 240

Asp Ala Ala Ile Cys Pro Val Thr Val Leu Pro Glu Glu Pro Val Thr
                245                 250                 255

Phe Gln Asp Val Ala Val Asp Phe Ser Arg Glu Glu Trp Gly Leu Leu
            260                 265                 270

Gly Pro Thr Gln Arg Thr Glu Tyr Arg Asp Val Met Leu Glu Thr Phe
        275                 280                 285

Gly His Leu Val Ser Val Gly Trp Glu Thr Thr Leu Glu Asn Lys Glu
    290                 295                 300

Leu Ala Pro Asn Ser Asp Ile Pro Glu Glu Glu Pro Ala Pro Ser Leu
305                 310                 315                 320

Lys Val Gln Glu Ser Ser Arg Asp Cys Ala Leu Ser Ser Thr Leu Glu
                325                 330                 335

Asp Thr Leu Gln Gly Gly Val Gln Glu Val Gln Asp Thr Val Leu Lys
            340                 345                 350

Gln Met Glu Ser Ala Gln Glu Lys Asp Leu Pro Gln Lys Lys His Phe
        355                 360                 365

Asp Asn Arg Glu Ser Gln Ala Asn Ser Gly Ala Leu Asp Thr Asn Gln
    370                 375                 380

Val Ser Leu Gln Lys Ile Asp Asn Pro Glu Ser Gln Ala Asn Ser Gly
385                 390                 395                 400

Ala Leu Asp Thr Asn Gln Val Leu Leu His Lys Ile Pro Pro Arg Lys
                405                 410                 415

Arg Leu Arg Lys Arg Asp Ser Gln Val Lys Ser Met Lys His Asn Ser
            420                 425                 430

Arg Val Lys Ile His Gln Lys Ser Cys Glu Arg Gln Lys Ala Lys Glu
        435                 440                 445

Gly Asn Gly Cys Arg Lys Thr Phe Ser Arg Ser Thr Lys Gln Ile Thr
    450                 455                 460

Phe Ile Arg Ile His Lys Gly Ser Gln Val Cys Arg Cys Ser Glu Cys
465                 470                 475                 480

Gly Lys Ile Phe Arg Asn Pro Arg Tyr Phe Ser Val His Lys Lys Ile
                485                 490                 495

His Thr Gly Glu Arg Pro Tyr Val Cys Gln Asp Cys Gly Lys Gly Phe
            500                 505                 510

Val Gln Ser Ser Ser Leu Thr Gln His Gln Arg Val His Ser Gly Glu
        515                 520                 525

Arg Pro Phe Glu Cys Gln Glu Cys Gly Arg Thr Phe Asn Asp Arg Ser
    530                 535                 540

Ala Ile Ser Gln His Leu Arg Thr His Thr Gly Ala Lys Pro Tyr Lys
545                 550                 555                 560

Cys Gln Asp Cys Gly Lys Ala Phe Arg Gln Ser Ser His Leu Ile Arg
                565                 570                 575

His Gln Arg Thr His Thr Gly Glu Arg Pro Tyr Ala Cys Asn Lys Cys
            580                 585                 590

Gly Lys Ala Phe Thr Gln Ser Ser His Leu Ile Gly His Gln Arg Thr
        595                 600                 605

His Asn Arg Thr Lys Arg Lys Lys Lys Gln Pro Thr Ser
    610                 615                 620

<210> 107
<211> 1135
<212> PRT
<213> Homo sapiens

<220>
<223> Designation: HFB341

<400> 107